(19) Europäisches Patentamt
European Patent Office
Office européen des brevets

(11) EP 1 003 775 B1

(12) EUROPEAN PATENT SPECIFICATION

(45) Date of publication and mention of the grant of the patent:
**16.03.2005 Bulletin 2005/11**

(21) Application number: **98939450.7**

(22) Date of filing: **10.08.1998**

(51) Int Cl.[7]: **C07K 14/18**, C07K 5/103, C07K 5/107, C07K 5/083, C07K 5/078, A61K 38/16, A61K 38/04

(86) International application number:
**PCT/CA1998/000765**

(87) International publication number:
**WO 1999/007733 (18.02.1999 Gazette 1999/07)**

# (54) HEPATITIS C INHIBITOR PEPTIDES

HEPATITIS C INHIBITOR PEPTIDE

PEPTIDES INHIBITEURS DE L'HEPATITE C

(84) Designated Contracting States:
**AT BE CH CY DE DK ES FI FR GB GR IE IT LI LU MC NL PT SE**

(30) Priority: **11.08.1997 US 55186 P**

(43) Date of publication of application:
**31.05.2000 Bulletin 2000/22**

(73) Proprietor: **BOEHRINGER INGELHEIM (CANADA) LTD.**
**Laval, Quebec, H7S 2G5 (CA)**

(72) Inventors:
- **LLINAS-BRUNET, Montse**
  **Pierrefonds, Québec H2V 2W8 (CA)**
- **POUPART, Marc-André**
  **Vimont, Québec H7M 1B3 (CA)**
- **RANCOURT, Jean**
  **Laval, Québec H7L 4W2 (CA)**
- **SIMONEAU, Bruno**
  **Laval, Québec H7N 5G3 (CA)**
- **TSANTRIZOS, Youla**
  **Saint-Laurent, Québec H4L 4P7 (CA)**
- **WERNIC, Dominik**
  **Laval, Québec H7X 3G5 (CA)**

(74) Representative: **Kläs, Heinz-Gerd, Dr. et al**
**Boehringer Ingelheim GmbH**
**Binger Strasse 173**
**55216 Ingelheim/Rhein (DE)**

(56) References cited:
**WO-A-98/17679**

- **MORI E.A.: "The N-terminal region of NS3 serine protease of HCV is important to maintain its enzymatic integrity" BIOCHEM.BIOPHYS.RES COMM., vol. 231, no. 3, 24 February 1997, pages 738-742, XP002086571**
- **LLINAS-BRUNET M ET AL: "Peptide-based inhibitors of the hepatitis C virus serine protease" BIOORGANIC & MEDICINAL CHEMISTRY LETTERS, vol. 8, no. 13, 7 July 1998, page 1713-1718 XP004137115**
- **INGALLINELLA E.A.: "Potent peptide inhibitors of human HCV NS3 protease are obtained by optimizing the cleavage products" BIOCHEMISTRY, vol. 37, no. 25, 23 June 1998, pages 8906-8914, XP002086572 cited in the application**
- **LANDRO E.A.: "Mechanistic role of NS4A peptide cofactor with the truncated NS3 protease of HCV: elucidation of the NS4A stimulatory efect via kinetic mapping and inhibitor mapping" BIOCHEMISTRY, vol. 36, no. 31, 5 August 1997, pages 9340-9348, XP002086573**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 003 775 B1

## Description

### Field of the invention

The present invention relates to compounds, compositions and methods for the treatment of hepatitis C virus (HCV) infection. In particular, the present invention provides novel peptides and analogues thereof, pharmaceutical compositions containing such peptides and methods for using these peptides in the treatment of HCV infection.

### Background of the invention

Hepatitis C virus (HCV) is the major etiological agent of post-transfusion and community-acquired non-A non-B hepatitis worldwide. It is estimated that over 100 million people worldwide are infected by the virus. A high percentage of carriers become chronically infected and many progress to chronic liver disease, so called chronic hepatitis C. This group is in turn at high risk for serious liver disease such as liver cirrhosis, hepatocellular carcinoma and terminal liver disease leading to death.

The mechanism by which HCV establishes viral persistence and causes a high rate of chronic liver disease has not been thoroughly elucidated. It is not known how HCV interacts with and evades the host immune system. In addition, the roles of cellular and humoral immune responses in protection against HCV infection and disease have yet to be established. Immunoglobulins have been reported for prophylaxis of transfusion-associated viral hepatitis. However, the Center for Disease Control does not presently recommend immunoglobulins for this purpose.

The lack of an effective protective immune response is hampering the development of a vaccine or adequate post-exposure prophylaxis measures, so in the near-term, hopes are firmly pinned on antiviral interventions.

Various clinical studies have been conducted with the goal of identifying pharmaceutical agents capable of effectively treating HCV infection in patients afflicted with chronic hepatitis C. These studies have involved the use of interferon-alpha, alone and in combination with other antiviral agents. Such studies have shown that a substantial number of the participants do not respond to these therapies, and of those that do respond favorably, a large proportion were found to relapse after termination of treatment.

Until recently, interferon (IFN) was the only available therapy of proven benefit approved in the clinic for patients with chronic hepatitis C. However the sustained response rate is low, and interferon treatment also induces severe side-effects (i.e. retinopathy, thyroiditis, acute pancreatitis, depression) that diminish the quality of life of treated patients. Recently, interferon in combination with ribavirin has been approved for patients non-responsive to IFN alone. However, the side effects caused by IFN are not alleviated with this combination therapy.

Therefore, a need exists for the development of effective antiviral agents for treatment of HCV infection that overcomes the limitations of existing pharmaceutical therapies.

HCV is an enveloped positive strand RNA virus in the Flaviviridae family. The single strand HCV RNA genome is approximately 9500 nucleotides in length and has a single open reading frame (ORF) encoding a single large polyprotein of about 3000 amino acids. In infected cells, this polyprotein is cleaved at multiple sites by cellular and viral proteases to produce the structural and non-structural (NS) proteins. In the case of HCV, the generation of mature nonstructural proteins (NS2, NS3, NS4A, NS4B, NSSA, and NS5B) is effected by two viral proteases. The first one, as yet poorly characterized, cleaves at the NS2-NS3 junction; the second one is a serine protease contained within the N-terminal region of NS3 (henceforth referred to as NS3 protease) and mediates all the subsequent cleavages downstream of NS3, both in *cis,* at the NS3-NS4A cleavage site, and in *trans,* for the remaining NS4A-NS4B, NS4B-NS5A, NSSA-NS5B sites. The NS4A protein appears to serve multiple functions, acting as a cofactor for the NS3 protease and possibly assisting in the membrane localization of NS3 and other viral replicase components. The complex formation of the NS3 protein with NS4A seems necessary to the processing events, enhancing the proteolytic efficiency at all of the sites. The NS3 protein also exhibits nucleoside triphosphatase and RNA helicase activities. NS5B is a RNA-dependent RNA polymerase that is involved in the replication of HCV.

A general strategy for the development of antiviral agents is to inactivate virally encoded enzymes that are essential for the replication of the virus. In this vein, patent application WO 97/06804 describes the (-) enantiomer of the nucleoside analogue cytosine-1,3-oxathiolane (also known as 3TC) as active against HCV. This compound, although reported as safe in previous clinical trials against HIV and HBV, has yet to be clinically proven active against HCV and its mechanism of action against the virus has yet to be reported.

Intense efforts to discover compounds which inhibit the NS3 protease or RNA helicase of HCV have led to the following disclosures:

- US patent 5,633,388 describes heterocyclic-substituted carboxamides and analogues as being active against HCV. These compounds are directed against the helicase activity of the NS3 protein of the virus but clinical tests have not yet been reported.

- A phenanthrenequinone has been reported by Chu *et al* (Tet. Lett., (1996), 7229-7232) to have activity against the HCV NS3 protease in vitro. No further development on this compound has been reported.
- A paper presented at the Ninth International Conference on Antiviral Research, Urabandai, Fukyshima, Japan (1996) (Antiviral Research, 30, 1, 1996; A23 (abstract 19)) reports thiazolidine derivatives to be inhibitory to the HCV protease.

Several studies have reported compounds inhibitory to other serine proteases, such as human leukocyte elastase. One family of these compounds is reported in WO 95/33764 (Hoechst Marion Roussel, 1995). The peptides disclosed in that application are morpholinylcarbonyl-benzoyl-peptide analogues that are structurally different from the peptides of the present invention.

- WO 98/17679 from Vertex Pharmaceuticals Inc. discloses inhibitors of serine protease, particularly, Hepatitis C virus NS3 protease. These inhibitors are peptide analogues based on the NSSA/5B natural substrate that contain C-terminal activated carbonyl function as an essential feature. These peptides were also reported to be active against other serine protease and are therefore not specific for HCV NS3 protease.
- Hoffman LaRoche has also reported hexapeptides that are proteinase inhibitors useful as antiviral agents for the treatment of HCV infection. These peptides contain an aldehyde or a boronic acid at the C-terminus.
- Steinkuhler *et al.* and Ingallinella et al. have published on NS4A-4B product inhibition (Biochemistry (1998), 37, 8899-8905 and 8906-8914). These peptides and peptide analogues were published after the priority date of the present application.

One advantage of the present invention is that it provides peptides that are inhibitory to the NS3 protease of the hepatitis C virus.

A further advantage of one aspect of the present invention resides in the fact that these peptides specifically inhibit the NS3 protease and do not show significant inhibitory activity at concentrations up to 300 μM against other serine proteases such as human leukocyte elastase (HLE), porcine pancreatic elastase (PPE), or bovine pancreatic chymotrypsin, or cysteine proteases such as human liver cathepsin B (Cat B).

**Summary of the invention**

We investigated peptides potentially inhibitory to the NS3 protease. The discovery that the N-terminal cleavage product **(Ac-D-D-I-V-P-C-OH)** of an analogue of a natural substrate of the NS3 protease was inhibitory led us to the peptide analogues of the present invention.

Disclosed are compounds of formula (I):

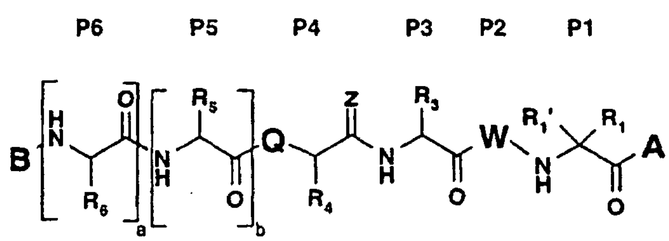

(I)

wherein **Q** is $CH_2$ or N-**Y** wherein **Y** is H or $C_{1-6}$ alkyl;

a) when **Q** is $CH_2$, **a** is 0, **b** is 0, and **B** is an amide derivative of formula $\mathbf{R_{11a}}$N($\mathbf{R_{11b}}$)-C(O)- wherein $\mathbf{R_{11a}}$ is H; $C_{1-10}$ alkyl optionally substituted with carboxyl or di($C_{1-6}$ alkyl) amino; $C_6$ aryl; $C_{7-10}$ alkylaryl; $C_{3-7}$ cycloalkyl; ($C_{3-7}$ cycloalkyl)-($C_{1-6}$ alkyl); heterocycle-$C_{1-6}$ alkyl such as

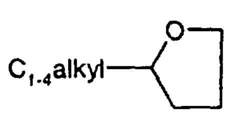

or

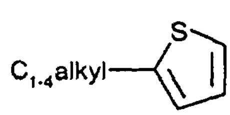

;

and **$R_{11b}$** is $C_{1-6}$ alkyl substituted with carboxyl, ($C_{1-6}$ alkoxy)carbonyl or phenylmethoxycarbonyl; or $C_{7-16}$ aralkyl substituted on the aromatic portion with carboxyl, ($C_{1-6}$ alkoxy)carbonyl or phenylmethoxycarbonyl or heterocycle-$C_{1-6}$ alkyl; or **$R_{11a}$** and **$R_{11b}$** are joined to form a 3 to 7-membered nitrogen-containing ring optionally substituted with carboxyl or ($C_{1-6}$ alkoxy) carbonyl;

or

b) when **Q** is N-**Y**, **a** is 0 or 1, **b** is 0 or 1, and **B** is an acyl derivative of formula **$R_{11}$**-C(O)-wherein **$R_{11}$** is (i) $C_{1-10}$ alkyl optionally substituted with carboxyl, $C_{1-6}$ alkanoyloxy (e.g. $AcOCH_2$) or $C_{1-6}$ alkoxy (e.g. Boc); (ii) $C_{3-7}$ cycloalkyl optionally substituted with carboxyl, ($C_{1-6}$ alkoxy)carbonyl or phenylmethoxycarbonyl; (iii) $C_{3-7}$ cycloalkyl substituted with carboxyl and one to three $C_{1-6}$ alkyl substituents (iv) $C_{4-10}$ (alkylcycloalkyl) optionally substituted on the cycloalkyl portion with carboxy, ($C_{1-6}$ alkoxy)carbonyl or phenylmethyoxycarbonyl; (v)

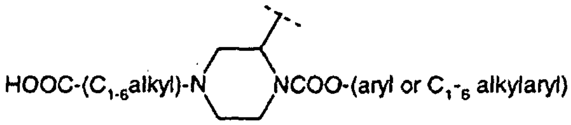 ,

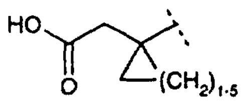 or 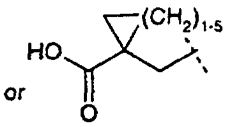 ;

(vi) $C_6$ or $C_{10}$ aryl or $C_{7-16}$ aralkyl optionally substituted with $C_{1-6}$ alkyl;

**$R_6$**, when present, is $C_{1-6}$ alkyl substituted with carboxyl;

**$R_5$**, when present, is $C_{1-6}$ alkyl optionally substituted with carboxyl;

or

when **Q** is either $CH_2$ or N-**Y**;

c) **$R_4$** is $C_{1-10}$ alkyl, $C_{3-7}$ cycloalkyl or $C_{4-10}$ (alkylcycloalkyl);

**Z** is oxo or thioxo;

**$R_3$** is $C_{1-10}$ alkyl optionally substituted with carboxyl, $C_{3-7}$ cycloalkyl or $C_{4-10}$ (alkylcycloalkyl) ;

**W** is a group of formula III':

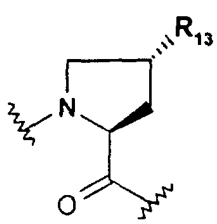

Formula III'

wherein **$R_{13}$** is OH; SH; $NH_2$; carboxyl; **$R_{12}$**; O**$R_{12}$**, S**$R_{12}$**, NH**$R_{12}$** or N**$R_{12}$R_{12}$'** wherein **$R_{12}$** and **$R_{12}$'** are independently: cyclic $C_{3-16}$ alkyl or acyclic $C_{1-16}$ alkyl or cyclic $C_{3-16}$ alkenyl or acyclic $C_{2-16}$ alkenyl, said alkyl or alkenyl optionally substituted with $NH_2$, OH, SH, halo, or carboxyl; said alkyl or alkenyl optionally containing at least one heteroatom independently selected from the group consisting of: O, S, and N; or

**$R_{12}$** and **$R_{12}$'** are independently $C_6$ or $C_{10}$ aryl or $C_{7-16}$ aralkyl optionally substituted with $C_{1-6}$ alkyl, $NH_2$, OH, SH, halo, carboxyl or carboxy(lower)alkyl; said aryl or aralkyl optionally containing at least one heteroatom independently selected from the group consisting of: O, S, and N;

said cyclic alkyl, cyclic alkenyl, aryl or aralkyl being optionally fused with a second 5-, 6-, or 7-membered ring to form a cyclic system or heterocyclic system, said second ring being optionally substituted with $NH_2$, OH, SH, halo, carboxyl or carboxy ($C_{1-6}$) alkyl; said second ring optionally containing at least one heteroatom independently selected from the group consisting of: O, S, and N; **$R_1$'** is hydrogen, and **$R_1$** is propyl or

**$R_1$'** and **$R_1$** together form a 3-membered ring optionally substituted with $C_{1-6}$ alkyl; and

A is hydroxy or a pharmaceutically acceptable salt or ester thereof.

Included within the scope of this invention is a pharmaceutical composition comprising an anti-hepatitis C virally effective amount of a compound of formula I, or a therapeutically acceptable salt or ester thereof, in admixture with a pharmaceutically acceptable carrier medium or auxiliary agent.

An important aspect of the invention involves a method of treating a hepatitis C viral infection in a mammal by administering to the mammal an anti-hepatitis C virally effective amount of the compound of formula I, or a therapeutically acceptable salt or ester thereof or a composition as described above.

Another important aspect involves a method of inhibiting the replication of hepatitis C virus by exposing the virus to a hepatitis C viral NS3 protease inhibiting amount of the compound of formula I, or a therapeutically acceptable salt or ester thereof or a composition as described above.

Still another aspect involves a method of treating a hepatitis C viral infection in a mammal by administering thereto an anti-hepatitis C virally effective amount of a combination of the compound of formula I, or a therapeutically acceptable salt or ester thereof, and an interferon. A pharmaceutical composition comprising the combination in admixture with a pharmaceutically acceptable carrier medium or auxiliary agent is also within the scope of this invention.

## Detailed description of the invention

As used herein, the following definitions apply unless otherwise noted:

With reference to the instances where (*R*) or *(S)* is used to designate the configuration of a radical, e.g. $R_4$ of the compound of formula I, the designation is done in the context of the compound and not in the context of the radical alone.

The natural amino acids, with exception of glycine, contain a chiral carbon atom. Unless otherwise specifically indicated, the compounds containing natural amino acids with the L-configuration are preferred. However, applicants contemplate that when specified, some amino acids of the formula I can be of either D- or L- configuration or can be mixtures of D- and L-isomers, including racemic mixtures.

The designation "P1, P2, P3 et." as used herein refer to the position of the amino acid residues starting from the C-terminus end of the peptide analogues and extending towards the N-terminus (i.e. P1 refers to position 1 from the C-terminus, P2: second position from the C-terminus, etc.) (see Berger A. & Schechter I., Transactions of the Royal Society London series B257, 249-264 (1970)).

The abbreviations for the α-amino acids are set forth in Table A.

Table A

| **AMINO ACID** | **SYMBOL** |
|---|---|
| Allylglycine | AlGly |
| Aminobutyric acid | Abu |
| 1-aminocyclopentylcarboxylic acid | Acpe |
| 1-aminocyclopropylcarboxylic acid | Acca |
| Alanine | Ala |
| Aspartic acid | Asp |
| Cysteine | Cys |
| Cyclohexylalanine | Cha |
| Cyclohexylglycine (also named: 2-amino-2-cyclohexylacetic acid) | Chg |
| Glutamic acid | Glu |
| Isoleucine | Ile |
| Leucine | Leu |
| Norvaline | Nva |
| Phenylalanine | Phe |
| Pipecolic acid | Pip |
| Proline | Pro |

Table A (continued)

| AMINO ACID | SYMBOL |
|---|---|
| 4(*R*)-Hydroxyproline | Hyp |
| 4(*R*)-Benzyloxyproline | Hyp(4-Bn) |
| Valine | Val |
| *tert*-Butylglycine | Tbg |

As used herein the term "aminobutyric acid" refers to a compound of formula:

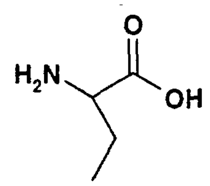

As used herein the term "allylglycine" refers to a compound of formula:

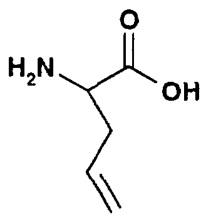

As used herein the term "1-aminocyclopropylcarboxylic acid" (Acca) refers to a compound of formula:

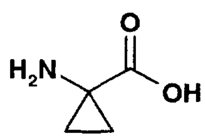

As used herein the term "*tert*-butylglycine" refers to a compound of formula:

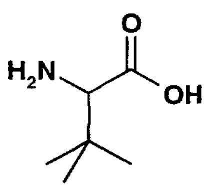

The term "residue" with reference to an amino acid or amino acid derivative means a radical derived from the corresponding α-amino acid by eliminating the hydroxyl of the carboxy group and one hydrogen of the α-amino group. For instance, the terms Gln, Ala, Gly, Ile, Arg, Asp, Phe, Ser, Leu, Cys, Asn, Sar and Tyr represent the "residues" of L-glutamine, L-alanine, glycine, L-isoleucine, L-arginine, L-aspartic acid, L-phenylalanine, L-serine, L-leucine, L-cysteine, L-asparagine, sarcosine and L-tyrosine, respectively.

The term "side chain" with reference to an amino acid or amino acid residue means a group attached to the

α-carbon atom of the α-amino acid. For example, the R-group side chain for glycine is hydrogen, for alanine it is methyl, for valine it is isopropyl. For the specific R-groups or side chains of the α-amino acids reference is made to A.L. Lehninger's text on Biochemistry (see chapter 4).

The term "halo" as used herein means a halogen radical selected from bromo, chloro, fluoro or iodo.

The term "$C_{1-6}$ alkyl" or "(lower)alkyl" as used herein, either alone or in combination with another radical, means straight chain or branched alkyl radicals containing up to six carbon atoms and includes, for example, methyl, ethyl, propyl, butyl, hexyl, 1-methylethyl, 1-methylpropyl, 2-methylpropyl, 1,1-dimethylethyl.

Likewise, the terms "$C_{1-3}$ alkyl" "$C_{1-4}$ alkyl" and "$C_{1-10}$ alkyl" are used to denote alkyl radials containing up to three, four and ten carbon atoms, respectively.

The term "$C_{3-7}$ cycloalkyl" as used herein, either alone or in combination with another radical, means a cycloalkyl radical containing from three to seven carbon atoms and includes cyclopropyl, cyclobutyl, cyclopentyl, cyclohexyl and cycloheptyl.

The term "$C_{4-10}$ (alkylcycloalkyl) as used herein means a cycloalkyl radical containing from three to seven carbon atoms linked to an alkyl radical, the linked radicals containing up to ten carbon atoms; for example, cyclopropylmethyl, cyclopentylethyl, cyclohexylmethyl, cyclohexylethyl or cycloheptylethyl.

The term "$C_{2-10}$ alkenyl" as used herein, either alone or in combination with another radical, means an alkyl radical as defined above containing from 2 to 10 carbon atoms, and further containing at least one double bond. For example alkenyl includes allyl.

The term "$C_{3-4}$ alkylene" as used herein means a divalent alkyl radical derived by the removal of two hydrogen atoms from a straight or branched chain aliphatic hydrocarbon containing from three to four carbon atoms and includes, for example, $-CH_2CH_2CH_2-$, $CH(CH_3)CH_2CH_2-$, $-CH_2C(CH_3)_2-$ and $-CH_2CH_2CH_2CH_2-$.

The term "$C_{1-6}$ alkoxy" as used herein, either alone or in combination with another radical, means the radical $-O-C_{1-6}$ alkyl wherein alkyl is as defined above containing up to six carbon atoms. Alkoxy includes methoxy, ethoxy, propoxy, 1-methylethoxy, butoxy and 1,1-dimethylethoxy. The latter radical is known commonly as *tert*-butoxy.

The term "$C_6$ or $C_{10}$ aryl" as used herein, either alone or in combination with another radical, means either an aromatic monocyclic system containing 6 carbon atoms or an aromatic cyclic system containing 10 carbon atoms. For example, aryl includes phenyl or naphthalene.

The term "$C_{7-16}$ aralkyl" as used herein, either alone or in combination with another radical, means an aryl as defined above linked through an alkyl group, wherein alkyl is as defined above containing from 1 to 6 carbon atoms. Aralkyl includes for example benzyl, and butylphenyl.

The term "carboxy(lower)alkyl" as used herein, either alone or in combination with another radical, means a carboxyl group (COOH) linked through a (lower)alkyl group as defined above and includes for example butyric acid or the groups:

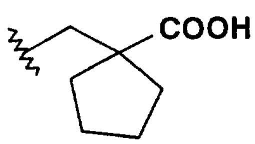

or

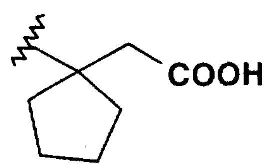

The term "cyclic" or "cyclic system" as used herein, either alone or in combination with another radical, means a monovalent radical derived by removal of a hydrogen from a saturated or unsaturated cyclic hydrocarbon, containing from three to seven carbon atoms, unless otherwise indicated and optioonally conctaing one or more heteroatom. The term cyclic or cyclic system includes, for example, cyclopropane, cyclopentane, cyclohexane, cyclohexene, decalin, tetralin, indene, and naphthalene.

The term "heterocycle" as used herein, either alone or in combination with another radical, means a monovalent radical derived by removal of a hydrogen from a five-, six-, or seven-membered saturated or unsaturated heterocycle containing from one to four heteroatoms selected from nitrogen, oxygen and sulfur. Examples of suitable heterocycles include: pyrrolidine, tetrahydrofuran, thiazolidine, pyrrole, thiophene, diazepine, 1H-imidazole, 1-methyl-1H-imidazole, isoxazole, thiazole, 2-methylthiazole, 2-aminothiazole, piperidine, 1,4-dioxane, 4-morpholine, pyridine, 2-methylpyridine, pyrimidine, 4-methylpyrimidine and 2,4-dimethylpyrimidine.

The term "heterocyclic system" as used herein, either alone or in combination with another radical, means a heterocycle as defined above fused to one or more other cycle be it a heretocycle or any other cycle. Examples of suitable heterocyclic systems include: thiazolo[4,5-b]-pyridine, quinoline, or indole.

The term "pharmaceutically acceptable ester" as used herein, either alone or in combination with another

radical, means esters of the compound of formula I in which any of the carboxyl functions of the molecule, but preferably the carboxy terminus, is replaced by an alkoxycarbonyl function:

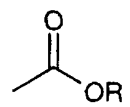

in which the **R** moiety of the ester is selected from alkyl (e.g. methyl, ethyl, *n*-propyl, *t*-butyl, *n*-butyl); alkoxyalkyl (e.g. methoxymethyl); alkoxyacyl (e.g. acetoxymethyl); aralkyl (e.g. benzyl); aryloxyalkyl (e.g. phenoxymethyl); aryl (e.g. phenyl), optionally substituted with halogen, $C_{1-4}$ alkyl or $C_{1-4}$ alkoxy. Other suitable prodrug esters can be found in Design of prodrugs, Bundgaard, H. Ed. Elsevier (1985). Such pharmaceutically acceptable esters are usually hydrolyzed *in vivo* when injected in a mammal and transformed into the acid form of the compound of formula I.

The term "pharmaceutically acceptable salt" as used herein includes those derived from pharmaceutically acceptable bases. Examples of suitable bases include choline, ethanolamine and ethylenediamine. $Na^+$, $K^+$, and $Ca^{++}$ salts are also contemplated to be within the scope of the invention (also see Pharmaceutical salts, Birge, S.M. et al., J. Pharm. Sci. (1977), 66, 1-19).

**Preferred embodiments**

A further preferred group of compounds are represented by formula Ia:

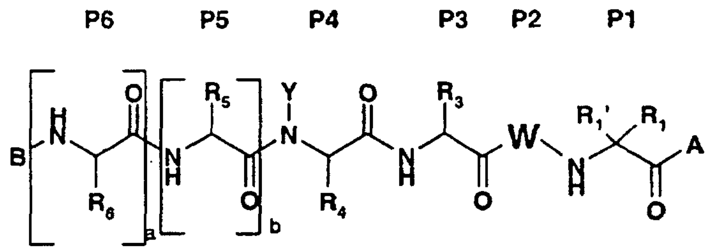

(Ia)

wherein **Y** is H or $C_{1-6}$ alkyl;
**a** is 0 or 1;
**b** is 0 or 1;
**B** is an acyl derivative of formula $\mathbf{R_{11}}$-C(O)-wherein $\mathbf{R_{11}}$ is (i) $C_{1-10}$ alkyl optionally substituted with carboxyl, $C_{1-6}$ alkanoyloxy or $C_{1-6}$ alkoxy; (ii) $C_{3-7}$ cycloalkyl optionally substituted with carboxyl, ($C_{1-6}$ alkoxy)carbonyl or phenylmethoxycarbonyl; (iii) $C_{3-7}$ cycloalkyl substituted with carboxyl and one to three $C_{1-6}$ alkyl substituents (iv) $C_{4-10}$ (alkylcycloalkyl) optionally substituted on the cycloalkyl portion with carboxy, ($C_{1-6}$ alkoxy)carbonyl or phenylmethyoxycarbonyl; (v)

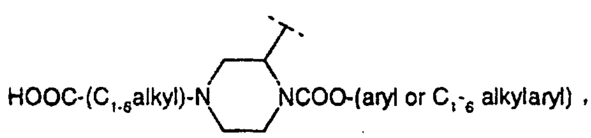

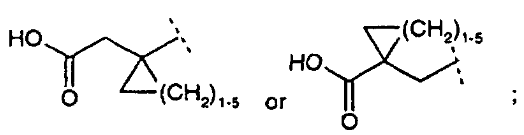

(vi) $C_6$ or $C_{10}$ aryl or $C_{7-16}$ aralkyl optionally substituted with $C_{1-6}$ alkyl;
$\mathbf{R_6}$, when present, is $C_{1-6}$ alkyl substituted with carboxyl;

**$R_5$,** when present, is $C_{1-6}$ alkyl optionally substituted with carboxyl; and
**$R_4$** is $C_{1-10}$ alkyl, $C_{3-7}$ cycloalkyl or $C_{4-10}$ (alkylcycloalkyl);
**$R_3$, W**, **$R_1$**, **$R_1$'** and **A** are as defined above.

Preferably, **B** is an acyl derivative of formula
**$R_{11}$**C(O)- wherein **$R_{11}$** is: $C_{1-6}$ alkyl optionally substituted with carboxyl, $C_{1-6}$ alkanoyloxy or $C_{1-6}$ alkoxy;
$C_{3-7}$ cycloalkyl optionally substituted with carboxyl, MeOC(O), EtOC(O) or BnOC(O);
3-carboxypropionyl (DAD) or 4-carboxybutyryl (DAE); or

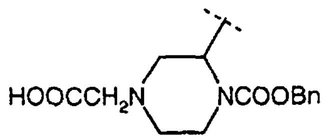
.

More preferably, **B** is acetyl, 3-carboxypropionyl, 4-carboxylbutyryl, $AcOCH_2C(O)$, $Me_3COC(O)$,

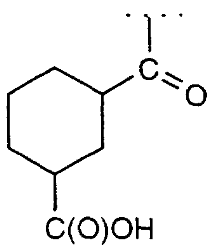
,
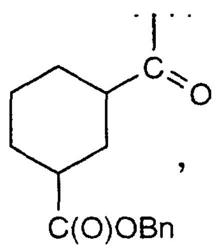
,
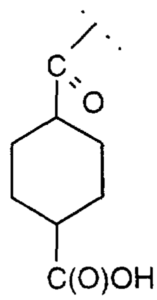
,
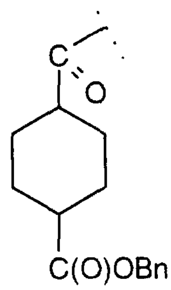
,

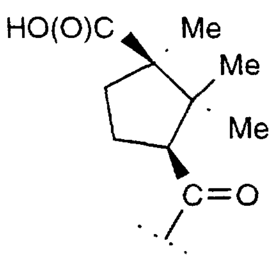
,
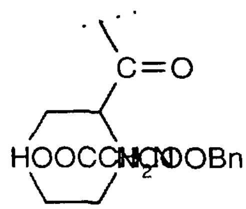
,

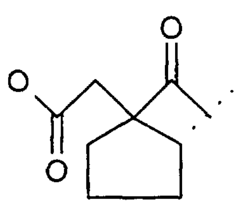
and
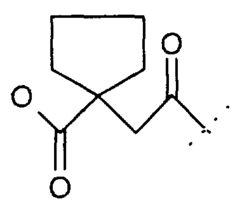
.

Still, more preferably, **B** is acetyl, 3-carboxypropionyl (DAD), 4-carboxybutyryl (DAE), $AcOCH_2C(O)$,

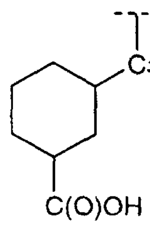

or

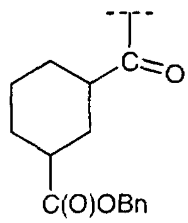

Most preferably, **B** is acetyl.

Preferably, $\mathbf{R_6}$**,** when present, is the side chain of Asp or Glu.
Most preferably, $\mathbf{R_6}$**,** when present, is the side chain of Asp.
Alternatively, preferably, **a** is 0 and then $\mathbf{R_6}$ is absent.

Preferably, $\mathbf{R_5}$**,** when present, is the side chain of an amino acid selected from the group consisting of: D-Asp, L-Asp, D-Glu, L-Glu, D-Val, L-Val, D-*tert*-butylglycine (Tbg), and L-Tbg.
More preferably, $\mathbf{R_5}$**,** when present, is the side chain of D-Asp, D-Val, or D-Glu.
Most preferably, $\mathbf{R_5}$**,** when present, is the side chain of D-Glu.
Alternatively, preferably **a** is 0 and **b** is 0, and then both $\mathbf{R_6}$ and $\mathbf{R_5}$ are absent.

Alternatively, another preferred group of compounds are represented by formula (Ib):

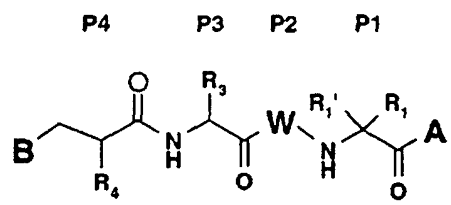

**(Ib)**

wherein **B** is preferably an amide of formula $\mathbf{R_{11a}}$N($\mathbf{R_{11b}}$)C(O)- wherein $\mathbf{R_{11a}}$ is preferably $C_{1-6}$ alkyl, optionally substituted with carboxy, $C_6$ aryl, $C_{7-10}$ arylalkyl, 2-tetrahydrofuranylmethyl, or 2-thiazolidylmethyl;
and $\mathbf{R_{11b}}$ is preferably $C_{1-4}$ alkyl substituted with carboxyl.

Most preferably, $\mathbf{R_{11a}}$ is isopropyl, carboxyethyl, benzylmethyl, benzyl, or 2-tetrahydrofuranylmethyl. More preferably $\mathbf{R_{11b}}$ is $C_{1-4}$ alkyl substituted with carboxyl. Most preferably, $\mathbf{R_{11b}}$ is ethyl carboxyl.

Compounds of the invention include compounds of formula I wherein, preferably, $\mathbf{R_4}$ is selected from the group consisting of: isopropyl, cyclopropyl, cyclohexyl, tert-butyl, 1-methylpropyl, and 2-methylpropyl.
More preferably, $\mathbf{R_4}$ is cyclopropyl or 1-methylpropyl. Most preferably, $\mathbf{R_4}$ is cyclopropyl.

Compounds of the invention include compounds of formula I wherein **Z** is preferably oxo.

Compounds of the invention include compounds of formula I wherein preferably, $\mathbf{R_3}$ is the side chain of an amino acid selected from the group consisting of:
Ile, allo-Ile, Chg, Cha, Val, Tbg or Glu.
More preferably, $\mathbf{R_3}$ is the side chain of Val, Tbg or Chg.
Most preferably, $\mathbf{R_3}$ is the side chain of Val.

In Formula III' :

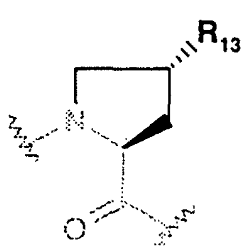

Formula III'

$\mathbf{R_{13}}$ is OH; SH; $NH_2$; carboxyl; $\mathbf{R_{12}}$; O$\mathbf{R_{12}}$, S$\mathbf{R_{12}}$, NH$\mathbf{R_{12}}$ or N$\mathbf{R_{12}}\mathbf{R_{12}}$' wherein $\mathbf{R_{12}}$ and $\mathbf{R_{12}}$' are independently:

cyclic $C_{3-16}$ alkyl or acyclic $C_{1-16}$ alkyl or cyclic $C_{3-16}$ alkenyl or acyclic $C_{2-16}$ alkenyl, said alkyl or alkenyl optionally substituted with $NH_2$, OH, SH, halo, or carboxyl; said alkyl or alkenyl optionally containing at least one heteroatom independently selected from the group consisting of: O, S, and N; or
$\mathbf{R_{12}}$ and $\mathbf{R_{12}}$' are independently $C_6$ or $C_{10}$ aryl or $C_{7-16}$ aralkyl optionally substituted with $C_{1-6}$ alkyl, $NH_2$, OH, SH, halo, carboxyl or carboxy($C_{1-6}$)alkyl; said aryl or aralkyl optionally containing at least one heteroatom independently selected from the group consisting of: O, S, and N;
said cyclic alkyl, cyclic alkenyl, aryl or aralkyl being optionally fused with a second 5-, 6-, or 7-membered ring to form a cyclic system or heterocyclic system, said second ring being optionally substituted with $NH_2$, OH, SH, halo, carboxyl or carboxy(lower)alkyl; said second ring optionally containing at least one heteroatom independently selected from the group consisting of: O, S, and N.

More preferably $\mathbf{R_{13}}$ is O$\mathbf{R_{12}}$ or S$\mathbf{R_{12}}$ wherein $\mathbf{R_{12}}$ is a $C_6$ or $C_{10}$ aryl or $C_{7-16}$ aralkyl, said first aryl or aralkyl optionally substituted with $C_{1-6}$ alkyl, $C_{3-7}$ cycloalkyl, $NH_2$, OH, SH, halo, $C_{1-6}$ alkoxy, carboxyl, carboxy(lower)alkyl, or a second aryl or aralkyl; said first and second aryl or aralkyl optionally containing at least one heteroatom selected independently from the group consisting of: O, S, and N.

Most preferably, $\mathbf{R_{13}}$ is Bn; $PhCH_2CH_2$; $PhCH_2CH_2CH_2$; O-Bn; o-tolylmethoxy; m-tolylmethoxy; p-tolylmethoxy; 1-naphtyloxy; 2-naphtyloxy; 1-naphthalenylmethoxy; 2-naphthalenylmethoxy; (4-tert-butyl)methoxy; (3I-Ph)$CH_2O$; (4Br-Ph)O; (2Br-Ph)O; (3Br-Ph)O; (4I-Ph)O; (3Br-Ph)$CH_2O$; (3,5-$Br_2$-Ph)$CH_2O$;

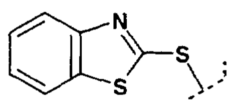 ; 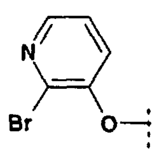 ; 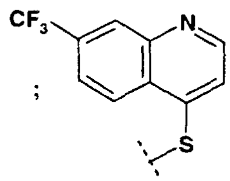 ; 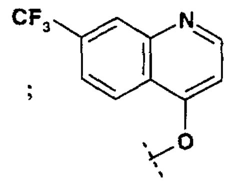 ;

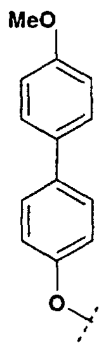 ; 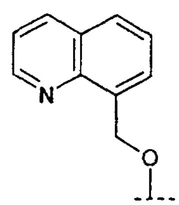 ; 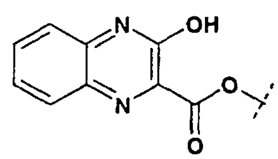 ;

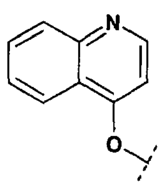 ; 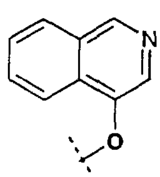 ; 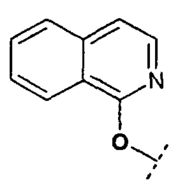 ; 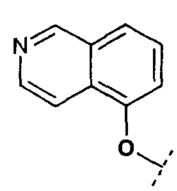 ;

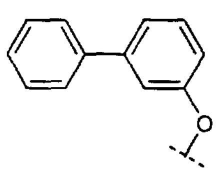 ; 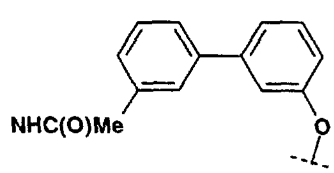 ;

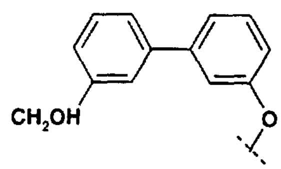 ; 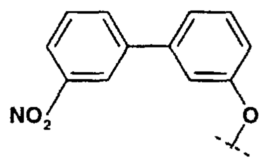 ;

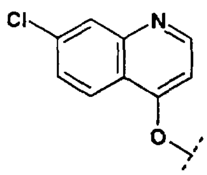 ; 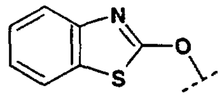 ; 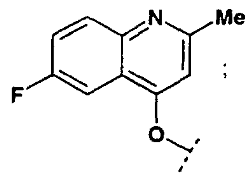 ;

Still most preferably, $\mathbf{R_{13}}$ is $PhCH_2CH_2CH_2$; O-Bn; 1-naphtyloxy; 2-naphtyloxy; 1-naphthalenylmethoxy; 2-naphthalenylmethoxy;

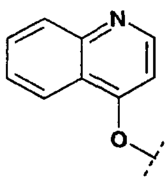 ; 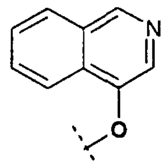 ; 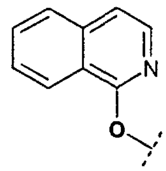 ; 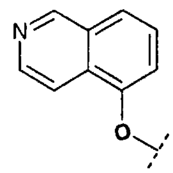 ;

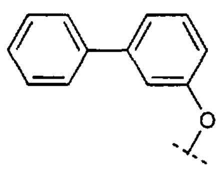 ; 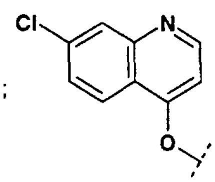 .

$\mathbf{R_1'}$ is hydrogen and $\mathbf{R_1}$ is propyl.

Alternatively, preferably, $\mathbf{R_1'}$ and $\mathbf{R_1}$ together form a 3-membered ring, said ring being optionally substituted with $C_{1-6}$ alkyl such as ethyl. For example, $\mathbf{R_1'}$ and $\mathbf{R_1}$ together can be the side chain (shown in bold) of the following amino acid:

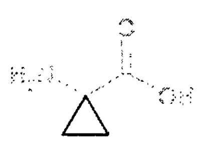

referred to as 1-aminocyclopropylcarboxylic acid (Acca).

Further included in the present invention are compounds of formula I wherein **A** is preferably hydroxy, a salt or an ester thereof. More preferably, **A** is hydroxy or an ester thereof. Most preferably, **A** is hydroxy.

More preferably, the ester is $C_{1-6}$ alkoxy, or (aryl $C_{1-6}$-alkoxy). Most preferably, the ester is methoxy, ethoxy, phenoxy, or benzyloxy

Included in the scope of the invention are compounds of formula I wherein **Q** is $CH_2$, **a** is 0, **b** is 0, and then **B** is an amide of formula $\mathbf{R_{11a}}N(\mathbf{R_{11b}})$-C(O)- wherein $\mathbf{R_{11a}}$ is $C_{1-6}$ alkyl, $C_{3-6}$ cycloalkyl, $C_{3-7}$ (alkylcylcoalkyl) optionally substituted with carboxy, $C_{1-3}$ carboxyalkyl, phenyl, $C_{7-10}$ arylalkyl, 2-tetrahydrofuranylmethyl, or 2-thiazolidylmethyl; and $\mathbf{R_{11b}}$ is phenyl; or $C_{1-6}$ alkyl substituted with carboxyl or $C_{1-4}$ carboxyalkyl;

or

**Q** is N-**Y** wherein **Y** is H or $C_{1-6}$ alkyl; **a** is 0 or 1; **b** is 0 or 1; and **B** is an acyl derivative of formula **$R_{11}$**-C(O)- wherein **$R_{11}$** is (i) $C_{1-6}$ alkyl, $C_{1-6}$ alkyl substituted with carboxyl, MeC(O)O-, MeO-, EtO-, $MeCH_2CH_2O$- or $Me_3C$-O-; (ii) cyclopentyl or cyclohexyl optionally substituted with carboxyl; (iv) $C_{4-10}$ (alkylcycloalkyl) optionally substituted on the cycloalkyl portion with carboxyl;

(v)

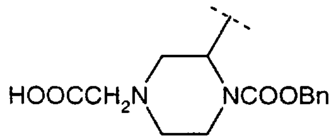

(vi) phenyl, benzyl or phenylethyl;

**$R_6$**, when present, is $CH_2COOH$ or $CH_2CH_2COOH$, **$R_5$**, when present, is $C_{1-6}$ alkyl or $CH_2COOH$ or $CH_2CH_2COOH$; and when **Q** is either $CH_2$ or N-**Y**,

**$R_4$** is $C_{1-6}$ alkyl, $C_{3-7}$ cycloalkyl or $C_{4-10}$ (alkylcycloalkyl);

**Z** is oxo or thio;

**$R_3$** is $C_{1-6}$ alkyl; $C_{3-7}$ cycloalkyl or $C_{4-10}$ (alkylcycloalkyl);

**W** is a group of formula III' as previously defined

**$R_{1'}$** is hydrogen and **$R_1$** is propyl; or **$R_{1'}$** and **$R_1$** together with the carbon atom to which they are attached form a cyclopropyl which may optionally be substituted with ethyl; and

**A** is hydroxy or a pharmaceutically acceptable salt thereof; $C_{1-6}$ alkoxy, or (aryl $C_{1-6}$-alkoxy).

Included in the scope of the invention are compounds of formula Ia, wherein **B** is an acyl derivative of formula **$R_{11}$**-C(O)- wherein **$R_{11}$** is $C_{1-6}$ alkoxy, $C_{1-10}$ alkyl optionally substituted with carboxyl; $C_{3-7}$ cycloalkyl optionally substituted with carboxyl or benzylcarboxy; or

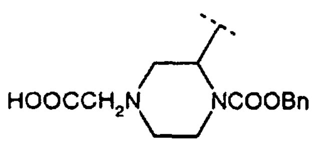
;

**$R_6$** is absent;

**$R_5$** is absent;

**$R_4$** is $C_{1-10}$ alkyl, $C_{3-7}$ cycloalkyl or $C_{4-10}$ (alkylcycloalkyl);

**$R_3$** is $C_{1-10}$ alkyl, $C_{3-7}$ cycloalkyl or $C_{4-10}$ (alkylcycloalkyl); **W** is a group of formula III' as previously defined; and

A is hydroxy or a pharmaceutically acceptable salt thereof; methoxy, ethoxy, phenoxy, or benzyloxy.

Included in the scope of the invention are compounds of formula Ia, wherein **B** is acetyl, 3-carboxypropionyl, 4-carboxylbutyryl, $AcOCH_2C(O)$, $Me_3COC(O)$,

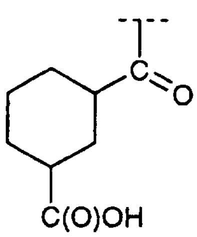
,
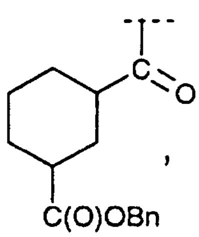
,
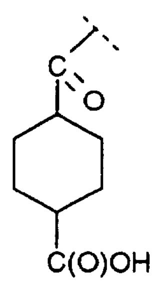
,
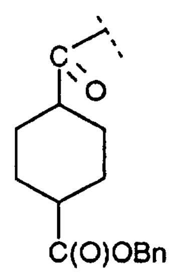
,

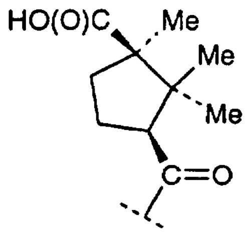 , 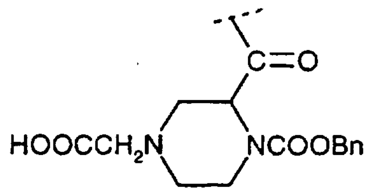 ,

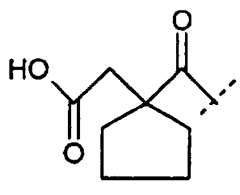 , 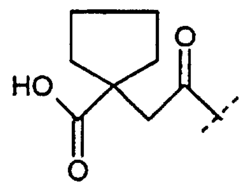 ;

**Y** is H or Me, **a** is 0 or 1, **b** is 0 or 1,
**$R_6$**, when present, is the side chain of Asp or Glu,
**$R_5$**, when present, is the side chain of Asp, D-Asp, Glu, D-Glu, Val, D-Val or Tbg,
**$R_4$** is the side chain of Val, Chg, Tbg, Ile or Leu,
**Z** is oxo or thioxo,
**$R_3$** is hydrogen or the side chain of Ile, Chg, Val, Glu;
**W** is group of formula III':

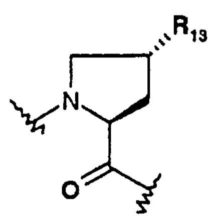 (III')

wherein **$R_{13}$** is Bn, $PhCH_2CH_2$, $PhCH_2CH_2CH_2$, O-Bn, o-tolylmethoxy, m-tolylmethoxy, p-tolylmethoxy, 1-naphthalenylmethoxy, 2-naphthalenylmethoxy, (4-*tert*-butyl)benzyloxy, (3I-Ph)$CH_2O$, (4Br-Ph)O, (2Br-Ph)O, (3Br-Ph)O, (4I-Ph)O, (3Br-Ph)$CH_2O$, (3,5-$Br_2$-Ph)$CH_2O$,

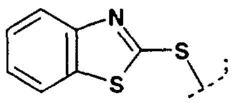 ; 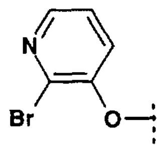 ; 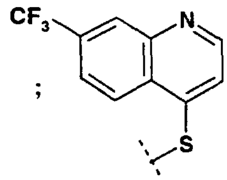 ; 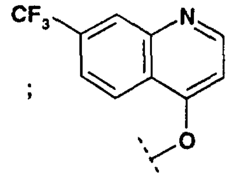 ;

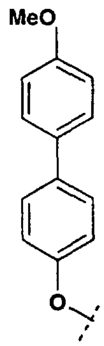 ; 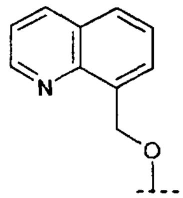 ; 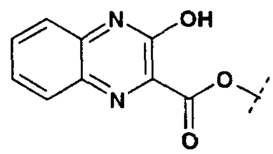 ;

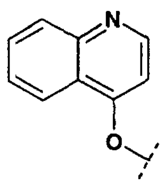 ; 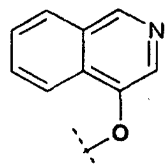 ; 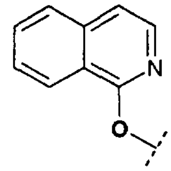 ; 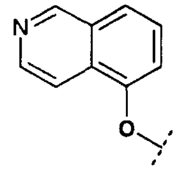 ;

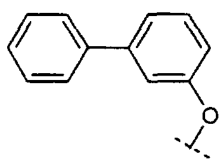 ; 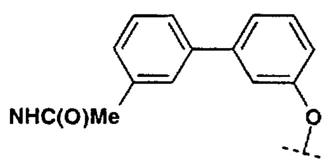 ;

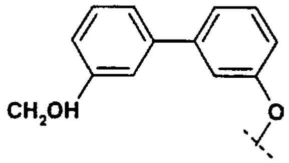 ; 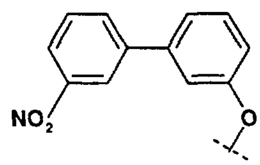 ;

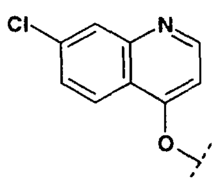 ; 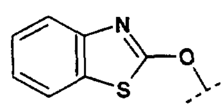 ; 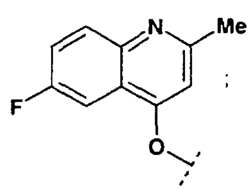 ;

**R$_{1'}$** is H and **R$_1$** is propyl; or

**R$_{1'}$** and **R$_1$** together with the carbon atom to which they are attached form a cyclopropyl; and **A** is hydroxyl.

Also included in the scope of the invention are compounds of formula Ib, wherein **B** is an amide of formula **R$_{11a}$**N(**R$_{11b}$**)-C(O)- wherein **R$_{11a}$** is $C_{1-6}$ alkyl, $C_{3-6}$ cycloalkyl, $C_{3-7}$ (alkylcylcoalkyl) optionally substituted with carboxy,

$C_{1-3}$ carboxyalkyl, phenyl, $C_{7-10}$ arylalkyl, 2-tetrahydrofuranylmethyl, or 2-thiazolidylmethyl; and $\mathbf{R_{11b}}$ is phenyl;or $C_{1-6}$ alkyl substituted with carboxyl or $C_{1-4}$ carboxyalkyl;
$\mathbf{R_4}$ is cyclohexyl;
**Z** is oxo;
$\mathbf{R_3}$ is hydrogen or the side chain of Ile, Chg, Val, Glu;
**W** is group of formula III':

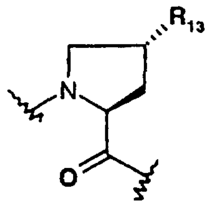

(III')

wherein $\mathbf{R_{13}}$ is Bn, $PhCH_2CH_2$, $PhCH_2CH_2CH_2$, O-Bn, o-tolylmethoxy, m-tolylmethoxy, p-tolylmethoxy, 1-naphthalenylmethoxy, 2-naphthalenylmethoxy, (4-*tert*-butyl)methoxy, (3I-Ph)$CH_2O$, (4Br-Ph)O, (2Br-Ph)O, (3Br-Ph) O, (41-Ph) O, (3Br-Ph) $CH_2O$, (3,5-$Br_2$-Ph)$CH_2O$,

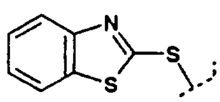 ; 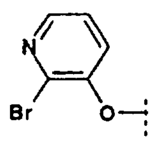 ; 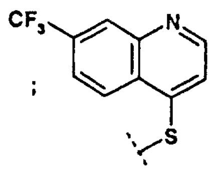 ; 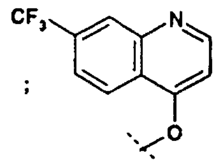 ;

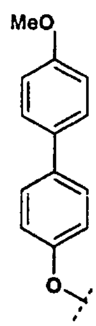 ; 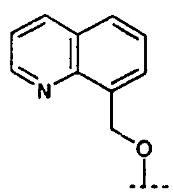 ; 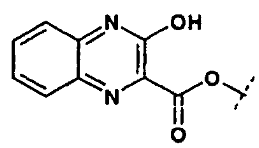 ;

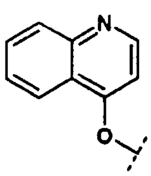 ; 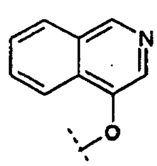 ; 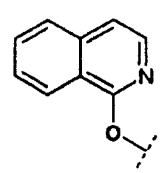 ; 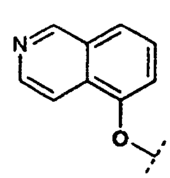 ;

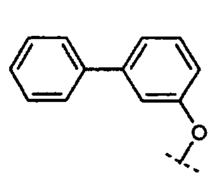 ; 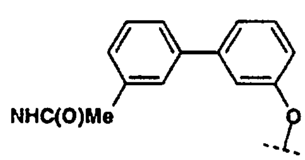 ;

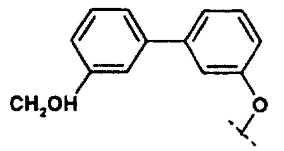 ; 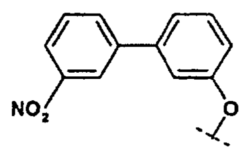 ;

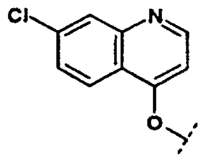 ; 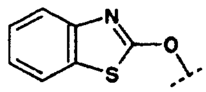 ; 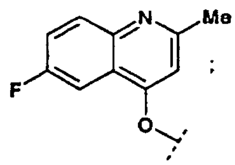 ;

$\mathbf{R_{1'}}$ is H and $\mathbf{R_1}$ is propyl; or
$\mathbf{R_{1'}}$ and $\mathbf{R_1}$ together with the carbon atom to which they are attached form a cyclopropyl; and **A** is hydroxyl.

Also disclosed are compounds of formula I: wherein **B** is an acyl derivative of formula $\mathbf{R_{11}}$-C(O)- wherein $\mathbf{R_{11}}$ is $C_{1-10}$ alkyl optionally substituted with carboxyl; $C_{3-7}$ cycloalkyl optionally substituted with carboxyl; or a $C_{4-10}$ (alkylcycloalkyl) optionally substituted on the cycloalkyl portion with carboxyl; or $\mathbf{R_{11}}$ is $C_6$ or $C_{10}$ aryl or $C_{7-16}$ aralkyl optionally substituted with a $C_{1-6}$ alkyl
**a** is 0 or 1;
$\mathbf{R_6}$, when present, is $C_{1-6}$ alkyl optionally substituted with carboxyl;
**b** is 0 or 1;
$\mathbf{R_5}$, when present, is $C_{1-6}$ alkyl optionally substituted with carboxyl;
**Q** is N-**Y** wherein **Y** is H or $C_{1-6}$ alkyl;
$\mathbf{R_4}$ is $C_{1-10}$ alkyl, $C_{3-7}$ cycloalkyl or $C_{4-10}$ (alkylcycloalkyl);
**Z** is oxo;
$\mathbf{R_3}$ is $C_{1-10}$ alkyl, $C_{3-7}$ cycloalkyl or $C_{4-10}$) (alkylcycloalkyl);
**W** is a group of formula III' as defined above.

$\mathbf{R_1'}$, is hydrogen, and $\mathbf{R_1}$ is propyl; or $\mathbf{R_1'}$ and $\mathbf{R_1}$ together form a 3-membered ring optionally substituted with $C_{1-6}$ alkyl; and **A** is OH or a pharmaceutically acceptable salt or ester thereof.

Finally, included in the scope of the invention are all compounds of formula I presented in Tables 1 to 4 wherein P1 represents Nva or Acca.

According to an alternate embodiment, the pharmaceutical compositions of this invention may additionally comprise an antiviral agent. Examples of antiviral agents include, ribavirin and amantadine.

According to another alternate embodiment, the pharmaceutical compositions of this invention may additionally comprise other inhibitors of HCV protease.

According to yet another alternate embodiment, the pharmaceutical compositions of this invention may additionally comprise an inhibitor of other targets in the HCV life cycle, such as helicase, polymerase, or metalloprotease.

The pharmaceutical compositions of this invention may be administered orally, parenterally or via an implanted reservoir. We prefer oral administration or administration by injection. The pharmaceutical compositions of this invention may contain any conventional non-toxic pharmaceutically-acceptable carriers, adjuvants or vehicles. In some cases, the pH of the formulation may be adjusted with pharmaceutically acceptable acids, bases or buffers to enhance the stability of the formulated compound or its delivery form. The term parenteral as used herein includes subcutaneous, intracutaneous, intravenous, intramuscular, intra-articular, intrasynovial, intrasternal, intrathecal, and intralesional injection or infusion techniques. The pharmaceutical compositions may be in the form of a sterile injectable preparation, for example, as a sterile injectable aqueous or oleaginous suspension. This suspension may be formulated according to techniques known in the art using suitable dispersing or wetting agents (such as, for example. Tween 80) and suspending agents.

The pharmaceutical compositions of this invention may be orally administered in any orally acceptable dosage form including, but not limited to, capsules, tablets, and aqueous suspensions and solutions. In the case of tablets for oral use, carriers which are commonly used include lactose and corn starch. Lubricating agents, such as magnesium stearate, are also typically added. For oral administration in a capsule form, useful diluents include lactose and dried corn starch. When aqueous suspensions are administered orally, the active ingredient is combined with emulsifying and suspending agents. If desired, certain sweetening and/or flavoring and/or coloring agents may be added.

Other suitable vehicles or carriers for the above noted formulations and compositions can be found in standard pharmaceutical texts, e.g. in "Remington's Pharmaceutical Sciences", The Science and Practice of Pharmacy, $19^{th}$ Ed. Mack Publishing Company, Easton, Penn., (1995).

Dosage levels of between about 0.01 and about 100 mg/kg body weight per day, preferably between about 0.5 and about 75 mg/kg body weight per day of the protease inhibitor compounds described herein are useful in a monotherapy for the prevention and treatment of HCV mediated disease. Typically, the pharmaceutical compositions of this invention will be administered from about 1 to about 5 times per day or alternatively, as a continuous infusion. Such administration can be used as a chronic or acute therapy. The amount of active ingredient that may be combined with the carrier materials to produce a single dosage form will vary depending upon the host treated and the particular mode of administration. A typical preparation will contain from about 5% to about 95% active compound (*w*/*w*). Preferably, such preparations contain from about 20% to about 80% active compound.

As the skilled artisan will appreciate, lower or higher doses than those recited above may be required. Specific dosage and treatment regimens for any particular patient will depend upon a variety of factors, including the activity of the specific compound employed, the age, body weight, general health status, sex, diet, time of administration, rate of excretion, drug combination, the severity and course of the infection, the patient's disposition to the infection and the judgment of the treating physician. Generally, treatment is initiated with small dosages substantially less than the optimum dose of the peptide. Thereafter, the dosage is increased by small increments until the optimum effect under the circumstances is reached. In general, the compound is most desirably administered at a concentration level that will generally afford antivirally effective results without causing any harmful or deleterious side effects.

When the compositions of this invention comprise a combination of a compound of formula I and one or more additional therapeutic or prophylactic agent, both the compound and the additional agent should be present at dosage levels of between about 10 to 100%, and more preferably between about 10 and 80% of the dosage normally administered in a monotherapy regimen.

When these compounds or their pharmaceutically acceptable salts are formulated together with a pharmaceutically acceptable carrier, the resulting composition may be administered *in vivo* to mammals, such as man, to inhibit HCV NS3 protease or to treat or prevent HCV virus infection. Such treatment may also be achieved using the compounds of this invention in combination with agents which include, but are not limited to: immunomodulatory agents, such as α-, β-, or γ-interferons; other antiviral agents such as ribavirin, amantadine; other inhibitors of HCV NS3 protease; inhibitors of other targets in the HCV life cycle such as helicase, polymerase, metalloprotease, or internal ribosome entry; or combinations thereof. The additional agents may be combined with the compounds of this invention to create a single dosage form. Alternatively these additional agents may be separately administered to a mammal as part of a multiple dosage form.

Accordingly, another embodiment of this invention provides methods of inhibiting HVC NS3 protease activity in mammals by administering a compound of the formula I, wherein the substituents are as defined above.

In a preferred embodiment, these methods are useful in decreasing HCV NS3 protease activity in a mammal. If the pharmaceutical composition comprises only a compound of this invention as the active component, such methods may additionally comprise the step of administering to said mammal an agent selected from an immunomodulatory agent, an antiviral agent, a HCV protease inhibitor, or an inhibitor of other targets in the HCV life cycle such as helicase, polymerase, or metallo protease. Such additional agent may be administered to the mammal prior to, concurrently with, or following the administration of the compositions of this invention.

In an alternate preferred embodiment, these methods are useful for inhibiting viral replication in a mammal. Such methods are useful in treating or preventing HCV disease. If the pharmaceutical composition comprises only a compound of this invention as the active component, such methods may additionally comprise the step of administering to said mammal an agent selected from an immunomodulatory agent, an antiviral agent, a HCV protease inhibitor, or an inhibitor of other targets in the HCV life cycle. Such additional agent may be administered to the mammal prior to, concurrently with, or following the administration of the composition according to this invention.

The compounds set forth herein may also be used as laboratory reagents. The compounds of this invention may also be used to treat or prevent viral contamination of materials and therefore reduce the risk of viral infection of laboratory or medical personnel or patients who come in contact with such materials (e.g. blood, tissue, surgical instruments and garments, laboratory instruments and garments, and blood collection apparatuses and materials).

**PROCESS**

The compounds of the present invention were synthesized according to the process as illustrated in scheme I (wherein PG1 is a carboxyl protecting group and PG2 is an amino protecting group):

Scheme I

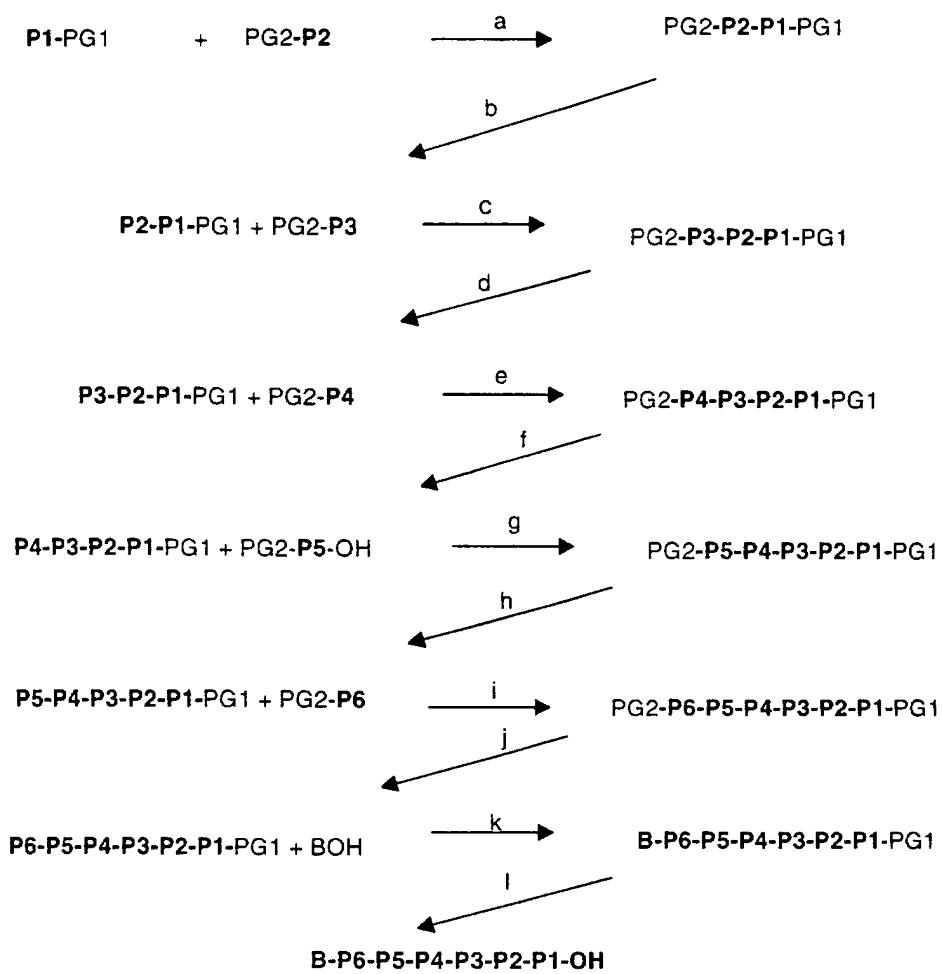

(I)

Briefly, the P1, P2, P3, P4, and optionally P5 and P6 can be linked by well known peptide coupling techniques. The P1, P2, P3, P4, and P5 and P6 groups may be linked together in any order as long as the final compound corresponds to peptides of formula **I**. For example, P6 can be linked to P5 to give P5-P6 that is linked to P4-P3-P2-P1 ; or P6 linked to P5-P4-P3-P2 then linked to an appropriately C-terminal protected P1.

Generally, peptides are elongated by deprotecting the α-amino group of the N-terminal residue and coupling the unprotected carboxyl group of the next suitably N-protected amino acid through a peptide linkage using the methods described. This deprotection and coupling procedure is repeated until the desired sequence is obtained. This coupling can be performed with the constituent amino acids in stepwise fashion, as depicted in Scheme I, or by condensation of fragments (two or several amino acids), or combination of both processes, or by solid phase peptide synthesis according to the method originally described in Merrifield, J. Am. Chem. Soc. (1963), 85, 2149-2154.

Coupling between two amino acids, an amino acid and a peptide, or two peptide fragments can be carried out using standard coupling procedures such as the azide method, mixed carbonic-carboxylic acid anhydride (isobutyl chloroformate) method, carbodiimide (dicyclohexylcarbodiimide, diisopropylcarbodiimide, or water-soluble carbodiimide) method, active ester (p-nitrophenyl ester, N-hydroxysuccinic imido ester) method, Woodward reagent K-method, carbonyldiimidazole method, phosphorus reagents or oxidation-reduction methods. Some of these methods (especially the carbodiimide method) can be enhanced by adding 1-hydroxybenzotriazole. These coupling reactions can be performed in either solution (liquid phase) or solid phase.

More explicitly, the coupling step involves the dehydrative coupling of a free carboxyl of one reactant with the free amino group of the other reactant in the presence of a coupling agent to form a linking amide bond. Descriptions of such coupling agents are found in general textbooks on peptide chemistry, for example, M. Bodanszky, "Peptide

Chemistry", 2nd rev ed., Springer-Verlag, Berlin, Germany, (1993). Examples of suitable coupling agents are *N,N'*-dicyclohexylcarbodiimide, 1-hydroxybenzotriazole in the presence of *N,N'*-dicyclohexylcarbodiimide or *N*-ethyl-*N'*-[(3-dimethylamino)propyl]carbodiimide. A very practical and useful coupling agent is the commercially available (benzotriazol-1-yloxy)tris-(dimethylamino)phosphonium hexafluorophosphate, either by itself or in the presence of 1-hydroxybenzotriazole. Another very practical and useful coupling agent is commercially available 2-(1H-benzotriazol-1-yl)-*N, N, N' , N'*-tetramethyluronium tetrafluoroborate. Still another very practical and useful coupling agent is commercially available O-(7-azabenzotriazol-1-yl)-*N,N,N',N'*-tetramethyluronium hexafluorophosphate.

The coupling reaction is conducted in an inert solvent, e.g. dichloromethane, acetonitrile or dimethylformamide. An excess of a tertiary amine, e.g. diisopropylethylamine, *N*-methylmorpholine or *N*-methylpyrrolidine, is added to maintain the reaction mixture at a pH of about 8. The reaction temperature usually ranges between 0°C and 50°C and the reaction time usually ranges between 15 min and 24 h.

When a solid phase synthetic approach is employed, the C-terminal carboxylic acid is attached to an insoluble carrier (usually polystyrene). These insoluble carriers contain a group that will react with the carboxylic group to form a bond that is stable to the elongation conditions but readily cleaved later. Examples of which are: chloro- or bromomethyl resin, hydroxymethyl resin, and aminomethyl resin. Many of these resins are commercially available with the desired C-terminal amino acid already incorporated. Alternatively, the amino acid can be incorporated on the solid support by known methods Wang, S.-S., J. Am. Chem. Soc., (1973), 95, 1328; Atherton, E.; Shepard, R.C. "Solid-phase peptide synthesis; a practical approach" IRL Press: Oxford, (1989); 131-148. In addition to the foregoing, other methods of peptide synthesis are described in Stewart and Young, "Solid Phase Peptide Synthesis", 2nd ed., Pierce Chemical Co., Rockford, IL (1984); Gross, Meienhofer, Udenfriend, Eds., "The Peptides: Analysis, Synthesis, Biology", Vol. 1, 2, 3, 5, and 9, Academic Press, New-York, (1980-1987); Bodansky et al., "The Practice of Peptide Synthesis" Springer-Verlag, New-York (1984).

The functional groups of the constituent amino acids generally must be protected during the coupling reactions to avoid formation of undesired bonds. The protecting groups that can be used are listed in Greene, "Protective Groups in Organic Chemistry", John Wiley & Sons, New York (1981) and "The Peptides: Analysis, Synthesis, Biology", Vol. 3, Academic Press, New York (1981).

The α-carboxyl group of the C-terminal residue is usually protected as an ester (PG1) that can be cleaved to give the carboxylic acid. Protecting groups that can be used include: 1) alkyl esters such as methyl, trimethylsilylethyl and *t*-butyl, 2) aralkyl esters such as benzyl and substituted benzyl, or 3) esters that can be cleaved by mild base treatment or mild reductive means such as trichloroethyl and phenacyl esters.

The α-amino group of each amino acid to be coupled to the growing peptide chain must be protected (PG2). Any protecting group known in the art can be used. Examples of such groups include: 1) acyl groups such as formyl, trifluoroacetyl, phthalyl, and *p*-toluenesulfonyl; 2) aromatic carbamate groups such as benzyloxycarbonyl (Cbz or Z) and substituted benzyloxycarbonyls, and 9-fluorenylmethyloxycarbonyl (Fmoc); 3) aliphatic carbamate groups such as *tert*-butyloxycarbonyl (Boc), ethoxycarbonyl, diisopropylmethoxycarbonyl, and allyloxycarbonyl; 4) cyclic alkyl carbamate groups such as cyclopentyloxycarbonyl and adamantyloxycarbonyl; 5) alkyl groups such as triphenylmethyl and benzyl; 6) trialkylsilyl such as trimethylsilyl; and 7) thiol containing groups such as phenylthiocarbonyl and dithiasuccinoyl. The preferred α-amino protecting group is either Boc or Fmoc. Many amino acid derivatives suitably protected for peptide synthesis are commercially available.

The α-amino protecting group of the newly added amino acid residue is cleaved prior to the coupling of the next amino acid. When the Boc group is used, the methods of choice are trifluoroacetic acid, neat or in dichloromethane, or HCl in dioxane or in ethyl acetate. The resulting ammonium salt is then neutralized either prior to the coupling or *in situ* with basic solutions such as aqueous buffers, or tertiary amines in dichloromethane or acetonitrile or dimethylformamide. When the Fmoc group is used, the reagents of choice are piperidine or substituted piperidine in dimethylformamide, but any secondary amine can be used. The deprotection is carried out at a temperature between 0°C and room temperature (RT), usually 20-22°C.

Any of the amino acids having side chain functionalities must be protected during the preparation of the peptide using any of the above-described groups. Those skilled in the art will appreciate that the selection and use of appropriate protecting groups for these side chain functionalities depend upon the amino acid and presence of other protecting groups in the peptide. The selection of such protecting groups is important in that the group must not be removed during the deprotection and coupling of the α-amino group.

For example, when Boc is used as the α-amino protecting group, the following side chain protecting groups are suitable: *p*-toluenesulfonyl (tosyl) moieties can be used to protect the amino side chain of amino acids such as Lys and Arg; acetamidomethyl, benzyl (Bn), or *t*-butylsulfonyl moieties can be used to protect the sulfide containing side chain of cysteine; benzyl (Bn) ethers can be used to protect the hydroxy containing side chains of serine, threonine or hydroxyproline; and benzyl esters can be used to protect the carboxy containing side chains of aspartic acid and glutamic acid.

When Fmoc is chosen for the α-amine protection, usually *tert*-butyl based protecting groups are acceptable.

For instance, Boc can be used for lysine and arginine, *tert*-butyl ether for serine, threonine and hydroxyproline, and *tert*-butyl ester for aspartic acid and glutamic acid. Triphenylmethyl (Trityl) moiety can be used to protect the sulfide containing side chain of cysteine.

Once the elongation of the peptide is completed all of the protecting groups are removed. When a liquid phase synthesis is used, the protecting groups are removed in whatever manner is dictated by the choice of protecting groups. These procedures are well known to those skilled in the art.

When a solid phase synthesis is used, the peptide is cleaved from the resin simultaneously with the removal of the protecting groups. When the Boc protection method is used in the synthesis, treatment with anhydrous HF containing additives such as dimethyl sulfide, anisole, thioanisole, or *p*-cresol at 0°C is the preferred method for cleaving the peptide from the resin. The cleavage of the peptide can also be accomplished by other acid reagents such as trifluoromethanesulfonic acid/ trifluoroacetic acid mixtures. If the Fmoc protection method is used, the N-terminal Fmoc group is cleaved with reagents described earlier. The other protecting groups and the peptide are cleaved from the resin using solution of trifluoroacetic acid and various additives such as anisole, etc.

When **Q** is $CH_2$, **a** is 0, **b** is 0 and **B** is $\mathbf{R_{11a}}$N($\mathbf{R_{11b}}$)C(O), the compounds were prepared according to a method analogous to the general method described for the peptides in Scheme I using a readily available succinyl intermediate, *t*-BuO-C(O)$CH_2$CH($\mathbf{R_4}$)-CO-PG1 (e.g. PG1= 2-oxo-4-substituted-oxazolidin-3-yl). This succinyl intermediate can easily be prepared according to the method of Evans'et al (J. Am. Chem. Soc. (1982), 104, 1737) using the appropriate 4-substituted-3-acyl-2-oxazolidinone in the presence of a strong base such as lithium diisopropylamide or sodium bis (trimethylsilyl)amide and *t*-butyl bromoacetate. After cleavage of the 2-oxazolidinone moiety with LiOOH (Evans'et al., Tetrahedron Lett. (1987), 28, 6141), the resulting acid was coupled to the P3-P2-P1-PG1 segment to give *t*-BuO-C(O) -$CH_2$CH($R_4$)-CO-P3-P2-P1-PG1. The latter was treated with hydrogen chloride to selectively convert the terminal t-butyl ester into the corresponding acid that was finally coupled to $\mathbf{R_{11a}}$NH($\mathbf{R_{11b}}$) to give, after removal of the protective group(s), the desired peptide derivative. The amines $\mathbf{R_{11a}}$NH($\mathbf{R_{11b}}$) are commercially available or the synthesis is well known in the art. A specific embodiment of this process is presented in Example 18.

Alternatively, starting with the same succinyl intermediate (*t*-BuO-C(O)$CH_2$CH($R_4$)-CO-PG1), the sequence of reactions can be inverted to introduce first $\mathbf{R_{11a}}$NH($\mathbf{R_{11b}}$) and then P3-P2-P1-PG1 to give the desired peptide derivative.

**Synthesis of capping group B and P6, P5, P4, and P3 moieties**

Different capping groups **B** are introduced to protected P6, P5, P4, the whole peptide or to any peptide segment with an appropriate acyl chloride that is either available commercially or for which the synthesis is well known in the art.

Different **P6** to **P3** moieties are available commercially or the synthesis is well known in the art.

**Synthesis of P2 moieties.**

1. Synthesis of precursors:

A) Synthesis of haloarylmethane derivatives.

The preparation of halomethyl-8-quinoline **IId** was done according to the procedure of K.N. Campbell et al., J. Amer. Chem. Soc., (1946), 68, 1844.

Scheme II

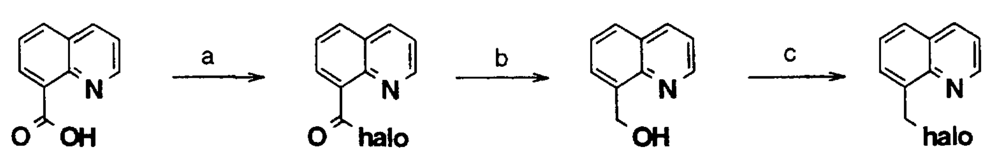


IIa IIb IIc IId

Briefly, 8-quinoline carboxylic acid **IIa** was converted to the corresponding alcohol **IIc** by reduction of the corresponding acyl halide **IIb** with a reducing agent such as lithium aluminium hydride. Treatment of alcohol **IIb** with the appropriate hydrohaloacid gives the desired halo derivative **IId.** A specific embodiments of this process is presented in Example 1.

2. Synthesis of P2:

A) The synthesis of 4-substituted proline (wherein $\mathbf{R^2}$ is attached to the ring via a carbon atom) (with the stereochemistry as shown):

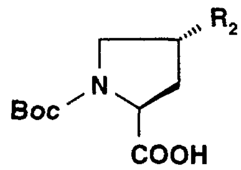

is done as shown in Scheme III according to the procedures described by J. Ezquerra et al. (Tetrahedron, (1993), 38, 8665-8678) and C. Pedregal et al. (Tetrahedron Lett., (1994), 35, 2053-2056).

Scheme III

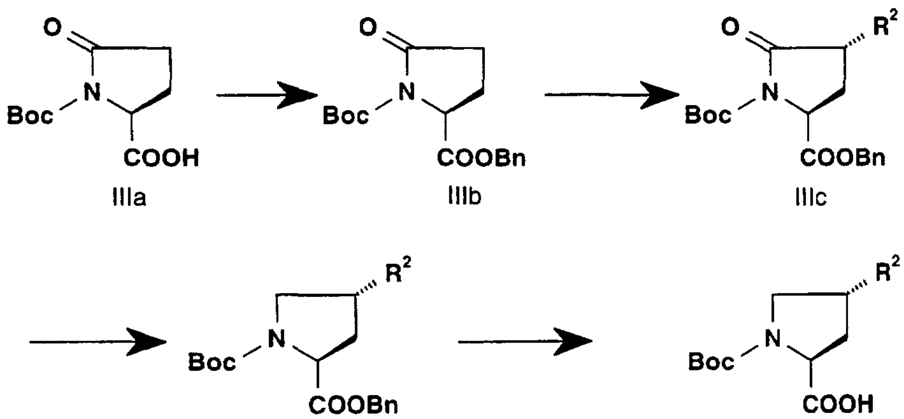

IIIa IIIb IIIc IIId IIIe

Briefly, Boc-pyroglutamic acid is protected as a benzyl ester. Treatment with a strong base such as lithium diisopropylamide followed by addition of an alkylating agent (Br-$\mathbf{R^2}$ or I-$\mathbf{R^2}$) gives the desired compounds **IIIe** after reduction of the amide and deprotection of the ester.

B) The synthesis of O-alkylated 4-(*R*)-hydroxyproline:

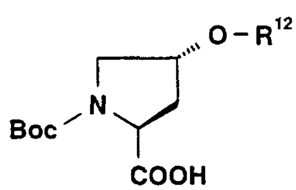

may be carried out using the different processes described below.

B.1) When $\mathbf{R^{12}}$ is aralkyl, the process can be carried out according to the procedure described by E.M. Smith et al. (J. Med. Chem. (1988), 31, 875-885). Briefly, commercially available Boc-4(*R*)-hydroxyproline is treated with a base such as sodium hydride and the resulting alkoxide reacted with an alkylating agent (Br-$\mathbf{R^{12}}$ or I-$\mathbf{R^{12}}$) to give the desired compounds. Specific embodiments of this process are presented in Examples 3 and 4.

B.2) When $\mathbf{R^{12}}$ is aryl, the compounds can be prepared via a Mitsunobu reaction (Mitsunobu (1981), Synthesis,

January, 1-28; Rano et al., (1995), Tet. Lett. 36(22), 3779-3792; Krchnak et al., (1995), Tet. Lett. 36(5), 62193-6196; Richter et al., (1994), Tet. Lett. 35(27), 4705-4706). Briefly, commercially available Boc-4(*S*)-hydroxyproline methyl ester is treated with the appropriate aryl alcohol or thiol in the presence of triphenylphosphine and diethylazodicarboxylate (DEAD) and the resulting ester is hydrolysed to the acid. Specific embodiments of this process are presented in Examples 5 and 6.

Scheme IV

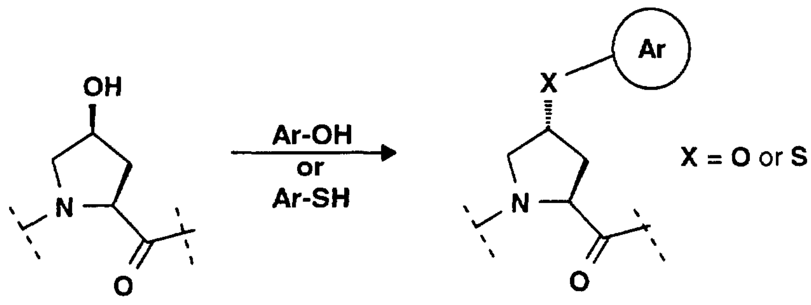


IVa IVb

Alternatively, the Mitsunobu reaction can be produced in solid phase (as shown in Scheme IV). The 96-well block of the Model 396 synthesizer (advanced ChemTech) is provided with aliquots of resin-bound compound **(IVa)** and a variety of aryl alcohols or thiols and appropriate reagents are added. After incubation, each resin-bound product (**IVb**) is washed, dried, and cleaved from the resin.

B.2.a) A Suzuki reaction (Miyaura *et al.*, (1981), Synth. Carom. 11, 513; Sato *et al.*, (1989), Chem. Lett., 1405; Watanabe et al., (1992), Synlett., 207; Takayuki *et al.,* (1993), J. Org. Chem. 58, 2201; Frenette et al., (1994), Tet. Lett. 35(49), 9177-9180; Guiles et al., (1996), J. Org. Chem. 61, 5169-5171) can also be used to further functionalize the aryl substituent.

**Examples**

The present invention is illustrated in further detail by the following non-limiting examples.

Temperatures are given in degrees Celsius. Solution percentages express a weight to volume relationship, and solution ratios express a volume to volume relationship, unless stated otherwise. Nuclear magnetic resonance (NMR) spectra were recorded on a Bruker 400 MHz spectrometer; the chemical shifts (δ) are reported in parts per million. Flash chromatography was carried out on silica gel ($SiO_2$) according to Still's flash chromatography technique (W.C. Still et al., J. Org. Chem. (1978), 43, 2923).

Abbreviations used in the examples include Bn:

benzyl; Boc: *tert*-butyloxycarbonyl {$Me_3COC(O)$}; BSA: bovine serum albumin; CHAPS: 3-[(3-cholamidopropyl)-dimethylammonio]-1-propanesulfonate; DBU: 1,8-diazabicyclo[5.4.0]undec-7-ene; $CH_2Cl_2$= DCM: methylene chloride; DIPEA: diisopropylethylamine; DMAP: dimethylaminopyridine; DCC: 1,3-dicyclohexylcarbodiimide; DME: 1,2-dimethyoxyethane; DMF: dimethylformamide; DMSO: dimethylsulfoxide; DTT: dithiothreitol or threo-1,4-dimercapto-2,3-butanediol; EDTA: ethylenediaminetetraacetic acid; Et: ethyl; EtOH: ethanol; EtOAc: ethyl acetate; $Et_2O$: diethyl ether; HPLC: high performance liquid chromatography; MS: mass spectrometry (MALDI-TOF: Matrix Assisted Laser Disorption Ionisation-Time of Flight, FAB: Fast Atom Bombardment);LAH: lithium aluminum hydride; Me: methyl; MeOH: methanol; MES: (2-{*N*-morpholino}ethane-sulfonic acid); NaHMDS: sodium bis(trimethylsilyl) amide; NMM: *N*-methylmorpholine; NMP: *N*-methylpyrrolidine; Pr: propyl; Succ: 4-hydroxy-1,4-dioxobutyl; PNA: 4-nitrophenylamino or p-nitroanalide; TBAF: tetra-*n*-butylammonium fluoride; TCEP: tris(2-carboxyethyl) phosphine hydrochloride; TFA: trifluoroacetic acid; THF: tetrahydrofuran; TIS: triisopropylsilane; TLC: thin layer chromatography; TMSE: trimethylsilylethyl; Tris/HCl: tris(hydroxymethyl)aminomethane hydrochloride.

**Example 1**

**Synthesis of bromomethyl-8-quinoline (1):**

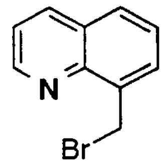

(**1**)

To commercially available 8-quinoline carboxylic acid (2.5 g, 14.4 mmol) was added neat thionyl chloride (10 ml, 144 mmol). This mixture was heated at 80°C for 1 h before the excess thionyl chloride was distilled off under reduced pressure. To the resulting brownish solid was added absolute EtOH (15 mL) which was heated at 80°C for 1 h before being concentrated *in vacuo.* The residue was partitioned between EtOAc and saturated aqueous $NaHCO_3$, and the organic phase dried ($MgSO_4$), filtered and concentrated to give a brownish oil (2.8 g). This material (ca. 14.4 mmol) was added dropwise over 35 min to a LAH (0.76 g, 20.2 mmol)/$Et_2O$ suspension which was cooled to -60°C. The reaction mixture was slowly warmed to -35°C over 1.5 h before the reaction was complete. The reaction was quenched with $MgSO_4.10H_2O$ slowly over 30 min and then wet THF. The mixture was partitioned between $Et_2O$ and 10% aqueous $NaHCO_3$. The organic phase was dried ($MgSO_4$), filtered and concentrated to give a yellowish solid (2.31 g, 80% over 2 steps) corresponding to the alcohol. The alcohol (2.3 g, 11.44 mmol) was dissolved in AcOH/HBr (20 mL, 30% solution from Aldrich) and heated at 70°C for 2.5 h. The mixture was concentrated *in vacuo* to dryness, partitioned between EtOAc (100 mL) and saturated aqueous $NaHCO_3$ before being dried ($MgSO_4$), filtered and concentrated to give the desired compound (**1**) as a brownish solid (2.54 g, 100%).

**Example 2**

**Synthesis of Boc-4(R)-(3-phenylpropyl)proline (2d).**

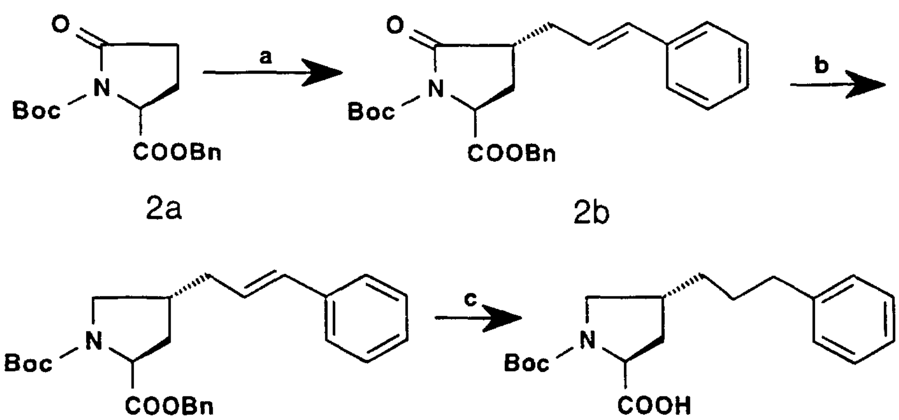


2a 2b

2c 2d

**a) Synthesis of compound 2b:**

To a solution of Boc-pyroglutamic acid benzyl ester **(2a)** (prepared as described by A.L Johnson et al., J. Med.

Chem. (1985), 28, 1596-1602) (500 mg, 1.57 mmol) in THF (10 mL) at -78 °C, was slowly added lithium hexamethydisilylazide (1.72 mL, 1M solution in THF).

After stirring for 1 h at -78°C, cinnamyl bromide (278 µL, 1.88 mmol) was added and the stirring continued for an additional 2 h. The reaction mixture was quenched with saturated ammonium chloride solution and extracted with ethyl ether (3 x 20 mL). The combined organic extracts were dried ($MgSO_4$), filtered and concentrated. The residue was purified by flash column chromatography (8:2 hexane:ethyl acetate) to give compound **2b** as an off-white solid (367 mg, 54% yield). $^1H$ NMR ($CDCl_3$): δ 7.35-7.19 (m, 10H), 6.43 (d, J=15 Hz, 1H), 6.11 (ddd, J=15, J'=J"=8 Hz, 1 H), 5.26 (d, J=16 Hz, 1H), 5.17 (d, J=16 Hz, 1H), 4.59 (dd, J=9.5, J'=2 Hz, 1 H), 2.83-2.70 (m, 2H), 2.41-2.34 (m, 1H), 2.22-2.16 (m, 1H), 2.10-2.02 (m, 1H) 1.42 (s, 9 H).

**b) Synthesis of compound 2c:**

At -78°C, lithium triethylborohydride (1M solution in THF, 1.01 mL, 1.01 mmol) was added to a solution of compound **2b** (367 mg, 0.843 mmol) in THF (5 mL), under a nitrogen atmosphere. After 30 min, the reaction mixture was quenched with saturated aqueous $NaHCO_3$ (2 mL) and warmed to 0°C. 30% $H_2O_2$ (5 drops) was added and the mixture was stirred at 0°C for 20 min. The organic volatiles were removed *in vacuo,* and the aqueous layer was extracted with $CH_2Cl_2$ (3 x 10 mL). The combined organic extracts were dried ($MgSO_4$), filtered and concentrated. To a cold (-78°C) solution of the residue and triethylsilane (134 µL, 0.843 mmol) in $CH_2Cl_2$ (3 mL) boron trifluoride etherate (118 µL,0.927 mmol) was added dropwise under an atmosphere of nitrogen. After 30 min, additional triethylsilane (134 µL) and boron trifluoride etherate (118 µL) were added. After stirring for 2 h at -78°C, the reaction mixture was quenched with saturated aqueous $NaHCO_3$ (2 mL) and extracted with DCM (3 x 10 mL). The combined organic extracts were dried ($MgSO_4$), filtered and concentrated. The crude product was purified by flash column chromatography (8:2 hexane: ethyl acetate) to give compound **2c** as a colorless oil (140 mg, 40% yield). $^1H$ NMR ($CDCl_3$) indicated the presence of two rotamers: δ 7.34-7.22 (m, 10H), 6.38 (d, J=15.5 Hz, 1H), 6.15-6.08 (m, 1H), 5.29-5.07 (m, 2H), 4.44 (d, J=7 Hz, 1/3H), 4.33 (d, J=7 Hz, 2/3H), 3.76 (dd, J=10.5, J'=8.5 Hz, 2/3H), 3.69 (dd, J=10.5, J'=8.5 Hz, 1/3H), 3.13 (dd, J=9, J'=8.5 Hz, 2/3H), 3.05 (dd, J=9, J'=8.5 Hz, 1/3H), 2.47-2.40 (m, 1H), 2.35-2.22 (m, 2H) 2.15-1.85 (m, 2H), 1.45 (s, (3/9) 9H), 1.33 (s, (6/9) 9H).

**c) Synthesis of compound 2d:**

To a solution of compound **20** (140 mg, 0.332 mmol) in ethanol (4 mL) was added 10% palladium on charcoal (30 mg). The mixture was stirred under an atmosphere of hydrogen for 2 h. The catalyst was removed by passing the mixture through a Millipore: Millex - HV 0.45 µm filter. The clear solution was concentrated to give the desired compound **2d** as a colorless oil (115 mg, quant. yield). $^1H$ NMR (DMSO-$d_6$) indicated the presence of two rotamers: δ 7.28-7.14 (m, 5H), 4.33 (br.s, 1H), 4.06-4.10, (m, 1H), 3.56-3.42 (m, 3H), 2.89-2.79 (m, 1H), ), 2.53-2.49 (m, 1H, under DMSO-$d_6$), 2.24-2.10 (m, 1H), 2.03-1.93 (m, 1H), 1.87-1.75 (m, 1H), 1.62-1.45 (m, 2H), 1.38 (s, (3/9) 9H), 1.33 (s, (6/9) 9H).

**Example 3**

**Synthesis of Boc-4(R)-(naphthalen-1-ylmethoxy) proline (3):**

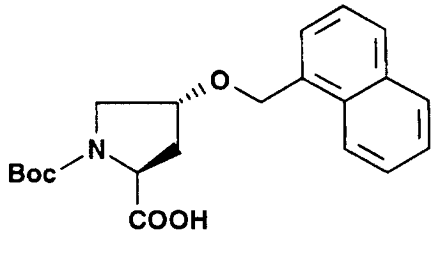

(3)

Commercially available Boc-4(*R*)-hydroxyproline (5.00 g, 21.6 mmol) was dissolved in THF (100 mL) and cooled to 0°C. Sodium hydride (60% dispersion in oil, 1.85 g, 45.4 mmol) was added portionwise over 10 minutes and the suspension was stirred at RT for 1 h. Then, 1-(bromomethyl)naphthalene (8.00 g, 36.2 mmol) (prepared as described in E.A. Dixon et al. Can. J. Chem., (1981), 59, 2629-2641) was added and the mixture was heated at reflux

for 18 h. The mixture was poured into water (300 mL) and washed with hexane. The aqueous layer was acidified with 10% aqueous HCl and extracted twice with ethyl acetate. The organic layers were combined and washed with brine, dried ($MgSO_4$), filtered and concentrated. The residue was purified by flash chromatography (49:49:2 hexane: ethyl acetate: acetic acid) to give the title compound as a colorless oil (4.51 g, 56% yield). $^1H$ NMR (DMSO-$d_6$) indicated the presence of two rotamers: δ 8.05 (m, 1H), 7.94 (m, 1H), 7.29 (d, J=14 Hz, 1H), 7.55-7.45 (m, 4H), 4.96 (m, 2H), 4.26 (br. s, 1H), 4.12 (dd, J=J=8 Hz, 1H), 3.54-3.42 (m, 2H), 2.45-2.34 (m, 1H), 2.07-1.98 (m, 1H) 1.36 (s, (3/9) 9H), 1.34 (s, (6/9) 9H).

**Example 4**

**Synthesis of Boc-4(*R*)-(8-quinoline-methyloxy) proline (4):**

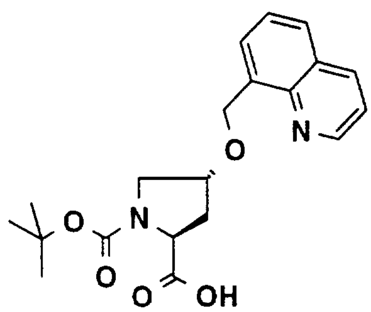

(4)

Boc-4(*R*)-hydroxyproline (1.96 g, 8.5 mmol) in anhydrous THF (20 mL) was added to a suspension of NaH (1.4 g, 60% in oil, 34 mmol) in THF (100 mL). This mixture was stirred 30 min before bromomethyl-8-quinoline from Example 1 (2.54 g, 11.44 mmol) was added in THF (30 mL). The reaction mixture was heated at 70°C (5 h) before the excess NaH was destroyed carefully with wet THF. The reaction was concentrated *in vacuo* and the resulting material was dissolved in EtOAc and $H_2O$. The basic aqueous phase was separated and acidified with 10% aqueous HCl to pH ~5 before being extracted with EtOAc (150 mL). The organic phase was dried ($MgSO_4$), filtered and concentrated to give a brown oil. Purification by flash chromatography (eluent: 10% MeOH/$CHCl_3$) gave the desired compound as a pale yellow solid (2.73 g, 86%). HPLC (97.5%); 1H-NMR (DMSO-$d_6$) shows rotamer populations in a 6:4 ratio, δ 12-11.4 (bs, 1H), 8.92 (2 x d, J = 4.14 and 4.14 Hz, 1H), 8.38 (2 x d, J = 8.27 and 8.27 Hz, 1H), 7.91 (d, J = 7.94 Hz, 1H), 7.77 (d, J = 7.0 Hz, 1H), 7.63-7.54 (m, 2H), 5.14 (2 x s, 2H), 4.32-4.29 (m, 1H), 4.14-4.07 (m, 1H), 3.52-3.44 (m, 2H), 2.43-2.27 (m, 1H), 2.13-2.04 (m, 1H), 1.36 and 1.34 (2 x s, 9H).

**Example 5**

**Preparation of Boc-4(*R*)-(7-chloroquinoline-4-oxo)proline (5):**

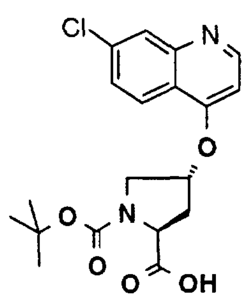

(5)

Commercially available Boc-4(*S*)-hydroxyproline methyl ester (500 mg, 2.04 mmol) and 7-chloro-4-hydroxyquinoline (440 mg, 2.45 mmol) were placed in dry THF (10 mL) at 0°C. Triphenylphosphine (641 mg, 2.95 mmol) was added, followed by slow addition of DIAD (426 mg, 2.45 mmol). The mixture was stirred at RT for 20 h. The reaction mixture was then concentrated, taken up in ethyl acetate and extracted three times with HCl 1N. The aqueous phase was basified with $Na_2CO_3$ and extracted twice with ethyl acetate. The organic layers were combined, dried over $MgSO_4$, filtered and concentrated to give a yellow oil. The oil was purified by flash chromatography to give compound (**5**) methyl ester as a white solid, 498 mg, 58% yield.

This methyl ester (400 mg, 0.986 mmol) was hydrolysed with 1M aqueous sodium hydroxide (1.7 mL, 1.7 mmol) in methanol (4 mL), at 0°C, for 3 h. The solution was concentrated to remove the methanol and neutralised with 1M aqueous HCl. The suspension was concentrated to dryness and taken up in methanol (20 mL), the salts were filtered off and the filtrate concentrated to give the desired compound (**5**) as a white solid, 387 mg, quant. yield.
$^1H$ NMR (DMSO-$d_6$) (ca. 1:1 mixture of rotamers) δ 8.74 (d, J = 5 Hz, 1 H), 8.13-8.09 (m, 1 H), 7.99 and 7.98 (s, 1 H), 7.58 (d, J = 9 Hz, 1 H), 7.02 (d, J = 5 Hz, 1 H), 5.26-5.20 (m, 1 H), 4.10- 4.01 (m, 1 H), 3.81-3.72 (m, 1 H), 3.59 (dd, J = 12, 10 Hz, 1 H), 2.41-2.31 (m, 2 H), 1.34 and 1.31 (s, 9H).

## Example 6

### General procedure for Mitsunobu reaction in solid phase (Scheme IV)

The polymer-bound peptide of general structure **IVa** (0.327 mmoles of peptide per gram of Wang resin) was dried under high vacuum in a desiccator over $P_2O_5$. The 96-well block of the Advanced ChemTech Model 396 synthesizer was furnished with aliquots of **IVa** (120 mg, 0.04 mmol peptide per well) and each sample was washed for 5 min with anhydrous $CH_2Cl_2$ (5x1200 μL) and then with anhydrous THF (5x1500 μL). Anhydrous THF (200 μL) was added to each sample and the synthesizer was temporarily stopped to allow the manual addition of reagents. $Ph_3P$ (5 eq. in 400 μL of anhydrous THF) and diethylazodicarboxylate (DIAD, 5 eq. in 250 μL of anhydrous THF) ) were added to each sample before the addition of a phenol or thiophenol reagent (5 eq, 0.2 mmol, dissolved in 500 μL of anhydrous THF); a library of reagents was used to produce the library of HCV protease inhibitors described in this patent application. After the addition of all reagents, the mixtures were shaken for a total of 4 h with a 10 min delay after each hour. Each resin-bound product was washed with THF (2x1500 μL), DMF (4x1500 μL), isopropanol (4x1500 μL), $CH_2Cl_2$ (4x1500 μL) and finally methanol (2x1500 μL). The sample was dried under vacuum and then treated with 40% TFA in $CH_2Cl_2$ for 1 h in order to cleave the peptide product (general structure **IVb**) from the resin. All products were purified by preparative HPLC on a reversed phase C18 column using a linear solvent gradient from 5% aqueous $CH_3CN$ to 100% $CH_3CN$.

The following description is an example of the further elaboration of the side chain $R_{12}$ at P2 by the application of a biaryl synthesis via Suzuki coupling on a solid support (cf. R. Frenette and R.W. Friesen, Tetrahedron Lett. (1994), 35, 9177).

The precursor, aromatic bromide compound **238** of Table 2, was first synthesized from the polymer-bound tetrapeptide having a *cis*-hydroxyproline at the P2 position and 4-bromophenol using the Mitsunobu protocol described above.

## Example 7

### Suzuki Library of Reactions in Solid Phase Synthesis

All reactions were carried out in 16x100 mm, high pressure screw-cap test tubes with teflon caps, equipped with small magnetic stirring bars. For each reaction, a degassed suspension of the polymer-bound peptide (100 mg of Wang resin with 0.033 mmol of bound peptide) was first added to the test tube, followed by the addition of DME (2 mL), $Pd(Ph_3P)_3$ (~3 mg, 0.05 eq.), $Na_2CO_3$ (70 μL of a 2M solution in $H_2O$, 2.5 eq.) and one of the phenyl boronic acid reagents from our library. The test tubes were flashed with nitrogen gas, sealed and placed in an oil bath at 80°C. All of the reactions were stirred gently and allowed to proceed for 15-18 h. Each resin bound peptide product was subsequently transferred into a plastic filtration tube, washed with DME:$H_2O$ (1:1, 5x 2 mL), DME (5x 2 mL), methanol (5x 2 mL), $CH_3CN$ (5x 2 mL), $CH_2Cl_2$ (5x 2 mL) and dried under high vacuum. Each product was cleaved from the resin by treating the sample with 45% TFA in $CH_2Cl_2$ (1 mL) for 1 hour. All products were purified by preparative HPLC on a reversed phase C18 column using a solvent linear gradient from 5% aqueous $CH_3CN$ to 100% $CH_3CN$.

**Example 8**

**Preparation of a library of Ac-Chg-Val-Hyp(aryl)-Acca-OH**

This compound was synthesized in accordance with the protocol of Example 6 where appropriate peptides were used.

**Example 9**

**Synthesis of Polymer-Bound Compound #246 of Table 2.**

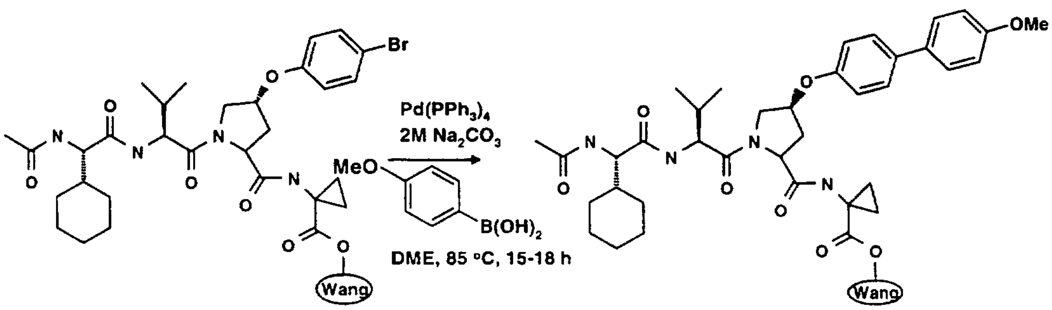


The synthesis of compound **246** was done according to the process Example 7.

Compound 246:

$ES^-$ MS m/z 675.3 [$(M-H)^-$]; ~95% pure by C18 reversed phase HPLC; Mixture of two rotamers in a ratio of ~1:3 based on $^1H$ NMR
$^1H$ NMR of major rotamer (400 MHz, DMSO): δ 8.44 (s, 1H), 7.84 (d, J=8.6 Hz, 1H), 7.82 (d, J=~8.6 Hz, 1H), 7.54 (bd, J=8.3 Hz, 4H), 6.99 (d, J=8.9 Hz, 2H), 6.98 (d, J=8.9 Hz, 2H), 5.11 (bs, 1H), 4.29-4.34 (m, 2H), 4.21 (bt, J=7.8 Hz, 1H), 3.94-4.02 (m, 2H), 3.78 (s, 3H), 2.29-2.33 (m, 2H), 2.15-2.21 (m, 1H), 1.95-1.99 (m, 1H), 1.83 (s, 3H), 1.45-1.70 (m, 8H), 1.33-1.40 (m, 1H), 1.20-1.28 (m, 1H),1.02-1.18 (m, 2H), ~0.9-1.02 (m, 2H), 0.90 (d, J= 6.7 Hz, 3H) 0.84 (d, J=6.7 Hz, 3H).

**Example 10**

**General procedure for coupling reactions done in solution {See also R. Knorr et al., Tetrahedron Letters, 30, 1927 (1989).}**

The reactants, i.e. a free amine (1 eq.) (or its hydrochloride salt) and the free carboxylic acid (1 eq.) were dissolved in $CH_2Cl_2$, $CH_3CN$ or DMF. Under a nitrogen atmosphere, four equivalents of *N*-methylmorpholine and 1.05 equivalents of the coupling agent were added to the stirred solution. After 20 min, one equivalent of the second reactant, i.e. a free carboxylic acid was added. (Practical and efficient coupling reagents for this purpose are (benzotriazol-1-yloxy)tris-(dimethylamino)phosphonium hexafluorophosphate (HOBT) or preferably 2-(1H-benzotriazol-1-yl)-*N,N,N',N'*-tetramethyluronium tetrafluoroborate (TBTU) or O-(7-azabenzotriazol-1-yl)-*N,N,N',N'*-tetramethyluronium tetrafluoroborate (HATU). The reaction was monitored by TLC. After completion of the reaction, the solvent was evaporated under reduced pressure. The residue was dissolved in EtOAc. The solution was washed successively with 10% aqueous citric acid, saturated aqueous $NaHCO_3$ and brine. The organic phase was dried ($MgSO_4$), filtered and concentrated under reduced pressure. When the residue was purified, it was done by flash chromatography as defined above.

**Example 11**

**Synthesis of "tripeptide segment": Ac-Chg-Chg-Pro (4(R)-naphthalen-1-ylmethoxy)-OH (11g)**

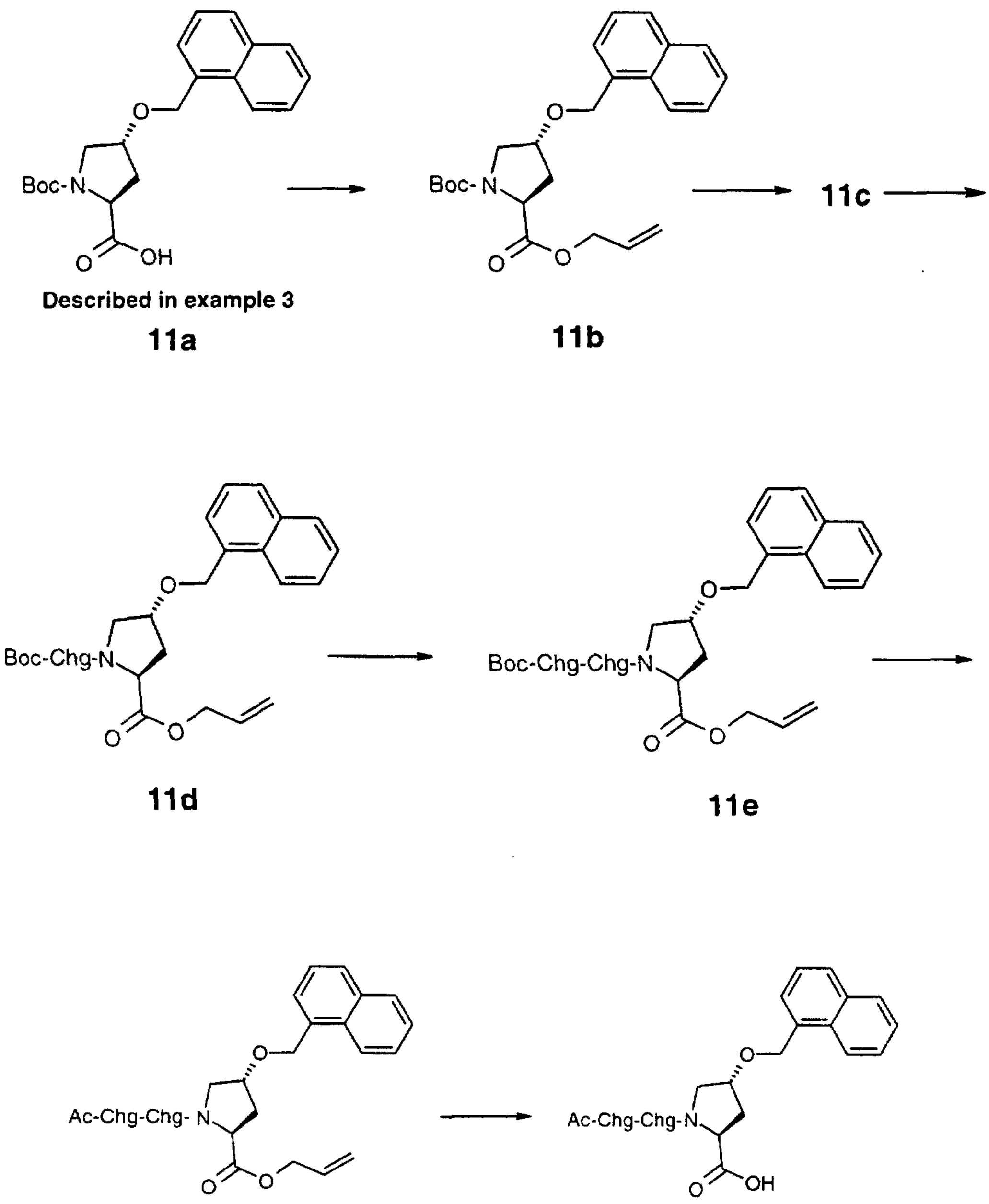

Described in example 3

11a

11b

11c

11d

11e

11f

11g

Compound **11a** (4.45g , 11.98 mmol) was dissolved in anhydrous $CH_3CN$ (60 mL). DBU (2.2 mL , 14.38mmol) and allyl bromide (1.1mL , 13.18 mmol) were added successively and the reaction mixture was stirred 24 h at RT The mixture was concentrated, the resulting oil was diluted with EtOAc and water and successively washed with water (2x) and brine (1x). The EtOAc layer was dried ($MgSO_4$), filtered and evaporated to dryness. The yellow oil was purified by flash chromatography (eluent:hexane:EtOAc;90:10 to 85:15 ) to provide the product **11b** as a yellow oil (2, 4.17g ; 85 % yield ). MS (FAB) 412 $MH^+$

$^1H$ NMR ($CDCl_3$) , mixture of rotamers ca.1:2 , δ (d, J= 8Hz, 1H), 7.87 (d, J= 8Hz, 1H), 7.82 (d, J= 8Hz, 1H), 7.55-7.41 (m, 4H), 5.95-5.85 (m, 1H), 5.34-5.21 (m, 2H), 5.03-4.88 (m, 2H), 4.70-4.56 (m, 2H), 4.48 & 4.39 (t, J= 8, 15Hz, 1H),

4.28-4.23 (m, 1H), 3.81-3.55 (m, 2H), 2.46-2.36 (m, 1H), 2.13-2.05 (m, 1H), 1.44 &1.41 (s, 9H).

Compound **11b** (2.08 g , 5.05 mmol) was treated for 30 min at RT with 4N HCl / dioxane. Evaporation to dryness provided the corresponding amine-HCl as an oil. The amine-HCl **11c** was dissolved in anhydrous DCM (25 mL), NMM (2.2 mL, 20.22 mmol), Boc-Chg-OH · $H_2O$ (1.53 g, 5.56 mmol) and TBTU (1.95 g, 6.07 mmol) were added successively. The reaction mixture was stirred at RT overnight, then, diluted with EtOAc and successively washed with 10% aqueous citric acid (2x), saturated aq. $NaHCO_3$ (2x), water (2x), and brine (1x). The EtOAc layer was dried ($MgSO_4$), filtered and evaporated to dryness to provide the crude product **11d** as a yellowish-white foam (ca 2.78 g, 100% yield). MS (FAB) 551.4 $MH^+$. $^1H$ NMR ($CDCl_3$) δ 8.03(d, J= 8Hz, 1H) , 7.86 (b d, J= 8.5Hz, 1H), 7.84 (d, J= 8Hz, 1H), 7.56-7.40 (m, 4H), 5.92-5.85 (m, 1H), 5.31 (dd, J= 1, 17Hz, 1H), 5.22 (dd, J= 1, 10Hz, 1H), 5.17 (d, J= 9Hz, 1H), 5.05 (d, J= 12Hz, 1H), 4.91 (d, J= 12Hz, 1H), 4.67-4.60 (m, 3H), 4.31-4.27 (m, 2H), 4.16 (b d, J= 11Hz, 1H), 3.71 (dd, J= 4, 11Hz, 1H), 2.47-2.41 (m, 1H), 2.08-1.99 (m, 1H), 1.85-1.63 (m, 5H), 1.44-1.40 (m, 1H), 1.36 (s, 9H), 1.28-1.00 (m, 5H).

The crude dipeptide **11d** (ca. 5.05 mmol) was treated with 4N HCl/dioxane (25 mL) as described for compound **11c.** The crude hydrochloride salt was coupled to Boc-Chg-OH · $H_2O$ (1.53g, 5.55 mmol) with NMM (2.22 mL, 20.22 mmol) and TBTU (1.95 g, 6.07 mmol) in DCM (25 mL) as described for compound **11d** to yield crude tripeptide as a yellow-oil foam. The crude material was purified by flash chromatography (eluent:hexane:EtOAc;80:20 to 75:25) to provide the tripeptide **11e** as a white foam (2.75g ; 79% yield over 2 steps). MS (FAB) 690.5 $MH^+$. $^1H$ NMR ($CDCl_3$), mainly one rotamer, δ 8.06 (d, J= 8Hz, 1H), 7.87 (b d, J= 8.5Hz, 1H), 7.82 (d, J= 8Hz, 1H), 7.57-7.40 (m, 4H), 6.41 (d, J= 8.5Hz, 1H), 5.92-5.84 (m, 1H), 5.31 (dd, J= 1, 17Hz, 1H), 5.23 (dd, J= 1, 10.5Hz, 1H), 5.04 (d, J= 12Hz, 1H), 4.98 (b d, J= 7Hz, 1H), 4.93 (d, J=12Hz, 1H), 4.63-4.58 (m, 4H), 4.29-4.25 (m, 1H), 4.10-4.07 (m, 1H), 3.90-3.84 (m, 1H), 3.72 (dd, J= 4, 11Hz, 1H), 2.48-2.40 (m, 1H), 2.07-1.99 (m, 1H), 1.83-1.55 (m, 12H), 1.43 (s, 9H), 1.23-0.89 (m, 10H)

The tripeptide **11e** (2.75 g, 3.99 mmol) was treated with 4N HCl/dioxane (20 mL) as described for compound **11c.** The crude hydrochloride salt was dissolved in anhydrous DCM (20 mL). NMM (1.75 mL, 15.94 mmol) and acetic anhydride (752 μL, 7.97 mmol) were added successively. The reaction mixture was stirred overnight at RT, then diluted with EtOAc. The organic layer was washed successively with 10% aqueous citric acid (2x), saturated aq. $NaHCO_3$ (2x), water (2x) and brine (1x), dried ($MgSO_4$), filtered, and evaporated to dryness to provide the crude tripeptide **11f** as a white foam (2.48 g, 98% yield).

MS (FAB) 632.4 $MH^{+1}$. $^1H$ NMR ($CDCl_3$), mainly one rotamer, δ 8.06(b d, J= 8Hz, 1H), 7.87 (b d, J= 8Hz, 1H), 7.83 (d, J= 8Hz, 1H), 7.58-7.40 (m, 4H), 6.36 (d, J= 9Hz, 1H), 6.01 (d, J= 9Hz, 1H), 5.94-5.83 (m, 1H), 5.34-5.28 (m, 1H), 5.25-5.21 (m, 1H), 5.05 (d, J= 12Hz, 1H), 4.94 (d, J= 12Hz, 1H), 4.64-4.57 (m, 4H), 4.30-4.23 (m, 2H), 4.12-4.08 (m, 1H), 3.73 (dd, J= 4, 11Hz, 1H), 2.49-2.42 (m, 1H), 2.08-2.01 (m, 1H), 1.99 (s, 3H), 1.85-1.53 (m, 11H), 1.25-0.88 (m, 11H).

The crude tripeptide **11f** (2.48 g, 3.93 mmol)was dissolved in an anhydrous mixture of $CH_3CN$ : DCM (20 mL). Triphenylphosphine (53.5 mg, 0.200 mmol) and tetrakis(triphenylphosphine)-palladium (0) catalyst (117.9 mg, 0.102 mmol) were added successively, followed by pyrrolidine (353.9 μL, 4.24 mmol). The reaction mixture was stirred at room temperature for 18 h. Thereafter, the solvent was evaporated. The residue was dissolved in EtOAc and 10% aqueous citric acid , then, further washes twice more with 10% aqueous citric acid, water (2x), and brine (1x). The organic layer was dried ($MgSO_4$), filtered and evaporated. The crude product was triturated in $Et_2O$: DCM (85:15) to provide after filtration the tripeptide **11g** as a white solid (2.09 g, 90% yield). MS (FAB) 592.4 $MH^+$ 614.3 $(M+Na)^+$. $^1H$ NMR ($CDCl_3$), mainly one rotamer, δ 8.08 (d, J= 8Hz, 1H), 7.93 (b d, J= 9Hz, 1H), 7.88 (b d, J= 8Hz, 1H), 7.82 (d, J= 8Hz, 1H), 7.57-7.41 (m, 4H), 6.47 (d, J= 8.5Hz, 1H), 5.05 (d, J= 12.5Hz, 1H), 4.94 (d, J= 12.5Hz, 1H), 4.73 (t, J= 9.5, 19Hz, 1H), 4.44-4.35 (m, 2H), 4.26 (b s, 1H), 4.19 (d, J= 11.5Hz, 1H), 3.75 (dd, J= 4, 11Hz, 1H), 2.47 (b dd, J= 7.5, 13.5Hz, 1H), 2.20-2.11 (m, 1H), 2.04 (s, 3H), 1.88-1.41 (m, 11H), 1.30-0.80 (11H).

**Example 12**

**Synthesis of "tripeptide segment" -Ac-Chg-Val-Pro(4(*R*)-naphthalen-1-ylmethoxy)-OH (12e).**

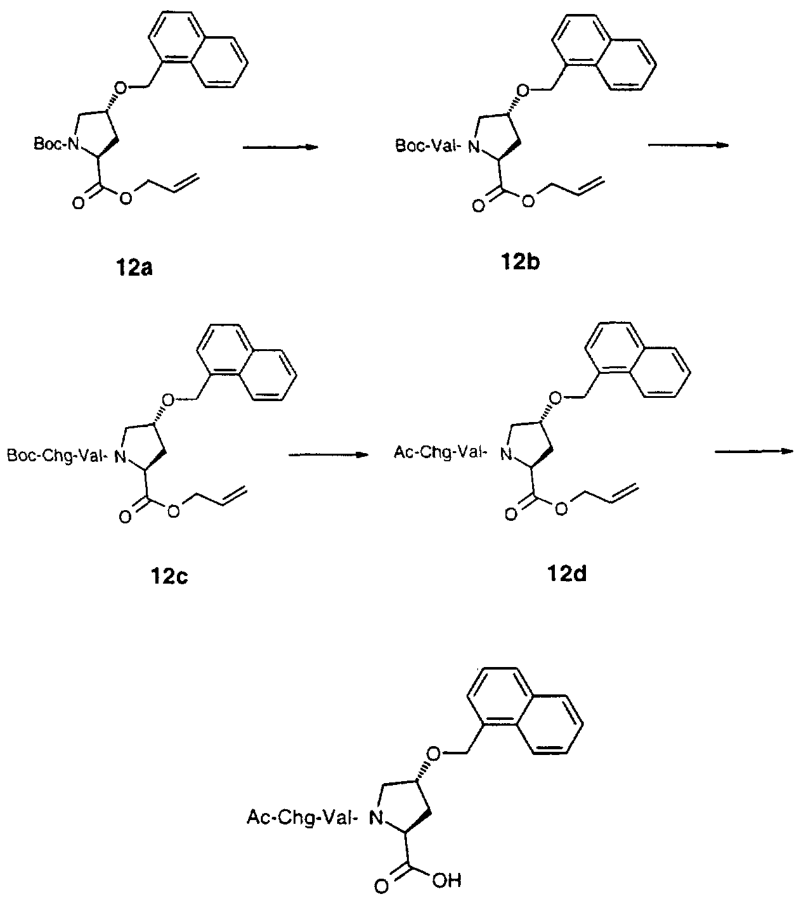

12a 12b

12c 12d

12e

Compound **12a** (2.89 g, 7.02mmol) was treated with 4N HCl/dioxane (30 mL) as described for compound **11c.** The crude hydrochloride salt was coupled to Boc-Val-OH (1.53 g, 7.73 mmol) with NMM (3.1 mL, 28.09 mmol) and TBTU (2.71 g, 8.43 mmol) in DCM (35 mL) for 3.5 h as described for compound 3 to provide the crude dipeptide **12b** as an ivory oil-foam (ca. 3.60 g, 100% yield). MS (FAB) 509.3 $MH^-$ 511.3 $MH^+$ 533.2 $(M+Na)^+$. $^1H$ NMR ( $CDCl_3$) δ 8.04 (b d, J= 8Hz, 1H), 7.87 (b d, J= 7Hz, 1H), 7.82 (d, J= 8Hz, 1H), 7.56-7.40 (m, 4H), 5.93-5.85 (m, 1H), 5.34-5.28 (m, 1H), 5.24-5.19 (m, 2H), 5.04 (d, J= 12Hz, 1H), 4.92 (d, J= 12Hz, 1H), 4.67-4.60 (m, 3H), 4.31-4.26 (m, 2H), 4.11-4.09 (m, 1H), 3.72 (dd, J= 4, 11Hz, 1H), 2.48-2.41 (m, 1H), 2.07-1.99 (m, 1H), 1.44-1.36 (m, 1H), 1.37 (s, 9H), 1.01 (d, J= 7Hz, 3H), 0.93 (d, J= 7Hz, 3H)

The crude dipeptide **12b** (ca. 7.02 mmol) was treated with 4N HCl/dioxane (30 mL) as described for compound **11c**. The crude hydrochloride salt was coupled to Boc-Chg-OH . $H_2O$ (2.13g , 7.73mmol) with NMM (3.1 mL, 28.09 mmol) and TBTU (2.71 g, 8.43 mmol) in $CH_2Cl_2$ (35 mL) as described for compound 3 to provide the crude tripeptide **12c** as an ivory foam (ca. 4.6 g, 100% yield). MS (FAB) 648.5 $MH^-$ 672.4 $(M+Na)^+$. $^1H$ NMR ($CDCl_3$) δ 8.06 (b d, J=8Hz, 1H), 7.87 (b d, J= 7.5 Hz, 1H), 7.82 (b d , J= 8Hz, 1H), 7.57-7.40 (m, 4H), 6.46 (b d, J= 8.5Hz, 1H), 5.94-5.84 (m, 1H), 5.31 (dd, J= 1, 17Hz, 1H), 5.23 (dd, J= 1, 10.5Hz, 1H), 5.03 (d, J= 12Hz, 1H), 5.00-4.97 (m, 1H), 4.93 (d, J=, 12Hz, 1H), 4.63-4.59 (m, 4H), 4.29-4.27 (m, 1H), 4.10-4.07 (m, 1H), 3.92-3.86 (m, 1H), 3.72 (dd, J= 5, 11Hz, 1H), 2.48-2.41 (m, 1H), 2.10-1.99 (m, 1H), 1.76-1.57 (m, 6H), 1.43 (s, 9H), 1.20-0.92 (m, 6H), 1.00 (d, J= 7Hz, 3H), 0.93 (d, J= 7Hz, 3H).

The crude tripeptide **12c** (ca. 7.02mmol) was treated with 4N HCl/dioxane (30 mL) as described for compound **11c.** The crude hydrochloride salt was further treated with acetic anhydride (1.33 mL, 14.05 mmol) and NMM (3.1 mL, 28.09 mmol) in $CH_2Cl_2$ (35 mL) as described for compound **11f.** The crude product was flash purified (eluent:hexane:

EtOAc;30:70) to provide the acetylated protected tripeptide **12d** as a white foam (3.39 g, 81% yield over 3 steps). MS (FAB) 590.3 $MH^-$ 592.4 $MH^+$ 614.4 $(M+Na)^+$

$^1$H NMR ($CDCl_3$), mainly one rotamer, δ 8.06 (d, J= 8Hz, 1H), 7.88 (b d, J= 8Hz, 1H), 7.83 (d, J= 8Hz, 1H), 7.58-7.41 (m, 4H), 6.37 (d, J= 9Hz, 1H), 5.97 (d, J= 8.5 Hz, 1H), 5.94-5.84 (m, 1H), 5.31 (dd, J= 1, 17Hz, 1H), 5.24 (dd, J= 1, 10.5 Hz, 1H), 5.05 (d, J= 12Hz, 1H), 4.94 (d, J= 12Hz, 1H), 4.66-4.57 (m, 4H), 4.31-4.22 (m, 2H), 4.11-4.05 (m, 1H), 3.73 (dd, J= 4.5, 11Hz, 1H), 2.50-2.43 (m, 1H), 2.09-2.01 (m, 2H), 2.00 (s, 3H), 1.68-1.55 (m, 5H), 1.15-0.89 (m, 6H), 0.99 (d, J= 7Hz, 3H), 0.91 (d, J= 7Hz, 3H).

The acetylated tripeptide **12d** (3.39 g, 5.73 mmol) was deprotected by tetrakis(triphenylphosphine)-palladium (0) catalyst (172.1 mg, 0.149 mmol) with triphenylphosphine (78.1 mg, 0.298 mmol) and pyrrolidine (516 μL, 6.19 mmol) in a 1:1 mixture of anhydrous $CH_3CN$ : DCM (30 mL) as described for compound **11g.** The crude light yellow foam product was triturated in $Et_2O$ : DCM (85:15)to provide after filtration the tripeptide **12e** as an off-white solid (3.0 g ; 95% yield). MS (FAB) 550.3 $MH^-$

$^1$H NMR ($CDCl_3$) δ 8.08 (d, J= 8Hz, 1H), 8.04 (b d, J= 9Hz, 1H), 7.88 (b d, J= 7.5Hz, 1H), 7.82 (d, J= 8Hz, 1H), 7.58-7.37 (m, 5H), 5.05 (d, J= 12Hz, 1H), 4.94 (d, J= 12Hz, 1H), 4.61 (t, J= 9.5, 19.5Hz, 1H), 4.46-4.37 (m, 2H), 4.27 (b s, 1H) , 4.17 (d, J= 11Hz, 1H) , 3.74 (dd, J= 4, 11Hz, 1H), 2.49 (b dd, J= 7.5, 13Hz, 1H), 2.17-2.09 (m, 1H), 2.04 (s, 3H), 2.03-1.94 (m, 1H), 1.79 (b d, J= 12.5Hz, 1H), 1.62-1.43 (m, 5H), 1.08-0.85 (m, 5H), 1.00 (d, J= 7Hz, 3H), 0.90 (d, J= 7Hz, 3H).

**Example 13**

**General procedure for coupling reactions done on solid support.**

The synthesis was done on a parallel synthesizer model ACT396 from Advanced ChemTech® with the 96 well block. Typically, 24 peptides were synthesized in parallel using standard solid-phase techniques. The starting Fmoc-Nva-Wang resin and the 1-(Fmoc-amino)cyclopropane carboxylic acid-Wang resin were prepared by the DCC/ DMAP coupling method (Atherton, E; Scheppard, R.C. *Solid Phase Peptide Synthesis, a Practical Approach;* IRL Press: Oxford (1989); pp 131-148). Other amino acid-Wang resins were obtained from commercial sources.

Each well was loaded with 100 mg of the starting resin (approximately 0.05 mmol). The resins were washed successively with 1.5 mL portions of NMP (1 X) and DMF (3 X). The Fmoc protecting group was removed by treatment with 1.5 mL of a 25% v/v solution of piperidine in DMF for 20 min. The resins were washed with 1.5 mL portions of DMF (4 X), MeOH (3 X) and DMF (3 X). The coupling was done in DMF (350 μL), using 400 μL (0.2 mmol) of a 0.5M solution of Fmoc-amino acid/HOBt hydrate in DMF, 400 μL (0.4 mmol) of a 0.5M solution of DIPEA in DMF and 400 μL (0.2 mmol) of a 0.5M solution of TBTU in DMF. After shaking for 1 h, the wells were drained, the resins were washed with 1.5 mL of DMF and the coupling was repeated once more under the same conditions. The resins were then washed as described above and the cycle was repeated with the next amino acid.

The capping groups were introduced in two ways:

1. In the form of a carboxylic acid using the protocol described above (for example acetic acid) or,
2. As an acylating agent such as an anhydride or an acid chloride. The following example illustrates the capping with succinic anhydride: After the Fmoc deprotection and subsequent washing protocol, DMF was added (350 μL), followed by 400 μL each of a DMF solution of succinic anhydride (0.5 M, 0.2 mmol) and DIPEA (1.0 M, 0.4 mmol). The resins were stirred for 2 h and a recoupling step was performed.

At the end of the synthesis the resin was washed with 1.5 mL portions of DCM (3 x), MeOH (3 x), DCM (3 x), and were dried under vacuum for 2 h.

The cleavage from the resin and concomitant side chain deprotection was effected by the addition of 1.5 mL of a mixture of TFA, $H_2O$, DTT and TIS (92.5: 2.5: 2.5: 2.5). After shaking for 2.5 h, the resin was filtered and washed with 1.5 mL of DCM. The filtrates were combined and concentrated by vacuum centrifugation.

Each compound was purified by preparative reversed phase HPLC using a C18 column (22 mm by 500 mm). The product-containing fractions were identified by MALDI-TOF mass spectrometry, combined and lyophilized.

**Example 14**

**Synthesis of compound 210 (Table 2) ; not part of this invention.**

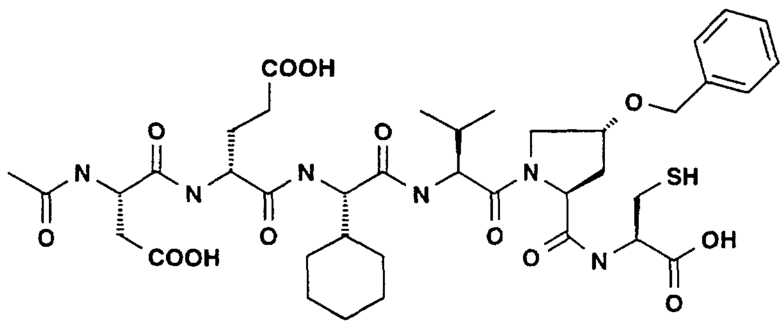

210

Using the experimental protocol described in Example 11 and starting with Fmoc-Cys(Trityl)-Wang resin, the above compound was obtained as a white solid (15.7 mg). MS (FAB) 849.2 ($MH^+$), $^1H$ NMR (DMSO-$d_6$) δ 12.8 (broad s, 1H), 12.1 (broad s, 2H), 8.27 (d, J = 8 Hz, 1H), 8.17 (d, J = 7.5 Hz, 1H), 8.07 (d, J = 8 Hz, 1H), 8.00 (d, J = 8.4 Hz, 1H), 7.75 (d, J = 8.9 Hz, 1H), 7.34-7.27 (m, 5H), 4.54-4.39 (m, 5H), 4.31-4.18 (m, 4H), 4.10 (d, J = 11 Hz, 1H), 3.68 (dd, J = 3.9 Hz, J' = 10.8 Hz, 1H), 2.90-2.82 (m, 1H), 2.78-2.70 (m, 1H), 2.67-2.42 (m, 4H), 2.21-2.17(m, 3H), 2.00-1.85 (m, 3H), 1.83 (s, 3H), 1.80-1.67 (m, 1H), 1.67-1.42 (m, 6H), 1.15-0.95 (m, 4H), 0.88 (dd, J = 6.9 Hz, J' = 8.9 Hz, 6H).

**Example 15**

**Synthesis of compound 215 (Table 2):**

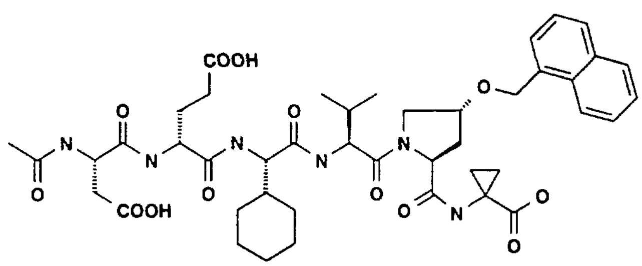

215

The synthesis was carried out as shown below:

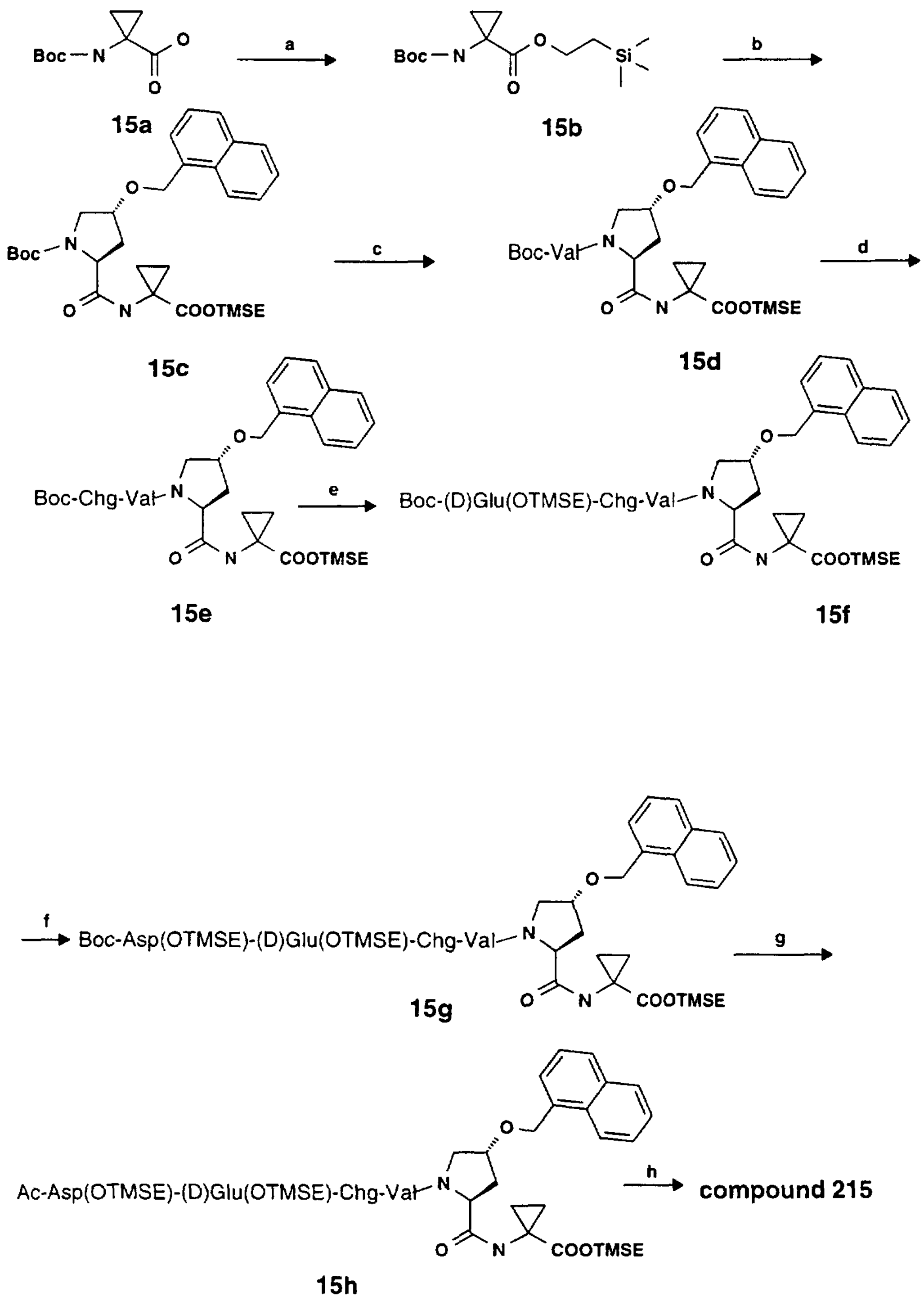


15a 15b 15c 15d 15e 15f 15g 15h

## a) Synthesis of compound 15b:

1-(*N*-*t*-Boc-amino)cyclopropanecarboxylic acid **(15a)** (997 mg, 4.96 mmol) was dissolved in a mixture of anhydrous $CH_2Cl_2$ (25 mL) and THF (10 mL) . The solution was cooled to 0°C , 2-trimethylsilylethanol (0.852 mL , 5.95 mmol), DMAP (121.1 mg, 0.991 mmol) and a DCC/$CH_2Cl_2$ solution (3.65 M; 1.63 mL, 5.95 mmol) were added successively. The reaction mixture was stirred at 0°C for ca.4 h then at RT overnight. The white suspension was filtered through a diatomaceous earth pad. The pad was and rinsed with $CH_2Cl_2$. Filtrate and washing were evaporated to dryness. The residue was diluted with EtOAc and sequentially washed with 10% aqueous citric acid (2x), saturated $NaHCO_3$ (2x), water (2x) and brine (1x). The organic layer was dried ($MgSO_4$), filtered, and evaporated to provide

ester **15b** as an oil (ca.1.5 g, 100%). $^1$H NMR ($CDCl_3$) δ 5.08 (s, 1H), 4.20-4.16 (m, 2H), 1.57-1.43 (m, 2H), 1.45 (s , 9H), 1.17-1.12 (m, 2H), 1.00-0.94 (m, 2H), 0.04 (s, 9H).

**b) Synthesis of compound 15c:**

Ester **15b** (ca.700 mg, 2.33 mmol) was treated for 40 min at RT with 4N HCl/dioxane (11 mL). The solution was concentrated to dryness to provide the amine hydrochloride as a white solid which was then subjected to the reaction conditions described in Example 6. The crude hydrochloride salt (950 mg, 2.55 mmol) and Boc-4(*R*)-(naphthalen-1-ylmethoxy)proline (**3**) were dissolved in anhydrous $CH_2Cl_2$. NMM (1.02 mL, 9.30 mmol) and HATU (1.06 g, 2.79 mmol) were added successively and the mixture was stirred at RT. After 1.75 h, the reaction mixture was diluted with EtOAc and washed sequentially with 10% aq. citric acid (2x), saturated aq. $NaHCO_3$ (2x), water (2x), and brine (1x). The EtOAc layer was dried ($MgSO_4$), filtered and concentrated to dryness to provide the crude dipeptide **15c** as an off-white foam (1.22 g). MS (FAB) 555.4 ($MH^+$). $^1$H NMR ($CDCl_3$) ; mixture of rotamers, δ 8.06-8.04 (m, 1H), 7.87-7.80 (m, 2H), 7.55-7.41 (m, 5H), 4.99-4.93 (m, 2H), 4.45-4.21 (m, 2H), 4.16-4.11 (m, 2H), 3.97-3.45 (m, 2H), 2.70-1.80 (m, 2H), 1.73-1.40 (m, 2H), 1.53 (s, (6/9) 9H), 1.44 (s, (3/9) 9H), 1.20-1.05 (m , 2H), 0.97-0.93 (m, 2H), 0.02 (s, 9H).

**c) Synthesis of compound 15d:**

The crude dipeptide **15d** (ca. 2.20 mmol) was treated with 4N HCl/dioxane (11 mL) 40 min, RT and the resulting hydrochloride salt was coupled to Boc-Val-OH (525 mg, 2.42 mmol) with NMM (968 mL, 8.80 mmol) and HATU (1.00 g, 2.64 mmol) as described for compound **15c** (with the modification of 2.5 h coupling time). The crude tripeptide **15d** was obtained as an off-white foam (1.5 g). MS (FAB) 654.4 ($MH^+$). $^1$H NMR ($CDCl_3$) δ 8.05-8.02 (m, 1H), 7.87-7.80 (m, 2H), 7.55-7.40 (m, 5H), 7.30-7.28 (m, 1H), 5.19-4.62 (m, 4H), 4.41-3.70 (m, 1H), 4.35-4.27 (m, 1H), 4.09-3.95 (m, 1H) 3.73-3.62 (m, 2H), 2.69-2.60 (m, 1H) , 2.14-1.94 (m, 2H), 1.55-1.38 (m, 2H), 1.39 (s, 9H), 1.22-1.18 (m, 1H), 1.11-1.07 (m, 1H), 0.98-0.90 (m, 8H), 0.02 (s, 9H).

**d) Synthesis of compound 15e:**

The crude tripeptide **15d** (ca. 2.20 mmol) was treated with 4N HCl/dioxane (11 mL) 40 min, RT and the resulting hydrochloride salt was coupled to Boc-Chg-OH (622 mg, 2.42 mmol) with NMM (968 mL, 8.80 mmol) and TBTU (847 mg, 2.64 mmol) as described for compound **15c** (with the modifications of using TBTU as a coupling agent and stirring at RT for ca. 64 h prior to work-up). The foam-like residue was purified by flash chromatography (eluent: hexane: EtOAc; 6:4) to provide the tetrapeptide **15e** as a white foam (710.8 mg ; 41% yield over 3 steps). MS (FAB) 793.4 ($MH^+$) . $^1$H NMR ($CDCl_3$) δ 8.07-8.05 (m, 1H), 7.87-7.80 (m, 2H), 7.57-7.41 (m, 4H), 7.35 (s, 1H), 6.72-6.64 (m, 1H), 5.02-4.95 (m, 3H), 4.68-4.62 (m, 2H), 4.43-4.40 (m, 1H), 4.15-4.00 (m, 2H), 3.96-3.93 (m, 2H), 3.68 (dd, J= 11, J'= 5 Hz, 1H), 2.62-2.56 (m, 1H), 2.16-2.00 (m, 2H), 1.70-1.54 (m, 6H), 1.49-1.42 (m, 2H), 1.43 (s, 9H), 1.14-1.02 (m, 5H), 0.95-0.88 (m, 10H), 0.02 (s, 9 H).

**e) Synthesis of compound 15f:**

Tetrapeptide **15e** (168.1 mg, 0.212 mmol) was treated with 4N HCl/dioxane solution (2 mL) and the resulting hydrochloride salt was coupled to Boc-(D)Glu(OTMSE)-OH (81.0 mg, 0.233 mmol) with NMM (94 mL, 0.848 mmol) and TBTU (81.7 mg, 0.254 mmol) as described for compound **15e** (with the modification of 17 h coupling time). The crude pentapeptide **15f** was obtained as an off-white foam (220 mg, 0.212 mmol) . MS (FAB) 1022.8 ($MH^+$) 1044.8 ($MNa^+$) . $^1$H NMR ($CDCl_3$) δ 8.07-8.05 (m, 1H), 7.88-7.81 (m, 2H), 7.57-7.41 (m, 4H), 7.29 (s, 1H), 6.70-6.55 (m, 2H), 5.45-5.35 (m, 1H), 4.99-4.98 (m, 2H) , 4.66-4.57 (m, 2H), 4.44-4.40 (m, 1H), 4.30-4.01 (m , 5H), 3.91 (dd, J= 11, J'= 4 Hz, 1H), 3.76-3.62 (m, 2H), 2.62-2.56 (m , 1H), 2.50-2.30 (m, 3H), 2.18-2.09 (m, 2H), 2.06-1.90 (m, 2H), 1.67-1.53 (m, 4H), 1.50-1.42 (m, 4H), 1.43 (s, 9H ) , 1.14-0.86 (m, 10H), 0.93 (d, J= 7 Hz, 3H), 0.87 (d, J= 7 Hz, 3H), 0.04 (s, 9H), 0.02 (s, 9H).

**f) Synthesis of compound 15g:**

The crude pentapeptide **15f** (ca. 0.212 mmol) was treated with 4N HCl/dioxane solution (2.5 mL) 40 min, RT and the resulting hydrochloride salt was coupled to Boc-Asp(OTMSE)-OH (77.8 mg, 0.233 mmol) with NMM (93 mL, 0.848 mmol) and TBTU (81.7 mg, 0.254 mmol) as described for compound **15e** (with the modification of 2.5 h coupling time). The crude hexapeptide **15g** was obtained as an ivory foam (278 mg, 0.212 mmol). MS (FAB) 1237.5 ($MH^+$) 1259 ($MNa^+$) .

**g) Synthesis of compound 15h:**

The crude hexapeptide **15g** (ca. 0.2 mmol) was treated for 40 min at RT with 2.5 mL 4N HCl/dioxane solution. Concentration to dryness provided the amine hydrochloride as a white solid. The crude hydrochloride salt was dissolved in anhydrous DMF (2.5 mL) and treated successively with pyridine (377 µL, 4.66 mmol) and acetic anhydride (378 µL, 4.01 mmol). The reaction mixture was stirred overnight at RT then poured into brine and extracted with EtOAc (3x). The combined organic layer was washed successively with 10% aqueous citric acid (2x), saturated $NaHCO_3$ (2x), water (2x), and brine (1x). The organic layer was dried ($MgSO_4$), filtered and evaporated to dryness. The foamy residue was purified by flash chromatography (eluent : hexane : EtOAc; 3:7) to provide the acetylated hexapept **15h** as an off-white foam (78.5 mg, 31% yield over 3 steps). MS (FAB) 1179.6 ($MH^+$) 1201.5 ($MNa^+$). $^1H$ NMR ($CDCl_3$) δ 8.11-8.09 (m, 1H), 7.86-7.79 (m, 2H), 7.55-7.41 (m, 5H), 7.28 (s, 1H), 7.02-6.96 (m, 2H), 6.70-6.68 (m, 1H), 5.13-5.10 (m, 1H), 4.96-4.91 (m, 2H), 4.58-4.41 (m, 4H), 4.22-4.08 (m, 8H), 3.77 (dd, J= 10.5, J'= 5 Hz, 1H), 3.09 (dd, J= 18, J'= 4 Hz, 1H), 2.76 (dd, J= 17.5, J'= 8 Hz, 1H), 2.51-2.20 (m, 3H), 2.12-2.08 (m, 2H), 2.09 (s, 3H), 1.73-1.53 (m, 8H), 1.27-1.09 (m, 7H), 1.01-0.85 (m, 8H), 0.98 (d, J= 6.5 Hz, 3H), 0.97(d, J= 6 Hz, 3H), 0.04 (s, 9H), 0.03 (s, 9H), 0.01 (s, 9H).

**h) Synthesis of compound 215:**

The acetylated hexapeptide **15h** (76.5 mg, 0.065 mmol) was dissolved in anhydrous THF (2 mL), a TBAF solution (1M in THF; 389 µL, 0.389 mmol) was added and the mixture was stirred at RT for 16 h. The solution was concentrated under vacuum and the residue was dissolved in glacial acetic acid, filtered through a Millipore®: Millex® -HV 0.45 µm filter unit and injected onto an equilibrated Whatman Partisil® 10-ODS-3 (2.2 x 50cm) C18 reverse phase column. Purification program: Linear Gradient at 15 mL/min, λ 230 nm, program at 5% A for 10 min, 5-30% A in 10 min, at 30% A for 10 min, 30-60% A in 90 min A:0.06% TFA/$CH_3CN$; B:0.06% TFA/$H_2O$. Fractions were analyzed by analytical HPLC. The product collected was lyophilized to provide the hexapeptide acid **215** as a white amorphous solid (26.9 mg; contains 41% by weight of tetrabutylammonium salts, 28% yield). MS (FAB) 879.4 ($MH^+$) 901.3 ($MNa^+$) . In order to remove the tetrabutylammonium salt, the above product (ca.18 mg) was dissolved in EtOAc and washed with 10% HCl (2x). The EtOAc layer was evaporated, then lyophilized with water to provide the salt -free product as a white amorphous solid (3.8 mg , 36% yield). $^1H$ NMR (DMSO-$d_6$) δ 8.39 (s, 1H), 8.10-7.81 (m, 7H), 7.57-7.45 (m, 4H), 5.07-4.87 (m, 2H), 4.55-4.00 (m, 7H), 3.76-3.71 (m, 1H), 2.67-2.62 (m,1H), 2.33-2.10 (m, 3H), 2.05-1.42 (m, 8H), 1.79 (s, 3H) , 1.38-0.71 (m, 1H), 0.89 (d, J= 6.68 Hz, 3H), 0.86 (d, J=6.36 Hz, 3H).

**Example 16**

**Synthesis of compound 214 (Table 2):**

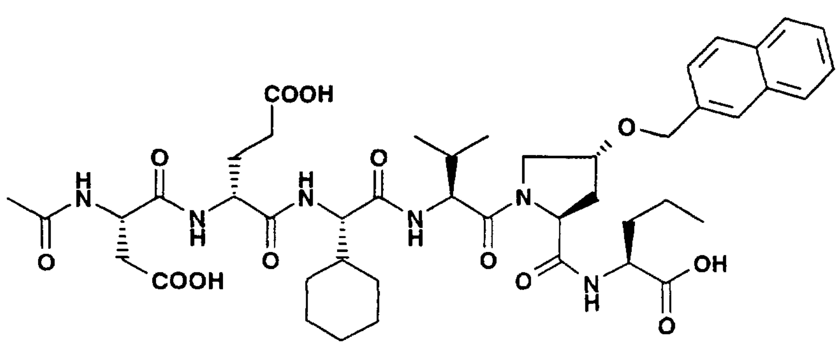

**214**

For the synthesis of compound **214** the procedure described in example 15 was followed, using Boc-4(*R*) -(naphthalen-2-ylmethoxy)proline for the introduction of the P2 fragment and with different protecting groups at the side chain carboxylic acid residues.

The synthesis is described below:

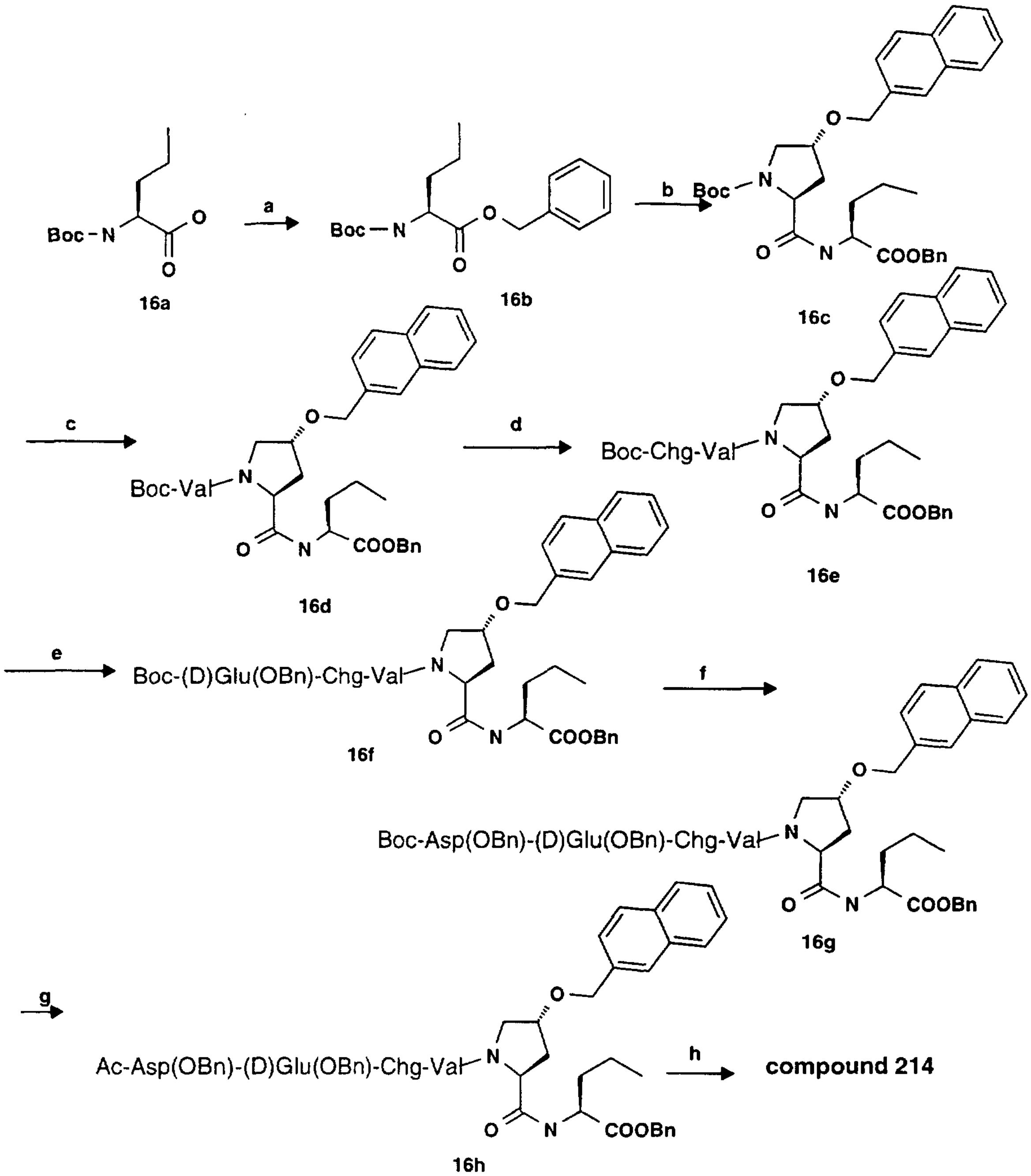


16a 16b 16c 16d 16e 16f 16g 16h

**a) Synthesis of compound 16b:**

At 0°C, benzyl bromide (5.74 mL, 48.3 mmol) was added to a mixture of Boc-norvaline **(16a)** (10.0 g, 46.0 mmol) and DBU (7.57 mL, 50.6 mmol) in acetonitrile (200 mL). After stirring at RT for 20 h, the solution was concentrated and the residue dissolved in ether. The organic solution was washed sequentially with 10% aqueous citric acid (2x), saturated aqueous. $NaHCO_3$ (2x) and brine (1x), dried ($MgSO_4$), filtered and concentrated to give the desired benzyl ester **16b** as a colorless oil (13.7 g, 97% yield). $^1H$ NMR ($CDCl_3$) δ 7.40-7.32 (m, 5H), 5.16 (dd, J = 26.7, J'= 12.4 Hz, 2H), 4.99 (d, J = 7.9 Hz, 1H), 4.35-4.32 (m, 1H), 1.82-1.73 (m, 1H), 1.66-1.57 (m, 1H), 1.43 (s, 9H), 1.41-1.32 (m, 2H), 0.90 (t, J = 7.3 Hz, 3H).

**b, c, d, e, f, g) Synthesis of compound 16h:**

The above Boc-Nva benzyl ester (121 mg, 0.48 mmol) was subjected to the same sequence of reactions as described in example 7. However, for the introduction of P2 (step b) Boc-4(*R*)-(naphthalen-2-ylmethoxy)proline was used. Also, for the introduction of P5 (step e) and P6 (step f) the corresponding Boc-D-Glu-OH and Boc-Asp-OH residues were protected as benzyl esters at the carboxylic acid side chain.

**h) Synthesis of compound 214:**

To a solution of hexapeptide **16h** (ca. 0.210 mmol) in ethanol (3 mL) was added 10% palladium on charcoal (10 mg) and ammonium acetate (10 mg). The mixture was stirred under an atmosphere of hydrogen for 5 h, then filtered through a Millipore®: Millex®-HV 0.45 μm filter unit and injected onto an equilibrated Whatman Partisil® 10-ODS-3 (2.2 x 50 cm) C18 reverse phase column. Purification program: Linear Gradient at 15 mL/min, λ 230 nm, at 5% to 50% A in 60 min A: 0.06% TFA/$CH_3CN$; B: 0.06% TFA/$H_2O$. Fractions were analyzed by HPLC . The collected product was lyophilized to provide **214** as a white solid (20 mg, 0.02 mmol). MS (FAB) 895.5 ($MH^+$). $^1H$ NMR ($CDCl_3$) δ 8.16 (d, J = 7.6 Hz, 1H), 8.11 (d, J = 8 Hz, 1H), 8.09 (d, J = 8 Hz, 1H), 7.98 (d, J = 9 Hz, 1H), 7.91-7.88 (m, 3H), 7.85 (s, 1H), 7.77 (d, J = 9 Hz, 1H), 7.51-7.46 (m, 3H), 4.70 (d, J = 12 Hz, 1H), 4.60 (d, J = 12 Hz, 1H), 4.53-4.45 (m, 2H), 4.33-4.10 (m, 6H), 3.69 (dd, J = 19, J'= 4.4 Hz, 1H), 2.66-2.60 (m, 1H), 2.49-2.43 (m, 1H), 2.21-2.18 (m, 3H), 2.07-1.94 (m, 3H), 1.82 (s, 3H), 1.76-1.33 (m, 10H), 1.04-0.86 (m, 15H).

**Example 17**

**Synthesis of compound 221 (Table 2):**

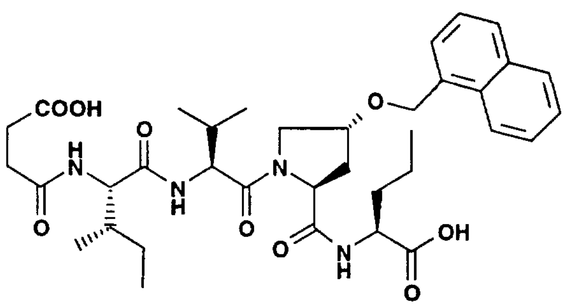

**221**

Mono-benzylsuccinic acid (prepared as described in: Bischoff, V. et al., Chem.Ber. (1902), 35, 4078) (27 mg, 0.134 mmol) was stirred in acetonitrile (2 mL) with TBTU (52 mg, 0.160 mmol) and NMM (47 mg, 0.469 mmol) for 5 min. To this mixture, the hydrochloride salt of the appropriate tetrapeptide (prepared as described for compound **16e** but using isoleucine instead of cyclohexylglycine and 4(*R*)-(naphthalen-1-ylmethoxy)proline instead of a 4(*R*)-(naphthalen-2-ylmethoxy)proline (97.0 mg, 0.134 mmol) was added. The mixture was stirred at RT for 2.5 h. Ethyl acetate was added and the mixture was washed with 10% aqueous citric acid (2x), with saturated aqueous $NaHCO_3$ (2x) and brine (1x), dried ($MgSO_4$), filtered and concentrated to afford the protected tetrapeptide as a yellow oil.

The above compound (ca. 0.134 mmol) was dissolved in ethanol (3 mL) and ammonium acetate (10 mg) and 20% palladium hydroxide on activated carbon (30 mg) were added. The mixture was stirred under 1 atmosphere of hydrogen for 18 h, then filtered through a Millipore® : Millex® -HV 0.45 μm filter unit and injected onto an equilibrated Whatman Partisil 10-ODS-3 (2.2 x 50 cm) C18 reverse phase column. Purification program: Linear Gradient at 15 mL/min, λ 230 nm, 5% A for 10 min, 5-60% A in 60 min (A: 0.06% TFA/$CH_3CN$; B: 0.06% TFA/$H_2O$). Fractions were analyzed by HPLC . The collected product was lyophilized to provide **221** as a white solid (21 mg). MS (FAB) 683 ($MH^+$). $^1H$ NMR (DMSO-$d_6$) δ 8.12 (d, J = 7.6 Hz, 1H), 8.07-8.03 (m, 1H), 7.96-7.81 (m, 4H), 7.59-7.51 (m, 3H), 7.55 (t, J = 8.0 Hz, 1H), 4.90 (d, J =8 Hz, 1H), 4.82 (d, J = 8 Hz, 1H), 4.45 (t, J = 8.0 Hz, 1H), 4.36-4.31 (m, 2H), 4.24-4.12 (m, 3H), 3.74-3.68 (m, 1H), 2.43-2.31 (m, 4H), 2.24-2.18 (m, 1H), 2.01-1.92 (m, 2H), 1.67-1.51 (m, 3H), 1.42-1.32 (m, 3H), 1.14-0.96 (m, 1H), 0.93-0.67 (m, 15H).

**Example 18**

The following description is an example of a compounds of formula I wherein Q is $CH_2C(O)$.

Preparation of compound **413** (Table 4)

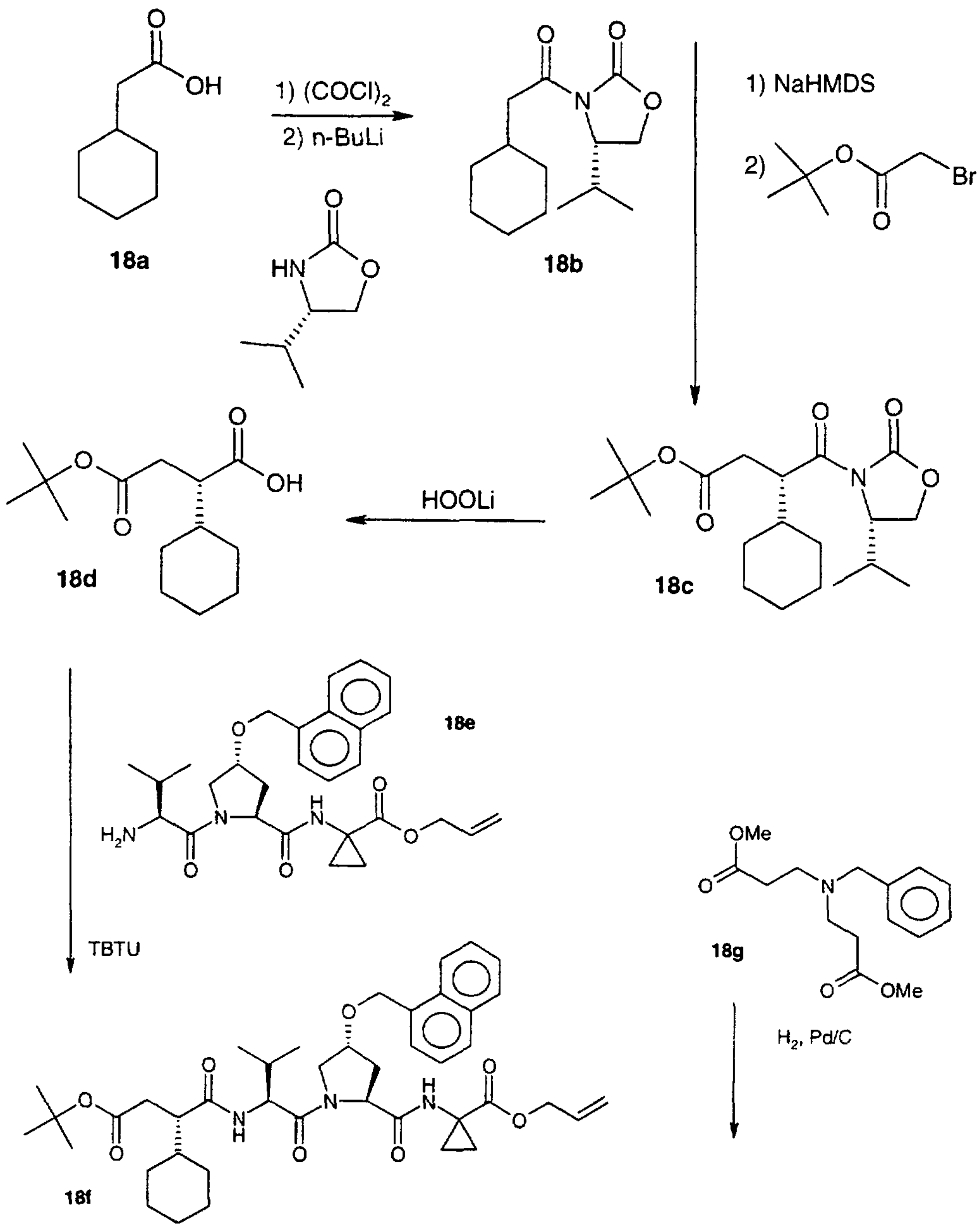


18a 18b 18c 18d 18e 18f 18g

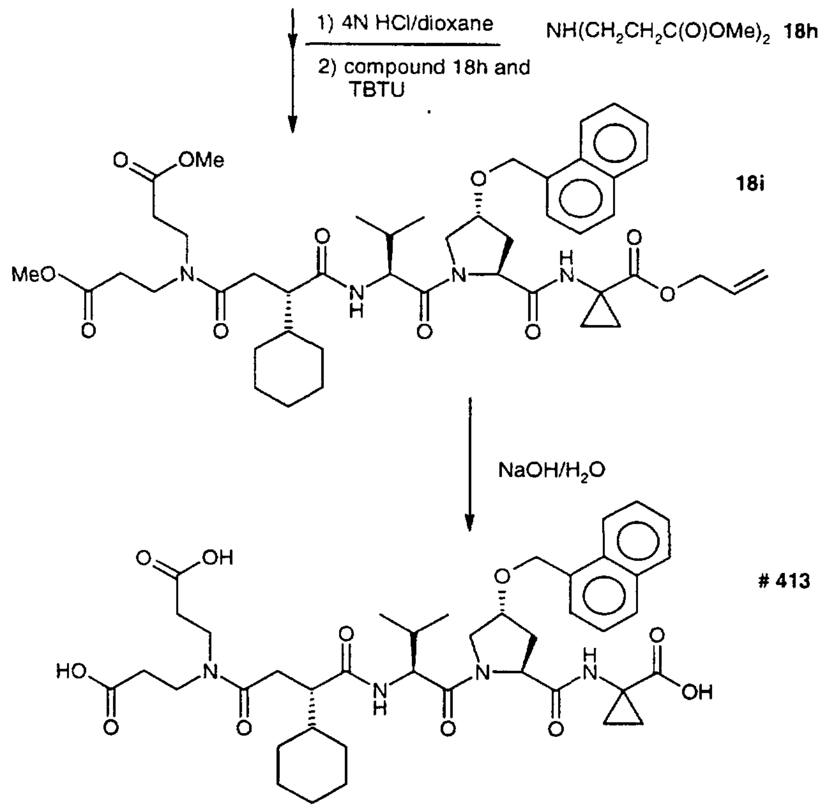


18i

# 413

**Compound 18b**

1) To cyclohexylacetic acid **(18a)** (8g, 56.25 mmol) in DCM (160 mL) at room temperature was added the oxalyl chloride (6.4 mL, 73.14 mmol) and 2 drops of DMF. The reaction mixture was stirred at room temperature for 1h, then concentrated under reduced pressure to give cyclohexylacetyl chloride.

2) The chiral auxiliary, (4S)-(-)-4-isopropyl-2-oxazolidinone, (7.63g, 59.06 mmol) was dissolved in THF (200 mL) and cooled to -78°C. *N*-butyllithium (1.6M) in hexane (36.9 mL, 59.06 mmol) was added slowly (over a 10 min period). The mixture was stirred at -78°C for 30 min (formed a gel). The aformentioned cyclohexylacetyl chloride was added in THF (50 mL) at -78°C. The reaction mixture was stirred at -78°C for 30 min and then at 0°C for 1h. The reaction was quenched by adding an aqueous solution of $NH_4Cl$ (16 mL). The reaction mixture was concentrated under reduced pressure. $Et_2O$ (300 mL) was added. The organic phase was separated and washed with a 10% aqueous solution of citric acid (2 x 200 mL), a saturated aqueous solution of $NaHCO_3$ (2 x 200 mL) and brine (200 mL), dried, filtered and concentrated under reduced pressure. The residue was purified by flash chromatography (silica gel, 40-60µ, 60 x 100 mm, 9/1 → 8/→2, hexane/EtOAc to give compound **18b** as a colorless oil (11.3 g, 79% yield). $^1H$ NMR ($CDCl_3$) δ 4.40-4.36 (m, 1H), 4.20 (dd, J = 8.3Hz, J=9.1Hz, 1H), 4.13 (dd, J = 2.9Hz, 9.1Hz, 1H), 2.86 (dd, J = 6.4Hz, 15.7Hz, 1H), 2.65 (dd, J = 7.1Hz, 15.7Hz, 1H), 2.35-2.27 (m, 1H), 1.83-1.76 (m, 1H), 1.70-1.57 (m, 5H), 1.26-0.90 (m, 5H), 0.85 (d, J = 7.0Hz, 3H), 0.81 (d, J = 6.7Hz, 3H).

**Compound 18c**

To a solution of compound **18b** (11.3 g, 44.68 mmol) in THF (125 mL) at -78°C was added a NaHMDS solution (1M in THF, 49.2 mL, 49.15 mmol). The reaction mixture was stirred at -78°C for 1.5 h. A solution of *tert*-butyl bromoacetate (8.67 mL, 53.62 mmol) in THF (25 mL) was added at -78°C. The mixture was stirred at that temperature for 3h. A saturated aqueous solution of $NH_4Cl$ solution (33 mL) was added slowly. The cold bath was removed and the mixture was stirred at room temperature for 10 min. The THF was removed. EtOAc was added (200 mL). The organic phase was separated, washed serially with a saturated aqueous solution of $NaHCO_3$ (200 mL), $H_2O$ (200 mL), aqueous 1N HCl solution (200 mL) and brine (200 mL), dried ($MgSO_4$), filtered and concentrated under reduced pressure. The

residue was purified by trituration with $Et_2O$ giving compound **18c** as a white solid (12.65g, 77% yield).
$^1H$ NMR (DMSO-$d_6$) δ 4.61-4.53 (m, 3H), 4.27-4.25 (m, 1H), 2.84-2.66 (m, 2H), 2.55-2.41 (m, 1H), 1.89-1.76 (m, 6H), 1.58 (s, 9H), 1.35-1.31 (m, 4H), 1.14-1.04 (m, 7H).

**Compound 18d**

To an ice-cold solution of compound **18c** (12.2 g, 33.28 mmol) in a mixture of THF/$H_2O$ (3/1 mixture, 495 mL/ 165 mL) was added $H_2O_2$ (30%, 15.1 mL, 133.1 mmol), followed by a slow addition of LiOH-$H_2O$ (2.79 g, 66.56 mmol). The reaction mixture was stirred at 0°C for 1 h, then at RT overnight. The mixture was cooled to 0°C and a 1.5N aqueous solution of $Na_2SO_3$ was added slowly to decompose excess peroxide (monitored by KI paper). The mixture was concentrated under reduced pressure, the residual aqueous solution was washed with DCM (2 x 150 mL). The aqueous layer was made acidic with a 10% aqueous solution of citric acid. The mixture was extracted with EtOAc (3 x 200 mL). The combined organic phase were washed with brine (200 mL), dried ($MgSO_4$), filtered and concentrated under reduced pressure. Compound **18d** was obtained as a colorless oil (8.38g, 98% yield).
$^1H$ NMR ($CDI_3$) δ 2.71-2.66 (m, 1H), 2.59 (dd, J = 10.8Hz, 16.0Hz, 1H), 2.36 (dd, J = 3.8Hz, 16.0Hz, 1H), 1.78-1.57 (m, 6H), 1.41 (s, 9H), 1.30-0.98 (m, 5H).

**Compound 18f**

1) The corresponding Boc derivative of compound **18e** (1.63 g, 2.74 mmol) was treated with HCl 4N/dioxane (14 mL, 54.91 mmol) at RT for 1 h. The reaction mixture was concentrated under reduced pressure. A 5% aqueous solution of $Na_2CO_3$ (25 mL) was added to the residue and the resulting solution was stirred vigorously for 5 min. EtOAc was added (75 mL). The two resulting phases were separated. The organic phase was washed with brine (50 mL), dried ($MgSO_4$), filtered and concentrated under reduced pressure to give **18e** which was used as such for the next step.
2) To the amino tripeptide in DMF (5 mL) at RT was added compound 18d (739 mg, 288 mmol) in DMF (5 mL), followed by DIPEA (1.43 mL, 8.24 mmol) and TBTU (502 mg, 2.88 mmol). The reaction mixture was stirred at RT overnight. EtOAc was added (125 mL). The organic phase was separated, washed with a saturated aqueous solution of $NaHCO_3$ (100 mL), $H_2O$ (100 mL) and brine (100 mL), dried ($MgSO_4$), filtered and concentrated under reduced pressure. The residue was purified by flash chromatography (silica gel, 40-60μ, 40 x 125mm, 6/4 → 5/5 hexane/EtOAc) to give the *tert*-butyl ester compound **18f** as a white foam (1.18g, 59% yield). $^1H$ NMR ($CDCl_3$) δ 8,06 (d, J = 8.3Hz, 1H) , 7.86 (d, J = 7.6Hz, 1H), 7.81 (d, J = 8.3Hz, 1H), 7.55-7.40 (m, 4H), 7.35 (s, 1H), 6.28 (d, J = 8.9Hz, 1H), 5.86-5.79 (m, 1H), 5.24 (dd, J = 1.6Hz, 17.2Hz, 1H), 5.17 (dd, J = 1. 3Hz, J = 10.5Hz, 1H), 4.98 (AB*q*, Δv=18.7Hz, J = 12.1Hz, 2H), 4.67-4.51 (m, 4H), 4.41-4.38 (m, 1H), 3.99 (dd, J = 3.8Hz, 10.8Hz, 1H), 2.64-2.59 (m, 2H), 2.42-2.38 (m, 2H), 2.10-1.95 (m, 2H), 1.68-1.53 (m, 9H), 1.43-1.41 (m, 1H), 1.42 (s, 9H), 1.15-1.04 (m, 4H), 0.97-0.91 (m, 8H).

**Compound 18h**

To the commercially available 3-[benzyl-2-methoxycarbonylethyl)amino]propionic acid methyl ester **(18g)** (2 g, 7.16 mmol) in MeOH (24 mL), was added the palladium catalyst (Pd/C 10%, 500 mg, 25 % w/w). The reaction mixture was stirred under a nitrogen atmosphere (balloon) for 18 h. The mixture was filtered through diatomaceous ester and the filter pad was washed with MeOH (20 mL). The MeOH (filtrate plus washing) was evaporated to give 1.2g (89% yield) of compound **18h** as a pale yellow oil. This product was used as such for the next step.

**Compound 18i**

1) The t-butyl ester compound **18f**, (1.18 g, 1.62 mmol) was treated with 4N HCl in dioxane (8.5 mL, 32.4 mol) at RT for 6 h. The mixture was concentrated under reduced pressure, and then co-evaporated with benzene/$Et_2O$ to give 1.04 g of the corresponding acid as a beige foam (95% yield).

2) To the latter acid (200 mg, 0.29 mmol) in DMF (1 mL) at RT was added the amine (compound **18h,** 59 mg, 0.31 mmol) in DMF (2 mL), followed by DIPEA (154 μL, 0.89 mmol) and TBTU (100 mg, 0.31 mmol). The reaction mixture was stirred at RT for 72 h. EtOAc (125 mL) was added. The organic phase was separated, washed with

a saturated aqueous solution of $NaHCO_3$ (75 mL), $H_2O$ (75 mL) and brine (75 mL), dried ($MgSO_4$), filtered and concentrated under reduced pressure. The product was purified by flash chromatography (silica gel, 40-60μ, 20 x 100 mm, 8/2 EtOAc/hexane to give compound **18i** as a yellow oil (82 mg, 33% yield).

MS (ESI) 869.3 $(M+Na)^+$, 845.4 $(M-H)^-$.

**Compound 413**

An aqueous 1M solution of NaOH (774 μL, 0.774 mmol) was added to a solution of compound **18i** (82 mg, 0.097 mmol) in a mixture of THF/MeOH (1/1, 1 mL each). The reaction mixture was stirred at RT for 18 h. $H_2O$ was added (15 mL). The aqueous phase was separated and washed with DCM (3 x 15 mL). The aqueous phase was made acidic (pH 3) by adding an aqueous solution of 1N HCl. The mixture was extracted with EtOAc (3 x 15 mL). The organic phase was washed with brine (25 mL), dried ($MgSO_4$), filtered and concentrated under reduced pressure. The residue was purified by preparative HPLC (5% → 53% MeCN in 60 min) to give compound **413** as a white lyophilized solid (31 mg, 41% yield).

MS (ESI) 779.3 $(M+H)^+$, 801.3 $(M+Na)^+$, 777.3 $(M-H)^-$

$^1H$ NMR (DMSO-$d_6$) δ 8.38 (S, 1H), 8.06 (d, J = 8.3Hz, 1H), 7.93 (d, J = 7.6Hz, 1H), 7.86 (d, J = 8.3Hz, 1H), 7.74 (d, J = 8.6Hz, 1H), 7.57-7.44 (m, 5H), 5.01 (d, J = 12.1Hz, 1H), 4.89 (d, J = 12.1Hz, 1H), 4.35-4.31 (m, 2H), 4.25 (dd, J = 7.9Hz, 8.3Hz, 1H), 4.18 (d, J = 11.1Hz, 1H), 3.80-3.49 (m, 3H), 3.37-3.34 (m, 2H), 2.63-2.61 (m, 2H), 2.56-2.52 (m, 1H), 2.39-2.35 (m, 2H), 2.25-2.20 (m, 2H), 2.05-1.91 (m, 2H), 1.62-1.59 (m, 1H), 1.41-1.22 (m, 5H), 0.96-0.73 (m, 16H).

**Example 19**

**RECOMBINANT HCV NS3 PROTEASE RADIOMETRIC ASSAY**

a) Cloning, expression and purification of the recombinant HCV NS3 protease type 1b

Serum from an HCV-infected patient was obtained through an external collaboration (Bernard Willems MD, Hôpital St-Luc, Montréal, Canada and Dr. Donald Murphy, Laboratoire de Santé Publique du Québec, Ste-Anne de Bellevue, Canada). An engineered full-length cDNA template of the HCV genome was constructed from DNA fragments obtained by reverse transcription-PCR (RT-PCR) of serum RNA and using specific primers selected on the basis of homology between other genotype 1b strains. From the determination of the entire genomic sequence, a genotype 1b was assigned to the HCV isolate according to the classification of Simmonds et al. (J. Clin. Microbiol. (1993), 31, 1493-1503.). The amino acid sequence of the non-structural region, NS2-NS4B, was shown to be greater than 93% identical to HCV genotype 1b (BK, JK and 483 isolates) and 88% identical to HCV genotype 1a (HCV-1 isolate). A DNA fragment encoding the polyprotein precursor (NS3/NS4A/NS4B/NS5A/NS5B) was generated by PCR and introduced into eucaryotic expression vectors. After transient transfection, the polyprotein processing mediated by the HCV NS3 protease was demonstrated by the presence of the mature NS3 protein using Western blot analysis. The mature NS3 protein was not observed with expression of a polyprotein precursor containing the mutation S1165A, which inactivates the NS3 protease, confirming the functionality of the HCV NS3 protease.

The DNA fragment encoding the recombinant HCV NS3 protease (amino acid 1027 to 1206) was cloned in the pET11d bacterial expression vector. The NS3 protease expression in *E. coli* BL21 (DE3)pLysS was induced by incubation with 1 mM IPTG for 3 h at 22°C. A typical fermentation (18 L) yielded approximately 100 g of wet cell paste. The cells were resuspended in lysis buffer (3.0 mL/g) consisting of 25 mM sodium phosphate, pH 7.5, 10% glycerol (v/v), 1 mM EDTA, 0.01% NP-40 and stored at -80°C. Cells were thawed and homogenized following the addition of 5 mM DTT. Magnesium chloride and DNase were then added to the homogenate at final concentrations of 20 mM and 20 μg/mL respectively. After a 25 min incubation at 4°C, the homogenate was sonicated and centrifuged at 15000 x *g* for 30 min at 4°C. The pH of the supernatant was then adjusted to 6.5 using a 1M sodium phosphate solution.

An additional gel filtration chromatography step was added to the 2 step purification procedure described in WO 95/22985. Briefly, the supernatant from the bacterial extract was loaded on a SP HiTrap® column (Pharmacia) previously equilibrated at a flow rate of 2 mL/min in buffer A (50 mM sodium phosphate, pH 6.5, 10% glycerol, 1 mM EDTA, 5 mM DTT, 0.01% NP-40). The column was then washed with buffer A containing 0.15 M NaCl and the protease eluted by applying 10 column volumes of a linear 0.15 to 0.3 M NaCl gradient. NS3 protease-containing fractions were pooled and diluted to a final NaCl concentration of 0.1 M. The enzyme was further purified on a HiTrap® Heparin column (Pharmacia) equilibrated in buffer B (25 mM sodium phosphate, pH 7.5, 10% glycerol, 5 mM DTT, 0.01% NP-40). The sample was loaded at a flow rate of 3 mL/min. The column was then washed with buffer B containing 0.15 M NaCl at a flow rate of 1.5 mL/min. Two step washes were performed in the presence of buffer B containing 0.3 or 1M NaCl. The protease was recovered in the 0.3M NaCl wash, diluted 3-fold with buffer B, reapplied on the HiTrap®

Heparin column and eluted with buffer B containing 0.4 M NaCl. Finally, the NS3 protease-containing fractions were applied on a Superdex 75 HiLoad® 16/60 column (Pharmacia) equilibrated in buffer B containing 0.3 M NaCl. The purity of the HCV NS3 protease obtained from the pooled fractions was judged to be greater than 95% by SDS-PAGE followed by densitometry analysis.

The enzyme was stored at -80°C and was thawed on ice and diluted just prior to use.

b) RECOMBINANT HCV NS3 PROTEASE RADIOMETRIC ASSAY

The substrate used for the HCV NS3 protease radiometric assay, DDIVPC-SMSYTW, is cleaved between the cysteine and the serine residues by the enzyme. The sequence DDIVPC-SMSYTW corresponds to the NS5A/NS5B natural cleavage site in which the cysteine residue in P2 has been substituted for a proline. The peptide substrate DDIVPC-SMSYTW and the tracer biotin-DDIVPC-SMS[$^{125}$I-Y]TW were incubated with the recombinant NS3 protease in the absence or in the presence of inhibitors. The separation of substrate from products was performed by adding avidin-coated agarose beads to the assay mixture followed by filtration. The amount of SMS [$^{125}$I-Y]TW product found in the filtrate (with or without inhibitor) allowed for the calculation of the percentage of substrate conversion and of the percentage of inhibition.

A. Reagents

Tris and Tris-HCl (UltraPure) were obtained from Life Technologies. Glycerol (UltraPure), MES and BSA were purchased from Sigma® . TCEP was obtained from Pierce, DMSO from Aldrich® and NaOH from Anachemia® .

Assay buffer: 50 mM Tris-HCl, pH 7.5, 30% (w/v) glycerol, 2% (w/v) CHAPS, 1 mg/mL BSA, 1 mM TCEP (TCEP added just prior to use from a 1 M stock solution in water).

Substrate: DDIVPC-SMSYTW, 25 μM final concentration (from a 2 mM stock solution in DMSO stored at -20°C to avoid oxidation).

Tracer: reduced mono-iodinated substrate(biotin-DDIVPC-SMS[$^{125}$I-Y)TW) (≈ 1 nM final concentration).

HCV NS3 protease type 1b, 25 nM final concentration (from a stock solution in 50 mM sodium phosphate, pH 7.5, 10% glycerol, 300 mM NaCl, 5 mM DTT, 0.01% NP-40).

B. Protocol

The assay was performed in a 96-well polypropylene plate. Each well contained:

- 20 μL substrate/tracer in assay buffer;
- 10 μL ± inhibitor in 20% DMSO/assay buffer;
- 10 μL NS3 protease 1b.

Blank (no inhibitor and no enzyme) and control (no inhibitor) were also prepared on the same assay plate.

The enzymatic reaction was initiated by the addition of the enzyme solution and the assay mixture was incubated for 60 min at 23°C under gentle agitation. Twenty (20) μL of 0.025 N NaOH were added to quench the enzymatic reaction.

Twenty (20) μL of avidin-coated agarose beads (purchased from Pierce® ) were added in a Millipore® MADP N65 filtration plate. The quenched assay mixture was transferred to the filtration plate, and incubated for 60 min at 23°C under gentle agitation.

The plates were filtered using a Millipore® MultiScreen Vacuum Manifold Filtration apparatus, and 40 μL of the filtrate was transferred to an opaque 96-well plate containing 60 μL of scintillation fluid per well.

The filtrates were counted on a Packard® TopCount instrument using a $^{125}$I-liquid protocol for 1 minute. The %inhibition was calculated with the following equation:

$$100- [(counts_{inh}-counts_{blank}) / (counts_{ctl}-counts_{blank}) \times 100]$$

A non-linear curve fit wich the Hill model was applied to the inhibition-concentration data, and the 50% effective concentration ($IC_{50}$) was calculated by the use of SAS software (Statistical Software System; SAS Institute, Inc., Cary, N.C.).

**Example 20**

**RECOMBINANT HCV NS3 PROTEASE/NS4A COFACTOR PEPTIDE RADIOMETRIC ASSAY**

The enzyme was cloned, expressed and prepared according to the protocol described in Example 19. The enzyme was stored at -80°C, thawed on ice and diluted just prior to use in the assay buffer containing the NS4A cofactor peptide.

The substrate used for the NS3 protease/NS4A cofactor peptide radiometric assay, DDIVPC-SMSYTW (SEQ ID No. 2), is cleaved between the cysteine and the serine residues by the enzyme. The sequence DDIVPC-SMSYTW corresponds to the NS5A/NS5B natural cleavage site in which the cysteine residue in P2 has been substituted for a proline. The peptide substrate DDIVPC-SMSYTW (SEQ ID No. 2) and the tracer biotin-DDIVPC-SMS [$^{125}$I-Y]TW (SEQ ID No. 3) are incubated with the recombinant NS3 protease and the NS4A peptide cofactor KKGSVVIVGRIILSGRK (SEQ ID No. 1) (molar ratio enzyme: cofactor 1:100) in the absence or presence of inhibitors. The separation of substrate from products is performed by adding avidin-coated agarose beads to the assay mixture followed by filtration. The amount of SMS [$^{125}$I-Y] TW product found in the filtrate allows for the calculation of the percentage of substrate conversion and of the percentage of inhibition.

A. Reagents

Tris and Tris-HCl (UltraPure) were obtained from Life Technologies. Glycerol (UltraPure), MES and BSA were purchased from Sigma® . TCEP was obtained from Pierce, DMSO from Aldrich® and NaOH from Anachemia® .

Assay buffer: 50 mM Tris HCl, pH 7.5, 30% (w/v) glycerol, 1 mg/mL BSA, 1 mM TCEP (TCEP added just prior to use from a 1 M stock solution in water).

Substrate: DDIVPCSMSYTW (SEQ ID No. 2), 25 μM final concentration (from a 2 mM stock solution in DMSO stored at -20°C to avoid oxidation).

Tracer: reduced mono iodinated substrate biotin DDIVPC SMS[$^{125}$I Y] TW (SEQ ID No. 3) (~1 nM final concentration) .

HCV NS3 protease type 1b, 25 nM final concentration (from a stock solution in 50 mM sodium phosphate, pH 7.5, 10% glycerol, 300 mM NaCl, 5 mM DTT, 0.01% NP-40).

NS4A Cofactor peptide: KKGSVVIVGRIILSGRK (SEQ ID No. 1), 2.5 μM final concentration (from a 2 mM stock solution in DMSO stored at -20°C).

B. Protocol

The assay was performed in a 96-well polypropylene plate. Each well contained:

- 20 μL substrate/tracer in assay buffer;
- 10 μL ± inhibitor in 20% DMSO/assay buffer;
- 10 μL NS3 protease 1b/NS4 cofactor peptide (molar ratio 1:100).

Blank (no inhibitor and no enzyme) and control (no inhibitor) were also prepared on the same assay plate.

The enzymatic reaction was initiated by the addition of the enzyme/NS4A peptide solution and the assay mixture was incubated for 40 min at 23°C under gentle agitation. Ten (10) μL of 0.5N NaOH were added and 10 μL 1 M MES, pH 5.8 were added to quench the enzymatic reaction.

Twenty (20) μL of avidin-coated agarose beads (purchased from Pierce® ) were added in a Millipore® MADP N65 filtration plate. The quenched assay mixture was transferred to the filtration plate, and incubated for 60 min at 23°C under gentle agitation.

The plates were filtered using a Millipore® MultiScreen Vacuum Manifold Filtration apparatus, and 40 μL of the filtrate was transferred in an opaque 96-well place containing 60 μL of scintillation fluid per well.

The filtrates were counted on a Packard® TopCount instrument using a$^{125}$I-liquid protocol for 1 minute.

The value of $IC_{50}$ was calculated in the same manner as in Example 19.

**Example 21**

**SPECIFICITY ASSAYS**

The specificity of the compounds was determined against a variety of serine proteases: human leukocyte elastase, porcine pancreatic elastase and bovine pancreatic α-chymotrypsin and one cysteine protease: human liver

cathepsin B. In all cases a 96-well plate format protocol using a colorimetric p-nitroanilide (pNA) substrate specific for each enzyme was used. Each assay included a 1 h enzyme-inhibitor pre-incubation at 30°C followed by addition of substrate and hydrolysis to ~30% conversion as measured on a UV Thermomax® microplate reader. Substrate concentrations were kept as low as possible compared to $K_M$ to reduce substrate competition. Compound concentrations varied from 300 to 0.06 μM depending on their potency. The final conditions for each assay were as follows:
50mM Tris-HCl pH 8, 0.5 M $Na_2SO_4$, 50 mM NaCl, 0.1 mM EDTA, 3% DMSO, 0.01% Tween-20 with;
[100 μM Succ-AAPF-pNA (SEQ ID No. 4) and 250 pM α-chymotrypsin], [133 μM Succ-AAA-pNA and 8 nM porcine elastase], [133 μM Succ-AAV-pNA and 8 nM leukocyte elastase]; or
[100 mM $NaHPO_4$ pH 6, 0.1 mM EDTA, 3% DMSO, 1mM TCEP, 0.01% Tween-20, 30 μM Z-FR-pNA and 5 nM cathepsin B (the stock enzyme was activated in buffer containing 20 mM TCEP before use)].

A representative example is summarized below for porcine pancreatic elastase:

In a polystyrene flat-bottom 96-well plate were added using a Biomek® liquid handler (Beckman):

- 40 μL of assay buffer (50 mM Tris-HCl pH 8, 50 mM NaCl. 0.1 mM EDTA);
- 20 μL of enzyme solution (50 mM Tris-/HCl pH 8, 50 mM NaCl, 0.1 mM EDTA, 0.02% Tween-20, 40 nM porcine pancreatic elastase); and
- 20 μL of inhibitor solution (50 mM Tris-HCl, pH 8, 50 mM NaCl, 0.1 mM EDTA, 0.02% Tween-20, 1.5 mM-0.3 μM inhibitor, 15% v/v DMSO).

After 60 min pre-incubation at 30°C, 20 μL of substrate solution (50 mM Tris-HCl, pH 8, 0.5 M $Na_2SO_4$, 50 mM NaCl, 0.1 mM EDTA, 665 μM Succ-AAA-pNA) were added to each well and the reaction was further incubated at 30°C for 60 min after which time the absorbance was read on the UV Thermomax® plate reader. Rows of wells were allocated for controls (no inhibitor) and for blanks (no inhibitor and no enzyme).

The sequential 2-fold dilutions of the inhibitor solution were performed on a separate plate by the liquid handler using 50 mM Tris-HCl pH 8, 50 mM NaCl, 0.1 mM EDTA, 0.02% Tween-20, 15% DMSO. All other specificity assays were performed in a similar fashion.

The percentage of inhibition was calculated using the formula:

$$(1- ( (UV_{inh}-UV_{blank}) / (UV_{ctl}-UV_{blank}))] \times 100$$

A non-linear curve fit with the Hill model was applied to the inhibition-concentration data, and the 50% effective concentration ($IC_{50}$) was calculated by the use of SAS software (Statistical Software System; SAS Institute, Inc., Cary, N.C.).

**Example 22**

**Tables of compounds**

The following tables list $IC_{50}$ values of compounds representative of the invention; the compounds marked with an asterisk (*) are part of this invention.

The following abbreviations are used:

$IC_{50}$: The concentration required to obtain 50% inhibition in the NS3 protease/NS4A cofactor peptide radiometric assay according to example 11; the results marked with an * indicate an $IC_{50}$ value obtained in the recombinant HCV NS3 protease radiometric assay according to example 10; **HLE:** The concentration required to obtain 50% inhibition in the human leukocyte elastase assay; **PPE:** The concentration required to obtain 50% inhibition in the porcine pancreatic elastase assay; **Other:** Figures unmarked indicate the concentration required to obtain 50% inhibition in the bovine pancreatic α-chymotrypsin assay; figures marked with ** indicate the concentration required to obtain 50% inhibition in the human liver cathepsin B assay; **MS:** Mass spectrometric data ($MH^+$ from FAB); **AAA**: amino acid analysis data expressed in % peptide recovery; **Acca:** 1-amino-cyclopropylcarboxylic acid; **Acpe:** 1-amino-cyclopentylcarboxylic acid; **Abu:** 2-aminobutyric acid; **Chg:** cyclohexylglycine (2-amino-2-cyclohexylacetic acid); **Hyp:** 4(*R*)-hydroxyproline; **Hyp(4-Bn):** 4 (*R*)-benzyloxyproline; **Pip:** pipecolic acid (i.e. homoprolyl); **Tbg:** *tert*-butylglycine ; **Ac:** acetyl; **Bn:** benzyl; **O-Bn:** benzyloxy;**DAD**: 3-carboxypropionyl; and **DAE:** 4-carboxybutyryl; **AlGly:** allylglycine (2-amino-4-pentenoic acid); **thioxolle:** L-thionoisoleucine; **Ph:** phenyl; **3I-Ph:** 3-iodophenyl; **4I-Ph:** 4-iodophenyl; **2Br-Ph:** 2-bromophenyl; **3Br-Ph:** 3-bromophenyl; **4Br-Ph:** 4-bromophenyl; **1-NpCH$_2$O:** naphtalen-1-ylmethoxy; **2-NpCH$_2$O:** naphthalen-2-ylmethoxy **3,5-Br$_2$Ph:** 3,5-dibromophenyl.

TABLE 1

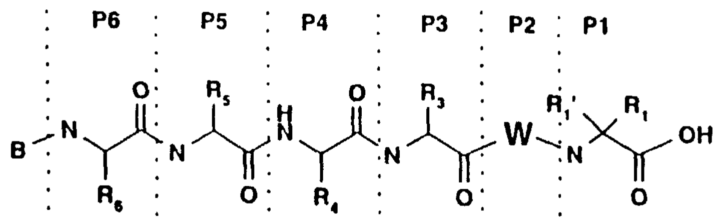


| Compound | B | P6 | P5 | P4 | P3 | W | P1 | $IC_{50}$ (µM) | HLE (µM) | PPE (µM) | Other (µM) | MS ($MH^+$) | AAA (%) |
|---|---|---|---|---|---|---|---|---|---|---|---|---|---|
| 101 | Ac | Asp | Asp | Ile | Val | Pro | Cys | 46 | | | | 703 | 113 |
| 102 | Ac | Glu | Asp | Ile | Val | Pro | Cys | 59 | | | | 717 | 85.4 ± 1.6 |
| 103 | DAD | --- | Asp | Ile | Val | Pro | Cys | 26 | | | | 646 | 100.3 ± 1.8 |
| 104 | Ac | Asp | D-Asp | Ile | Val | Pro | Cys | 8.5 | | | | 703 | 113.85 ± 4.9 |
| 105 | Ac | Asp | D-Glu | Ile | Val | Pro | Cys | 1.5 | | | | 717 | 95.8 ± 0.8 |
| 106 | Ac | Asp | Glu | Ile | Val | Pro | Cys | 16* | | | | 717 | 98.8 ± 2.6 |
| 107 | Ac | Asp | Val | Ile | Val | Pro | Cys | 85* | | | | 687 | 85.9 ± 1.1 |
| 108 | Ac | Asp | Tbg | Ile | Val | Pro | Cys | 31 | | | | 701 | 101.15 ± 1.65 |
| 109 | Ac | Asp | Asp | Val | Val | Pro | Cys | 80* | | | | 689 | 99.2 ± 5 |
| 110 | Ac | Asp | Asp | Chg | Val | Pro | Cys | 24* | | | | 729 | 102.95 ± 3.65 |
| 111 | Ac | Asp | Asp | Tbg | Val | Pro | Cys | 79 | | | | 703 | |
| 112 | Ac | Asp | Asp | Leu | Val | Pro | Cys | 92* | | | | 703 | 109.7 ± 6.9 |
| 113 | Ac | Asp | Asp | Ile | Ile | Pro | Cys | 56* | | | | 717 | 72.4 ± 2.4 |
| 114 | Ac | Asp | Asp | Ile | Chg | Pro | Cys | 50* | | | | 743 | 103.65 ± 3.8 |
| 115 | Ac | Asp | Asp | Ile | Val | Abu | Cys | 58* | | | | 691 | 59.4 ± 2.85 |
| 116 | Ac | Asp | Asp | Ile | Val | Leu | Cys | 16* | | | | 719 | 95.4 ± 1.5 |

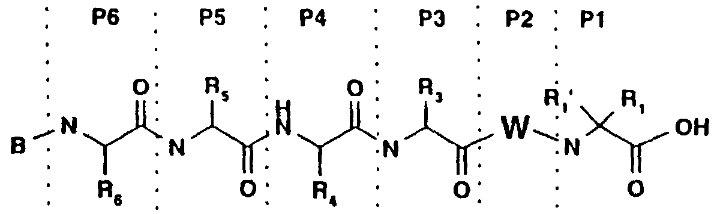

| Compound | B | P6 | P5 | P4 | P3 | W | P1 | $IC_{50}$ (μM) | HLE (μM) | PPE (μM) | Other (μM) | MS ($MH^+$) | AAA (%) |
|---|---|---|---|---|---|---|---|---|---|---|---|---|---|
| 117 | Ac | Asp | Asp | Ile | Val | Phe | Cys | 25* | | | | 753 | 99.6 |
| 118 | Ac | Asp | Asp | Ile | Val | Val | Cys | 133* | | | | 705 | 96.8 ± 1 |
| 119 | Ac | Asp | Asp | Ile | Val | Ile | Cys | 90 | | | | 719 | 87.0 ± 3.0 |
| 120 | Ac | Asp | Asp | Ile | Val | Ala | Cys | 76* | | | | 677 | N.S. |
| 121 | Ac | Asp | Asp | Ile | Val | Hyp(4-Bn) | Cys | 1.7 | | | | 809 | 101 |
| 122 | Ac | Asp | Asp | Ile | Val | Pro | Abu | 315 | | | | 685 | 91.0 ± 4.5 |
| 123 * | Ac | Asp | Asp | Ile | Val | Pro | Nva | 220 | >300 | >300 | | 699 | 107.6 |
| 124 | Ac | Asp | Asp | Ile | Val | Pro | AlGly | 210 | | | | 697 | 106.3 ± 8.2 |
| 125 | Ac | Asp | Asp | Ile | Val | Pro | Acpe | 210 | | | | 711 | 94.02 ± 3.19 |
| 126 * | Ac | Asp | Asp | Ile | Val | Pro | Acca | 45 | | | | 683 | 100.2 |
| 127 * | Ac | Asp | Asp | Ile | Val | Pip | Nva | 605* | | | | 713 | 107 |
| 128 * | Ac | Asp | D-Glu | Ile | Val | Pro | Nva | 7.4 | | | | 713 | 100.9 ± 3.6 |
| 129 * | Ac | Asp | Tbg | Ile | Val | Pro | Nva | 270* | | | | 697 | 99.8 ± 0.6 |
| 130 * | DAD | --- | Asp | Ile | Val | Pro | Nva | 123 | | | | 642 | 107 |
| 131 | Ac | Asp | Glu | Chg | Glu | Glu | Cys | 24 | | | | | |
| 132 * | Ac | Asp | D-Glu | Chg | Glu | Glu | Acca | 36 | | | | | |

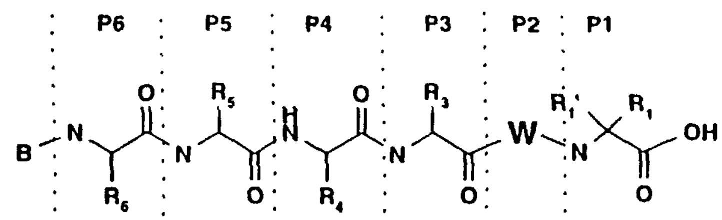

| Compound | B | P6 | P5 | P4 | P3 | W | P1 | $IC_{50}$ (μM) | HLE (μM) | PPE (μM) | Other (μM) | MS ($MH^+$) | AAA (%) |
|---|---|---|---|---|---|---|---|---|---|---|---|---|---|
| 133 * | Ac | Asp | Glu | Chg | Val | Glu(OBn) | Acca | 39 | | | | | |

**TABLE 2**

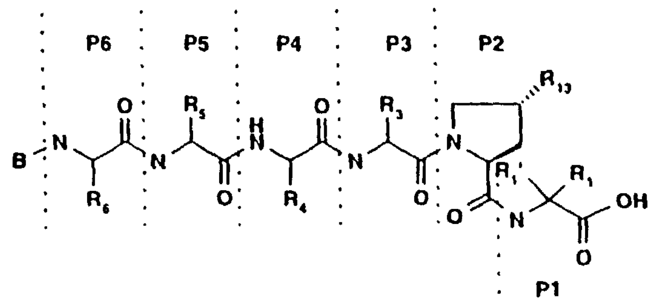

| Comp. | B | P6 | P5 | P4 | P3 | $R_{13}$ | P1 | $IC_{50}$ (μM) | HLE (μM) | PPE (μM) | Other (μM) | MS ($MH^+$) | AAA (%) |
|---|---|---|---|---|---|---|---|---|---|---|---|---|---|
| 201* | Ac | Asp | Asp | Ile | Val | O-Bn | Nva | 7.2 | | | | 805 | 107 |
| 202* | Ac | Asp | D-Val | Ile | Val | O-Bn | Nva | 0.93 | | | | 789 | 103 |
| 203* | Ac | Asp | D-Glu | Ile | Val | O-Bn | Nva | 0.6 | >300 | >300 | >300** | 819 | 96.3 ± 1.7 |
| 204* | Ac | Asp | Asp | Ile | Val | *o*-tolyl-methoxy | Nva | 9.4* | | | | 819 | 95 |
| 205* | Ac | Asp | Asp | Ile | Val | *m*-tolyl-methoxy | Nva | 6.7* | | | | 819 | 98.7 |
| 206* | Ac | Asp | Asp | Ile | Val | *p*-tolyl-methoxy | Nva | 6.4* | | | | 819 | 101.9 |
| 207* | Ac | Asp | Asp | Ile | Val | 1-$NpCH_2O$ | Nva | 0.39 | | | | 855 | 112 |
| 208* | Ac | Asp | Asp | Ile | Val | 2-$NpCH_2O$ | Nva | 0.71 | | | | 855 | 104 |
| 209* | Ac | Asp | Asp | Ile | Val | 4-*tert*-butyl-phenyl)-methoxy | Nva | 2.6 | | | | 861 | 114 |
| 210 | Ac | Asp | D-Glu | Chg | Val | O-Bn | Cys | 0.033 | >300 | >300 | >300 | 849 | 101.7 ± 5.4 |

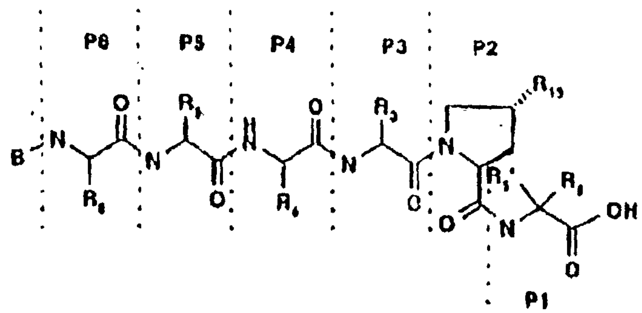

| Comp. | B | P6 | P5 | P4 | P3 | $R_{13}$ | P1 | $IC_{50}$ (μM) | HLE (μM) | PPE (μM) | Other (μM) | MS (MH⁺) | AAA (%) |
|---|---|---|---|---|---|---|---|---|---|---|---|---|---|
| 211* | Ac | Asp | D-Glu | Chg | Val | O-Bn | Nva | 0.12 | | | | 845 | 93.4 ± 2 |
| 212* | Ac | Asp | D-Glu | Ile | Val | O-Bn | Acca | 0.21 | >300 | >300 | | 803 | 99.4 ± 2 |
| 213* | Ac | Asp | D-Glu | Ile | Val | 2-Np$CH_2O$ | Nva | 0.036 | | | | 869 | 101.8 |
| 214* | Ac | Asp | D-Glu | Chg | Val | 2-Np$CH_2O$ | Nva | 0.028 | >300 | >300 | >300<br>>300** | 895 | 104.1 |
| 215* | Ac | Asp | D-Glu | Chg | Val | 1-Np$CH_2O$ | Acca | 0.014 | | | | 879 | --- |
| 216* | Ac | Asp | Asp | Ile | Val | Bn | Nva | 60 | | | | 789 | 100.6 ± 0.8 |
| 217* | Ac | Asp | Asp | Ile | Val | $Ph(CH_2)_3$ | Nva | 3 | | | | 818 | 94.6 ± 3 |
| 218* | Ac | Asp | D-Glu | Ile | Val | O-Bn | Nva | 0.49 | | | | 910 | 111.2 |
| 219* | Ac | --- | Asp | Ile | Val | 1-Np$CH_2O$ | Nva | 2.3 | | | | 740 | 95.7 |
| 220* | DAD | --- | --- | N(Me)Ile | Val | 1-Np$CH_2O$ | Nva | 31 | | | | 697 | --- |
| 221* | DAD | --- | --- | Ile | Val | 1-Np$CH_2O$ | Nva | 22 | | | | 683 | |
| 222* | DAE | --- | --- | Ile | Val | 1-Np$CH_2O$ | Nva | 20 | | | | 698 | N.S. |
| 223* | 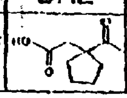 | --- | --- | Ile | Val | 1-Np$CH_2O$ | Nva | 51 | | | | 737 | N.S. |

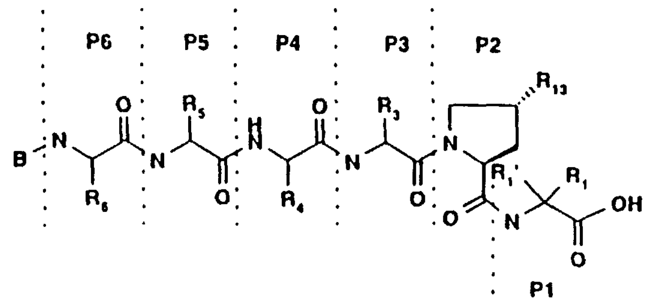

| Comp. | B | P6 | P5 | P4 | P3 | $R_{13}$ | P1 | $IC_{50}$ (μM) | HLE (μM) | PPE (μM) | Other (μM) | MS ($MH^+$) | AAA (%) |
|---|---|---|---|---|---|---|---|---|---|---|---|---|---|
| | | | | | | | | | | | | | |
| 224 * | 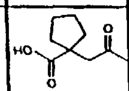 | --- | --- | Ile | Val | 1-Np$CH_2O$ | Nva | 56 | | | | 737 | N.S. |
| 225 * | Ac | --- | --- | Ile | Val | 1-Np$CH_2O$ | Nva | 45 | | | | 929 | --- |
| 226 * | DAE | --- | --- | Chg | Val | 1-Np$CH_2O$ | Acca | 0.76 | | | | 707 | --- |
| 227 * | Ac | --- | --- | Chg | Val | 1-Np$CH_2O$ | Acca | 3 | >600 | | | 635 | |
| 228 * | Ac | --- | --- | Chg | Val | O-Bn | 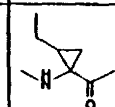 | 35 | >600 | | | 613.4 | |
| 230 * | Ac | Asp | Asp | Ile | Val | Ph$(CH_2)_3$ | Nva | 3.3 | | | | 818 | |
| 231 * | Ac | --- | --- | Chg | Chg | 1-Np$CH_2O$ | Acca | 2.6 | | | | 675.4 | |
| 232 * | Ac$OCH_2$ -C(O) | --- | --- | Chg | Chg | 1-Np$CH_2O$ | Acca | 1.4 | | | | | |
| 233 * | Ac | Asp | Glu | Ile | Val | (3I-Ph)$CH_2O$ | Acca | 0.14 | | | | 929.2 | |

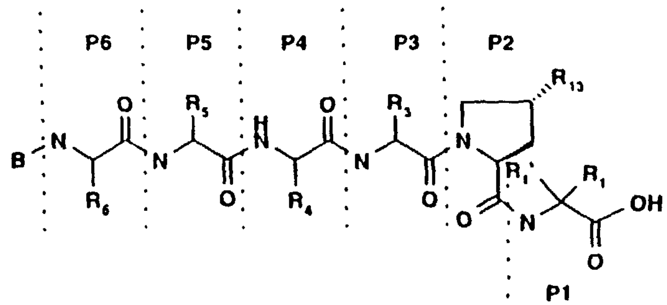

| Comp. | B | P6 | P5 | P4 | P3 | $R_{13}$ | P1 | $IC_{50}$ (μM) | HLE (μM) | PPE (μM) | Other (μM) | MS ($MH^+$) | AAA (%) |
|---|---|---|---|---|---|---|---|---|---|---|---|---|---|
| 234 * | Ac | --- | --- | Chg | Chg | O-Bn | Acca | 41 | | | | | |
| 235 * | Boc | --- | --- | Chg | Chg | 1-$NpCH_2O$ | Acca | 12 | | | | | |
| 236 * | Ac | --- | Gly | thioxo-Ile | Val | 1-$NpCH_2O$ | Nva | 4.0 | | | | 720 (M+Na) | |
| 237 * | DAE | --- | --- | Ile | Val | 1-$NpCH_2O$ | Acca | 5.5 | | | | 598 (M+Na) | |
| 238 * | Ac | --- | --- | Chg | Val | (4Br-Ph)O | Acca | 27 | 195 | | | | |
| 239 * | Ac | --- | --- | Chg | Val | (2Br-Ph)O | Acca | 27 | | | | | |
| 240 * | Ac | --- | --- | Chg | Val | (3Br-Ph)O | Acca | 42 | | | | | |
| 241 * | Ac | --- | --- | Chg | Val | 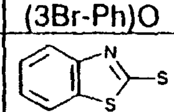 | Acca | 18 | | | | | |
| 242 * | Ac | --- | --- | Chg | Val | (4Br-Ph)S | Acca | 36 | | | | | |
| 243 * | Ac | --- | --- | Chg | Val | 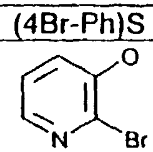 | Acca | 35 | | | | | |

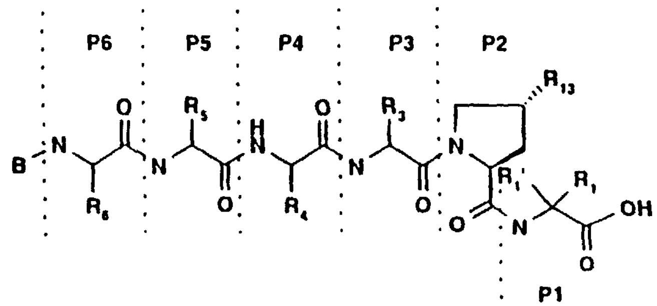

| Comp. | B | P6 | P5 | P4 | P3 | $R_{13}$ | P1 | $IC_{50}$ (μM) | HLE (μM) | PPE (μM) | Other (μM) | MS ($MH^+$) | AAA (%) |
|---|---|---|---|---|---|---|---|---|---|---|---|---|---|
| 244 * | Ac | --- | --- | Chg | Val | 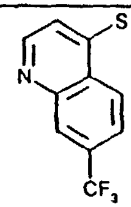 | Acca | 10 | | | | | |
| 245 * | Ac | --- | --- | Chg | Val | 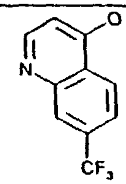 | Acca | 5.0 | | | | | |

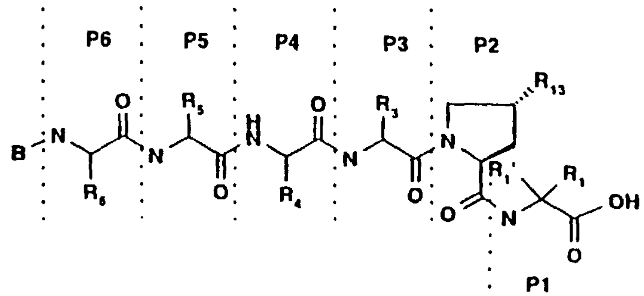

| Comp. | B | P6 | P5 | P4 | P3 | $R_{13}$ | P1 | $IC_{50}$ (μM) | HLE (μM) | PPE (μM) | Other (μM) | MS ($MH^+$) | AAA (%) |
|---|---|---|---|---|---|---|---|---|---|---|---|---|---|
| 246* | Ac | --- | --- | Chg | Val | 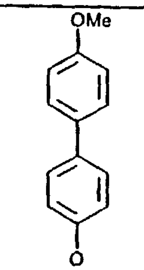 | Acca | 33 | | | | | |
| 247* | Ac | Asp | Asp | Ile | Val | $Ph(CH_2)_2$ | Nva | 10 | | | | 803.6 | 119±1 |
| 248* | Ac | --- | --- | Chg | Chg | 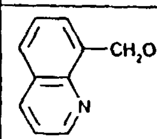 | Acca | 3.6 | | | | | |
| 249* | Ac | --- | --- | Chg | Val | (4I-Ph)O | Acca | 9.7 | | | | | |

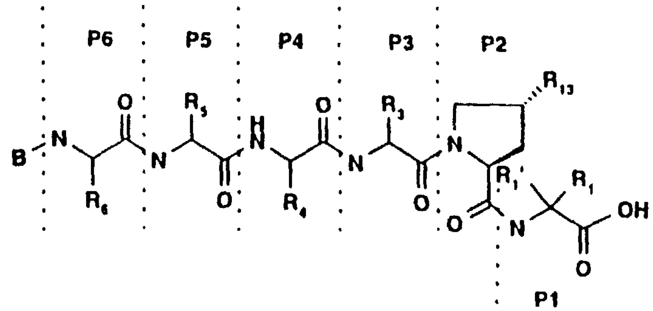

| Comp. | B | P6 | P5 | P4 | P3 | $R_{13}$ | P1 | $IC_{50}$ (μM) | HLE (μM) | PPE (μM) | Other (μM) | MS ($MH^+$) | AAA (%) |
|---|---|---|---|---|---|---|---|---|---|---|---|---|---|
| 250* | Ac | --- | --- | Chg | Val | 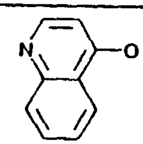 | Acca | 4.5 | | | | | |
| 251* | Ac | --- | --- | Chg | Val | 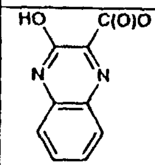 | Acca | 13 | | | | | |
| 252* | Ac | --- | --- | Chg | Val | 1-$NpCH_2O$ | Nva | 20 | | | | 651.4 | 91±1 |
| 253* | Ac | --- | --- | Chg | Val | 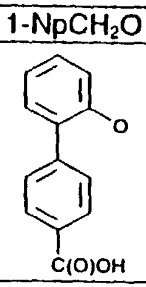 | Acca | 28 | | | | | |

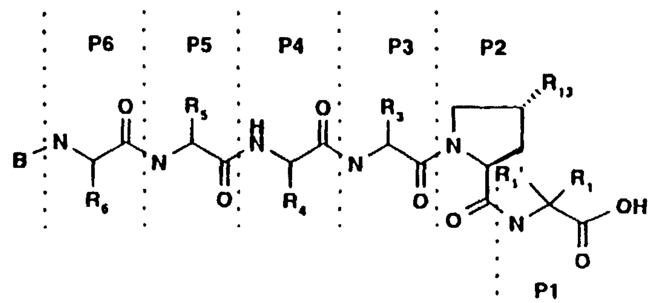

| Comp. | B | P6 | P5 | P4 | P3 | $R_{13}$ | P1 | $IC_{50}$ (μM) | HLE (μM) | PPE (μM) | Other (μM) | MS ($MH^+$) | AAA (%) |
|---|---|---|---|---|---|---|---|---|---|---|---|---|---|
| 254* | Ac | --- | --- | Chg | Val | 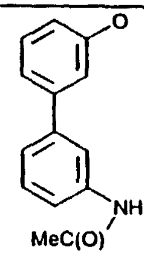 | Acca | 5.1 | | | | | |
| 255* | Ac | --- | --- | Chg | Val | 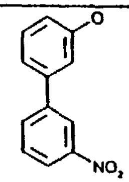 | Acca | 4.5 | | | | | |
| 256* | Ac | --- | --- | Chg | Val | 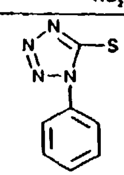 | Acca | 11 | | | | | |

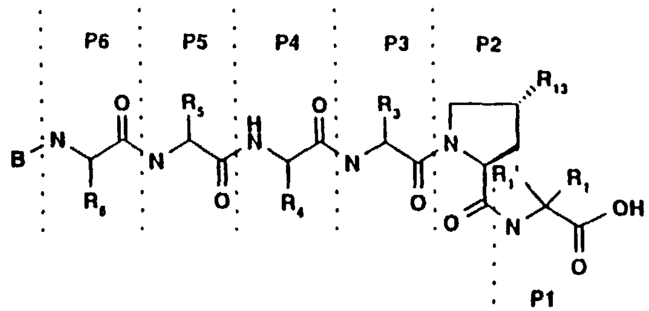

| Comp. | B | P6 | P5 | P4 | P3 | $R_{13}$ | P1 | $IC_{50}$ (µM) | HLE (µM) | PPE (µM) | Other (µM) | MS ($MH^+$) | AAA (%) |
|---|---|---|---|---|---|---|---|---|---|---|---|---|---|
| 257* | Ac | --- | --- | Chg | Val | 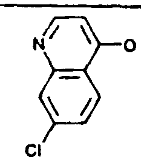 | Acca | 2.2 | >300 | | | | |
| 258* | Ac | --- | --- | Chg | Val | 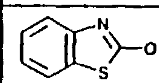 | Acca | 16 | | | | | |
| 259* | Ac | --- | --- | Chg | Val | 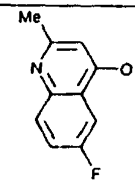 | Acca | 28 | | | | | |
| 260 | Ac | Asp | D-Glu | Ile | Val | O-Bn | Cys | 0.18 | | | | | |
| 261 | Ac | --- | --- | Chg | Val | O-Bn | Cys | 28 | | | | | |
| 262* | Ac | --- | --- | Ile | Val | 1-Np$CH_2$O | Acca | 40 | | | | 631 (M+Na) | |

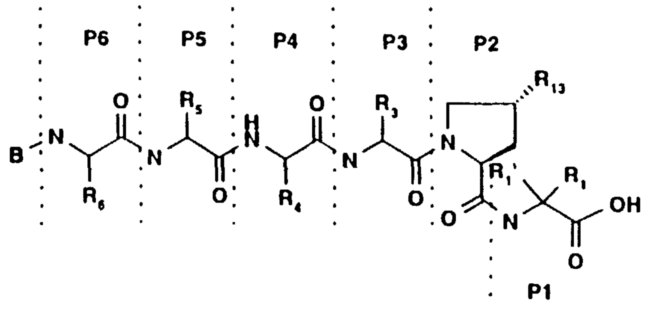

| Comp. | B | P6 | P5 | P4 | P3 | $R_{13}$ | P1 | $IC_{50}$ (μM) | HLE (μM) | PPE (μM) | Other (μM) | MS ($MH^+$) | AAA (%) |
|---|---|---|---|---|---|---|---|---|---|---|---|---|---|
| 263* | 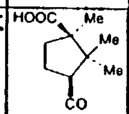 | --- | --- | Ile | Val | 1-NpCH$_2$O | Acca | 17 | | | | 771 (M+Na) | |
| 264* | 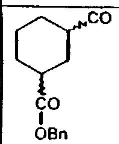 | --- | --- | Ile | Val | 1-NpCH$_2$O | Acca | 6.4 | | | | 811 | |
| 265* | 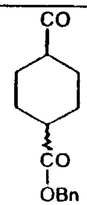 | --- | --- | Ile | Val | 1-NpCH$_2$O | Acca | 10 | | | | 811 | |
| 266* | 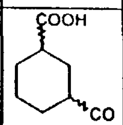 | --- | --- | Ile | Val | 1-NpCH$_2$O | Acca | 9.7 | | | | 721.4 | |

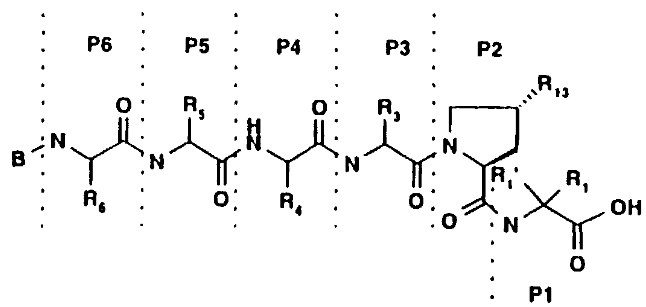

| Comp. | B | P6 | P5 | P4 | P3 | $R_{13}$ | P1 | $IC_{50}$ (µM) | HLE (µM) | PPE (µM) | Other (µM) | MS ($MH^+$) | AAA (%) |
|---|---|---|---|---|---|---|---|---|---|---|---|---|---|
| 267✱ | 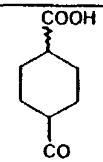 | --- | --- | Ile | Val | 1-Np$CH_2$O | Acca | 12 | | | | 721.4 | |
| 268✱ | Ac | --- | --- | Chg | Val | (3Br-Ph)$CH_2$O | Acca | 24 | | | | 665.1 | |
| 269✱ | 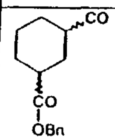 | --- | --- | Chg | Val | 1-Np$CH_2$O | Acca | 2.2 | | | | 835.5 (M-H) | |
| 270✱ | 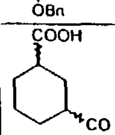 | --- | --- | Chg | Val | 1-Np$CH_2$O | Acca | 2.0 | | | | 745 (M-H) | |

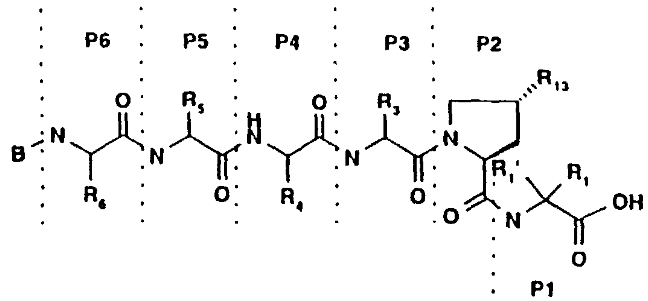

| Comp. | B | P6 | P5 | P4 | P3 | $R_{13}$ | P1 | $IC_{50}$ (μM) | HLE (μM) | PPE (μM) | Other (μM) | MS ($MH^+$) | AAA (%) |
|---|---|---|---|---|---|---|---|---|---|---|---|---|---|
| 271 * | 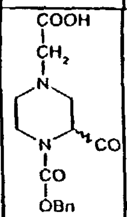 | --- | --- | Chg | Val | 1-$NpCH_2O$ | Acca | 3.8 | | | | | |
| 272 * | Ac | --- | --- | Chg | Val | (3,5-$Br_2$-Ph)$CH_2O$ | Acca | 27 | | | | | |
| 273 * | Ac | Asp | Asp | Ile | Val | H | Nva | 17.5 | | | | | |
| 274 | Ac | Asp | D-Val | Ile | Val | H | Cys | 7.6 | | | | | |
| 275 * | Ac | --- | --- | Chg | Val | 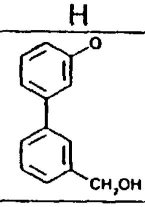 | Acca | 6.2 | | | | | |

TABLE 3

P6 P5 P4 P3 P2 P1

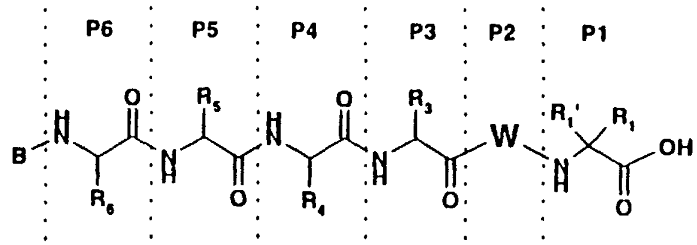

| Entry # | B | P6 | P5 | P4 | P3 | W | P1 | $IC_{50}$ (μM) | HLE (μM) | PPE (μM) | Other (μM) | MS ($MH^+$) | AAA (%) |
|---|---|---|---|---|---|---|---|---|---|---|---|---|---|
| 301* | Ac | Asp | Asp | Ile | Val | 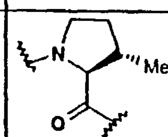 | Nva | 98* | | | | 713 | 99.8 |
| 302* | Ac | Asp | Asp | Ile | Val | 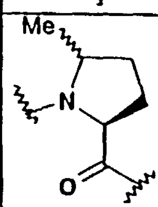 | Nva | 89* | | | | 713 | 102 |
| 303* | Ac | Asp | Asp | Ile | Val | 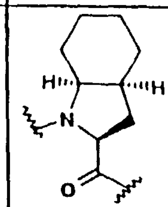 | Nva | 44* | | | | 753 | 104.4 |

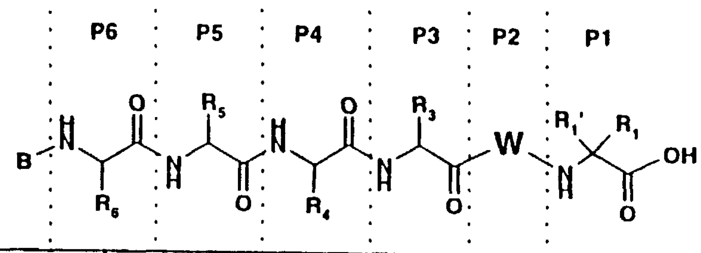

| Entry # | B | P6 | P5 | P4 | P3 | W | P1 | $IC_{50}$ (μM) | HLE (μM) | PPE (μM) | Other (μM) | MS ($MH^+$) | AAA (%) |
|---|---|---|---|---|---|---|---|---|---|---|---|---|---|
| 304✗ | Ac | --- | --- | Chg | Val | 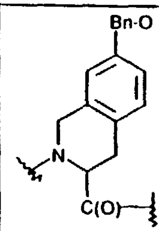 | Acca | 1.1 | | | | | |

# TABLE 4

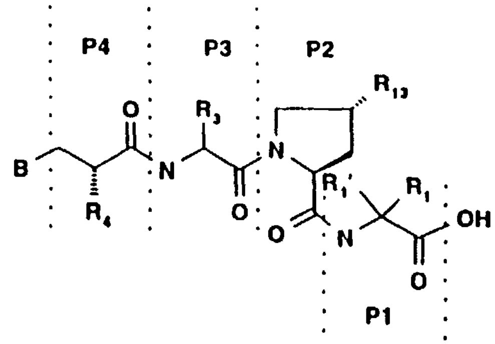

| Comp. | B | P6 | P5 | P3 | $R_4$ | $R_{13}$ | P1 | $IC_{50}$ (μM) | HLE (μM) | PPE (μM) | MS ($MH^+$) | AAA (%) |
|---|---|---|---|---|---|---|---|---|---|---|---|---|
| 401* | 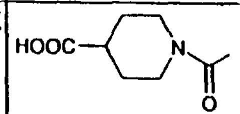 | | | Val | cyclohexyl | 1-Np$CH_2$O | Acca | 7.9 | | | 747.4 | |
| 402* | 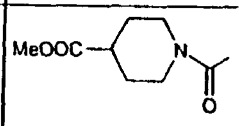 | | | Val | cyclohexyl | 1-Np$CH_2$O | Acca | 28 | | | 761.4 | |
| 403* | 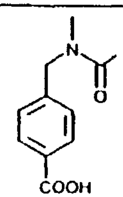 | | | Val | cyclohexyl | 1-Np$CH_2$O | Acca | 9.6 | | | 783.3 | |

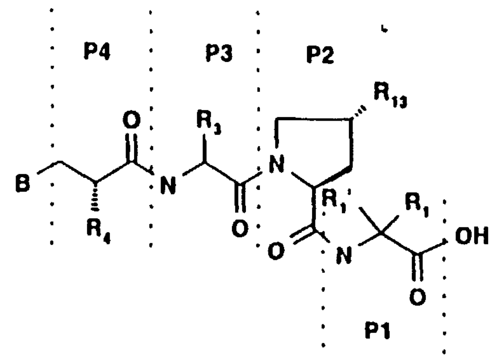

| Comp. | B | P6 | P5 | P3 | $R_4$ | $R_{13}$ | P1 | $IC_{50}$ (μM) | HLE (μM) | PPE (μM) | MS ($MH^+$) | AAA (%) |
|---|---|---|---|---|---|---|---|---|---|---|---|---|
| 404* | 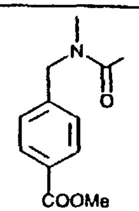 | | | Val | cyclohexyl | 1-Np$CH_2$O | Acca | 13 | | | 797.3 | |
| 405* | HOOC-$CH_2CH_2$-N(Me)C(O)- | | | Val | cyclohexyl | 1-Np$CH_2$O | Acca | 0.8 | | | 721.4 | |
| 406* | MeOOC-$CH_2$-$CH_2$-N(Me)C(O)- | | | Val | cyclohexyl | 1-Np$CH_2$O | Acca | 25 | | | 735.3 | |

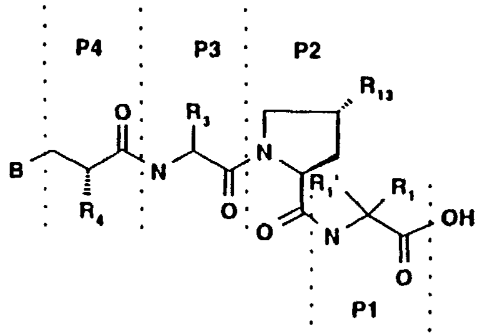

| Comp. | B | P6 | P5 | P3 | $R_4$ | $R_{13}$ | P1 | $IC_{50}$ (μM) | HLE (μM) | PPE (μM) | MS ($MH^+$) | AAA (%) |
|---|---|---|---|---|---|---|---|---|---|---|---|---|
| 407* | HOOC-$CH_2CH_2$-N(CH(Me)$_2$)-C(O)- | | | Val | cyclohexyl | 1-Np$CH_2O$ | Acca | 1.5 | | | 749.3 | |
| 408* | MeOOC-$(CH_2)_2$-N(CH(Me)$_2$)-C(O)- | | | Val | cyclohexyl | 1-Np$CH_2O$ | Acca | 11 | | | 763.3 | |
| 409* | HOOC-$CH_2$-N(CH(Me)$_2$)-C(O)- | | | Val | cyclohexyl | 1-Np$CH_2O$ | Acca | 24 | | | 735.4 | |
| 410* | EtOOC-$CH_2$-N(CH(Me)$_2$)-C(O)- | | | Val | cyclohexyl | 1-Np$CH_2O$ | Acca | 32 | | | 763.4 | |

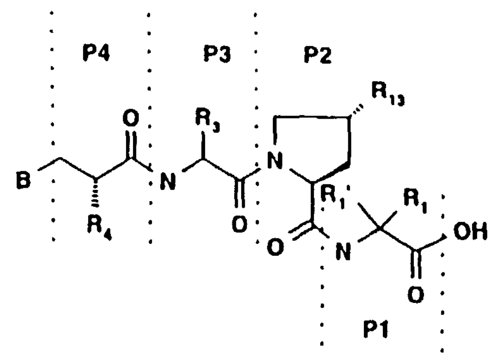

| Comp. | B | P6 | P5 | P3 | $R_4$ | $R_{13}$ | P1 | $IC_{50}$ (µM) | HLE (µM) | PPE (µM) | MS ($MH^+$) | AAA (%) |
|---|---|---|---|---|---|---|---|---|---|---|---|---|
| 411 * | HOOC-$(CH_2)_3$-N(CH(Me)$_2$-C(O) | | | Val | cyclohexyl | 1-NpCH$_2$O | Acca | 7.4 | | | 763.4 | |
| 412 * | [HOOC-CH$_2$]$_2$-NC(O)- | | | Val | cyclohexyl | 1-NpCH$_2$O | Acca | 0.8 | | | 751.3 | |
| 413 * | [HOOC-$(CH_2)_2$]$_2$-NC(O)- | | | Val | cyclohexyl | 1-NpCH$_2$O | Acca | 0.12 | | | 779.3 | |

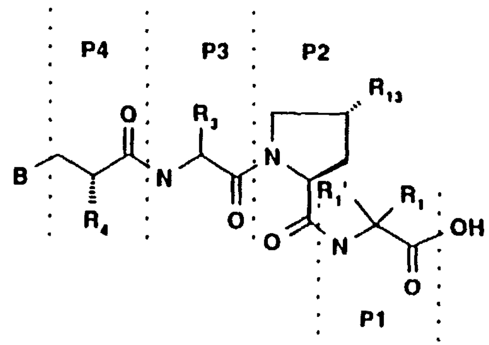

| Comp. | B | P6 | P5 | P3 | $R_4$ | $R_{13}$ | P1 | $IC_{50}$ (μM) | HLE (μM) | PPE (μM) | MS ($MH^+$) | AAA (%) |
|---|---|---|---|---|---|---|---|---|---|---|---|---|
| 414* | 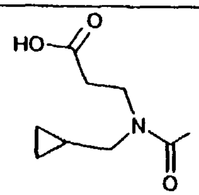 | | | Val | cyclohexyl | 1-Np$CH_2$O | Acca | 0.78 | | | 761.3 | |
| 415* | 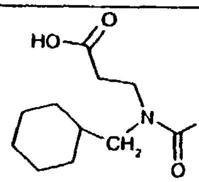 | | | Val | cyclohexyl | 1-Np$CH_2$O | Acca | 0.89 | | | 803.2 | |
| 416* | 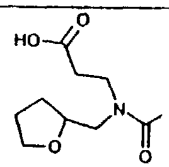 | | | Val | cycloheyxl | 1-Np$CH_2$O | Acca | 0.41 | | | 791.1 | |

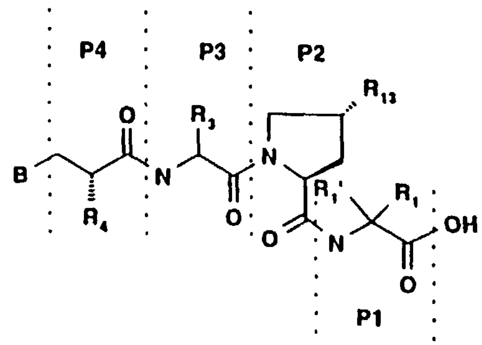


| Comp. | B | P6 | P5 | P3 | $R_4$ | $R_{13}$ | P1 | $IC_{50}$ (µM) | HLE (µM) | PPE (µM) | MS ($MH^+$) | AAA (%) |
|---|---|---|---|---|---|---|---|---|---|---|---|---|
| 417* | 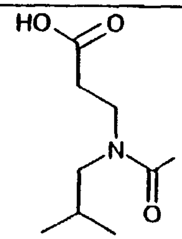 | | | Val | cyclohexyl | 1-Np$CH_2$O | Acca | 0.45 | | | 763.2 | |
| 418* | 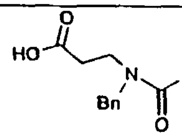 | | | Val | cyclohexyl | 1-Np$CH_2$O | Acca | 0.63 | | | 797.3 | |
| 419* | 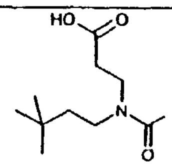 | | | | cyclohexyl | 1-Np$CH_2$O | Acca | 1.4 | | | 775.6 $(M-H)^-$ | |

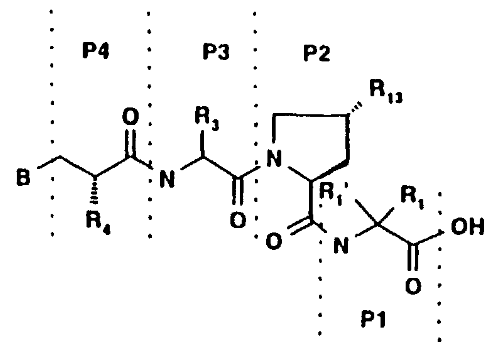

| Comp. | B | P6 | P5 | P3 | $R_4$ | $R_{13}$ | P1 | $IC_{50}$ (µM) | HLE (µM) | PPE (µM) | MS ($MH^+$) | AAA (%) |
|---|---|---|---|---|---|---|---|---|---|---|---|---|
| 420 * | 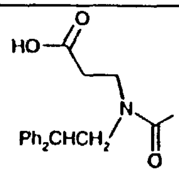 | | | Val | cyclohexyl | 1-$NpCH_2O$ | Acca | 0.52 | | | 925.6 $(MK)^+$ | |
| 421 * | 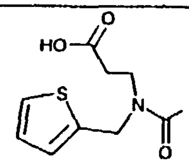 | | | Val | cyclohexyl | 1-$NpCH_2O$ | Acca | 1.7 | | | 841.5 $(MK)^+$ | |
| 422 * | 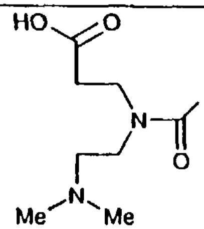 | | | Val | cyclohexyl | 1-$NpCH_2O$ | Acca | 4.0 | | | 778.4 | |

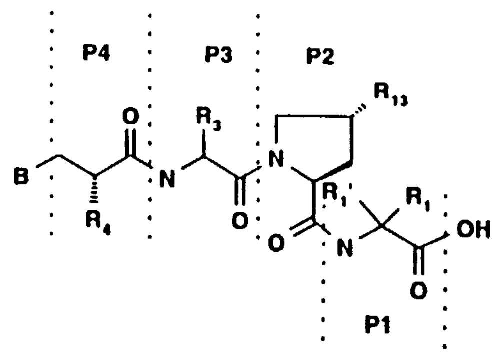

| Comp. | B | P6 | P5 | P3 | $R_4$ | $R_{13}$ | P1 | $IC_{50}$ (μM) | HLE (μM) | PPE (μM) | MS ($MH^+$) | AAA (%) |
|---|---|---|---|---|---|---|---|---|---|---|---|---|
| 423* | 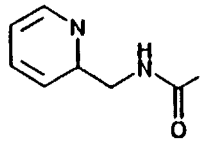 | | | Val | cyclohexyl | 1-NpCH$_2$O | Acca | 7.9 | | | 726.3 | |

(*) = compounds of this invention.

## Claims

**1.** A compound of formula (I):

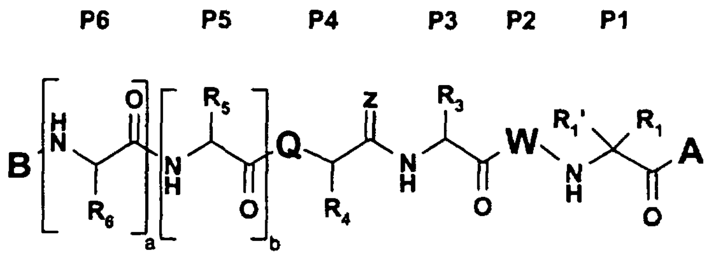

**(I)**

wherein **Q** is $CH_2$ or N-**Y**, wherein **Y** is H or $C_{1-6}$ alkyl;

a) when **Q** is $CH_2$, **a** is 0, **b** is 0, and **B** is an amide derivative of formula $\mathbf{R_{11a}}$N $\mathbf{(R_{11b})}$ -C(O)- wherein $\mathbf{R_{11a}}$ is H; $C_{1-10}$ alkyl optionally substituted with carboxyl or di ($C_{1-6}$ alkyl) amino; $C_{3-7}$ cycloalkyl; $C_6$ aryl; $C_{7-10}$ alkylaryl; ($C_{3-7}$ cycloalkyl)-($C_{1-6}$ alkyl); heterocycle-$C_{1-6}$ alkyl;
and $\mathbf{R_{11b}}$ is $C_{1-6}$ alkyl substituted with carboxyl, ($C_{1-6}$ alkoxy)carbonyl or phenylmethoxycarbonyl; or $C_{7-16}$ aralkyl substituted on the aromatic portion with carboxyl, ($C_{1-6}$ alkoxy)carbonyl, phenylmethoxycarbonyl, or heterocycle-$C_{1-6}$ alkyl;
or $\mathbf{R_{11a}}$ and $\mathbf{R_{11b}}$, are joined to form a 3 to 7-membered nitrogen-containing ring optionally substituted with carboxyl or ($C_{1-6}$ alkoxy) carbonyl;
or

b) when **Q** is N-**Y**; **a** is 0 or 1, **b** is 0 or 1, and **B** is an acyl derivative of formula $\mathbf{R_{11}}$-C(O)-wherein $\mathbf{R_{11}}$ is (i) $C_{1-10}$ alkyl optionally substituted with carboxyl, $C_{1-6}$ alkanoyloxy (e.g. $AcOCH_2$) or $C_{1-6}$ alkoxy (e.g. Boc); (ii) $C_{3-7}$ cycloalkyl optionally substituted with carboxyl, ($C_{1-6}$ alkoxy)carbonyl or phenylmethoxycarbonyl; (iii) $C_{3-7}$ cycloalkyl substituted with carboxyl and one to three $C_{1-6}$ alkyl substituents (iv) $C_{4-10}$ (alkylcycloalkyl) optionally substituted on the cycloalkyl portion with carboxy, ($C_{1-6}$ alkoxy)carbonyl or phenylmethyoxycarbonyl; (v)

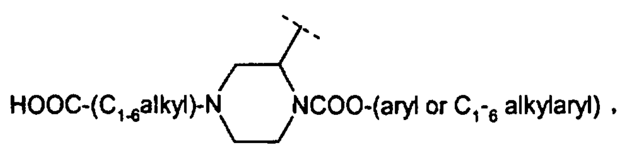

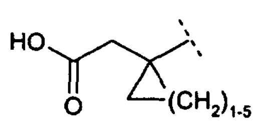 or 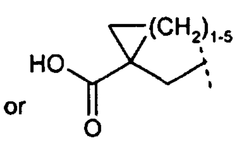 ;

(vi) $C_6$ or $C_{10}$ aryl or $C_{7-16}$ aralkyl optionally substituted with $C_{1-6}$ alkyl;
$\mathbf{R_6}$, when present, is $C_{1-6}$ alkyl substituted with carboxyl; and
$\mathbf{R_5}$, when present, is $C_{1-6}$ alkyl optionally substituted with carboxyl;
or

c) when **Q** is either $CH_2$ or N-**Y**;
$\mathbf{R_4}$ is $C_{1-10}$ alkyl, $C_{3-7}$ cycloalkyl or $C_{4-10}$ (alkylcycloalkyl);

**Z** is oxo or thioxo;
$\mathbf{R_3}$ is $C_{1-10}$ alkyl optionally substituted with carboxyl, $C_{3-7}$ cycloalkyl or $C_{4-10}$ (alkylcycloalkyl);
**W** is a group of formula III':

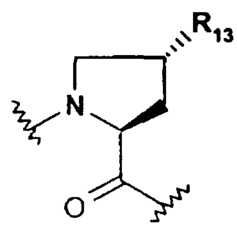

Formula III'

wherein $\mathbf{R_{13}}$ is OH; SH; $NH_2$; carboxyl; $\mathbf{R_{12}}$; $O\mathbf{R_{12}}$, $S\mathbf{R_{12}}$, $NH\mathbf{R_{12}}$ or $N\mathbf{R_{12}}\mathbf{R_{12}}'$ wherein $\mathbf{R_{12}}$ and $\mathbf{R_{12}}'$ are independently:
cyclic $C_{3-16}$ alkyl or acyclic $C_{1-16}$ alkyl or cyclic $C_{3-16}$ alkenyl or acyclic $C_{2-16}$ alkenyl, said alkyl or alkenyl optionally substituted with $NH_2$, OH, SH, halo, or carboxyl; said alkyl or alkenyl optionally containing at least one heteroatom independently selected from the group consisting of: O, S, and N; or
$\mathbf{R_{12}}$ and $\mathbf{R_{12}}'$ are independently $C_6$ or $C_{10}$ aryl or $C_{7-16}$ aralkyl optionally substituted with $C_{1-6}$ alkyl, $NH_2$, OH, SH, halo, carboxyl or carboxy $C_{1-6}$ alkyl; said aryl or aralkyl optionally containing at least one heteroatom independently selected from the group consisting of: O, S, and N;
said cyclic alkyl, cyclic alkenyl, aryl or aralkyl being optionally fused with a second 5-, 6-, or 7-membered ring to form a cyclic system or heterocyclic system, said second ring being optionally substituted with $NH_2$, OH, SH, halo, carboxyl or carboxy $C_{1-6}$ alkyl; said second ring optionally containing at least one heteroatom independently selected from the group consisting of: O, S, and N.
$\mathbf{R_1}'$ is hydrogen, and $\mathbf{R_1}$ is propyl; or
$\mathbf{R_1}'$ and $\mathbf{R_1}$ form together a 3-membered ring optionally substituted with $C_{1-6}$ alkyl; and
**A** is hydroxy or a pharmaceutically acceptable salt or ester thereof.

**2.** A compound of claim 1 of formula (Ia):

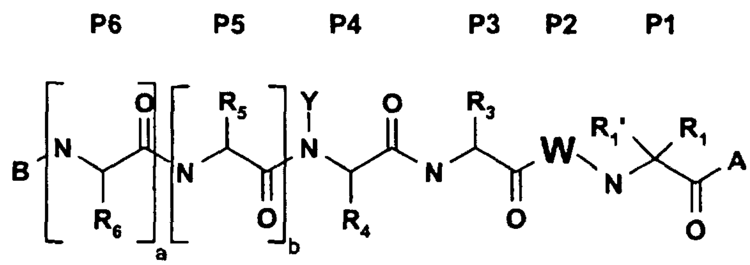

(Ia)

wherein **Y** is H or $C_{1-6}$ alkyl;
**a** is 0 or 1;
**b** is 0 or 1;
**B** is an acyl derivative of formula $\mathbf{R_{11}}$-C(O)-wherein $\mathbf{R_3}$, $\mathbf{R_4}$, $\mathbf{R_5}$, $\mathbf{R_6}$, $\mathbf{R_{11}}$, **W**, $\mathbf{R_1}$, $\mathbf{R_1}'$ and **A** are as defined in claim 1.

**3.** A compound of formula Ia according to claim 2, wherein **B** is an acyl derivative of formula $\mathbf{R_{11}}$C(O)- wherein $\mathbf{R_{11}}$ is:

$C_{1-6}$ alkyl optionally substituted with carboxyl, $C_{1-6}$ alkanoyloxy or $C_{1-6}$ alkoxy;
$C_{3-7}$ cycloalkyl optionally substituted with carboxyl, MeOC(O), EtOC(O) or BnOC(O);
3-carboxypropionyl (DAD) or 4-carboxybutyryl (DAE); or

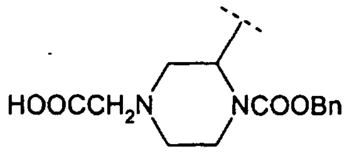

4. A compound of formula Ia according to claim 3, wherein **B** is acetyl, 3-carboxypropionyl, 4-carboxylbutyryl, $AcOCH_2C(O)$, $Me_3COC(O)$,

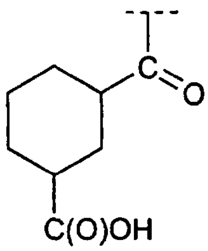
,
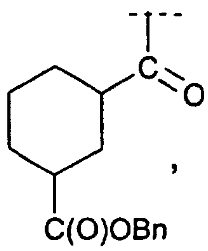
,
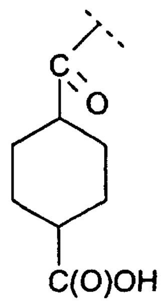
,
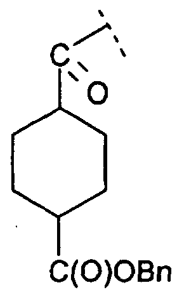
,

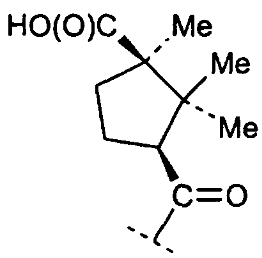
,
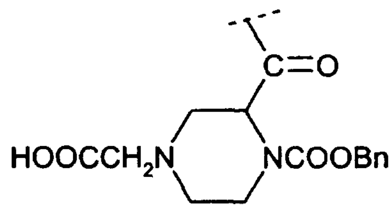
,

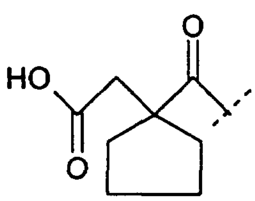
,
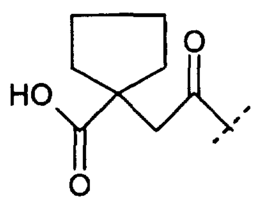
.

5. A compound of formula Ia according to claim 4, wherein **B** is acetyl, 3-carboxypropionyl (DAD), 4-carboxybutyryl (DAE), $AcOCH_2C(O)$,

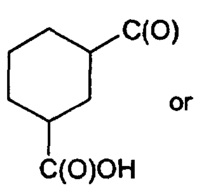
or
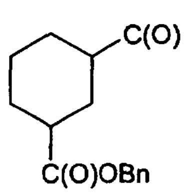
.

6. A compound of formula Ia according to claim 5, wherein, **B** is acetyl.

7. A compound of formula Ia according to claim 2, wherein $\mathbf{R_6}$, when present, is the side chain of Asp or Glu.

8. A compound of formula Ia according to claim 7, wherein $\mathbf{R_6}$, when present, is the side chain of Asp.

9. A compound of formula Ia according to claim 2, wherein $\mathbf{R_5}$, when present, is the side chain of an amino acid selected from the group consisting of D-Asp, Asp, D-Glu, Glu, D-Val, Val, D-Tbg and Tbg.

10. A compound of formula Ia according to claim 9, wherein $\mathbf{R_5}$, when present, is the side chain of D-Asp, D-Val or D-Glu.

11. A compound of formula Ia according to claim 10, wherein $\mathbf{R_5}$, when present, is the side chain of D-Glu.

12. A compound of claim 1 of formula (Ib):

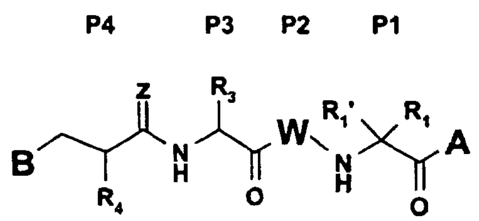

(Ib)

wherein **B** is an amide of formula $\mathbf{R_{11a}}$N $(\mathbf{R_{11b}})$ C(O)- wherein $\mathbf{R_{11a}}$ is $C_{1-6}$ alkyl optionally substituted with carboxy or $\mathbf{R_{11a}}$ is $C_6$ aryl, $C_{7-10}$ arylalkyl, 2-tetrahydrofuranylmethyl, or 2-thiazolidylmethyl; and $\mathbf{R_{11b}}$ is $C_{1-6}$ alkyl substituted with carboxyl.

13. A compound of formula (Ib) according to claim 12, wherein $\mathbf{R_{11a}}$ is isopropyl, carboxyethyl, benzylmethyl, benzyl, or 2-tetrahydrofuranylmethyl.

14. A compound of formula (Ib) according to claim 13, wherein $\mathbf{R_{11b}}$ is $C_{1-4}$ alkyl substituted with carboxyl.

15. A compound of formula (Ib) according to claim 14, wherein $\mathbf{R_{11b}}$ is ethyl carboxyl.

16. A compound of formula I according to claim 1, wherein $\mathbf{R_4}$ is selected from the group consisting of: isopropyl, cyclopropyl, tert-butyl, 1-methylpropyl, or 2-methylpropyl.

17. A compound of formula I according to claim 16, wherein $\mathbf{R_4}$ is cyclopropyl or 1-methylpropyl.

18. A compound of formula Ia according to claim 17, wherein $\mathbf{R_4}$ is cyclopropyl.

19. A compound of formula I according to claim 1, wherein **Z** is oxo.

20. A compound of formula I according to claim 1, wherein $\mathbf{R_3}$ is the side chain of Ile, allo-Ile, Chg, Cha, Val, Tbg or Glu.

21. A compound of formula I according to claim 20, wherein $\mathbf{R_3}$ is the side chain of Val, Tbg or Chg.

22. A compound of formula I according to claim 21, wherein $\mathbf{R_3}$ is the side chain of Val.

23. A compound of formula I according to claim 1, wherein $\mathbf{R_{13}}$ is O$\mathbf{R_{12}}$ or S$\mathbf{R_{12}}$ wherein $\mathbf{R_{12}}$ is a $C_6$ or $C_{10}$ aryl or $C_{7-16}$ aralkyl, said first aryl or aralkyl optionally substituted with $C_{1-6}$ alkyl, $C_{3-7}$ cycloalkyl, $NH_2$, OH, SH, halo, $C_{1-6}$ alkoxy, carboxyl, carboxy $C_{1-6}$ alkyl, or a second aryl or aralkyl; said first and second aryl or aralkyl optionally containing at least one heteroatom selected independently from the group consisting of: O, S, and N.

**24.** A compound according to claim 23, wherein $\mathbf{R_{13}}$ is Bn; $PhCH_2CH_2$; $PhCH_2CH_2CH_2$; O-Bn; o-tolylmethoxy; m-tolylmethoxy; p-tolylmethoxy; 1-naphtyloxy; 2-naphtyloxy; 1-naphthalenylmethoxy; 2-naphthalenylmethoxy; (4-tert-butyl)methoxy; (3I-Ph)$CH_2O$; (4Br-Ph)O; (2Br-Ph)O; (3Br-Ph)O; (4I-Ph) O; (3Br-Ph)$CH_2O$; (3,5-$Br_2$-Ph) $CH_2O$;

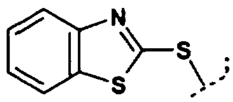 ; 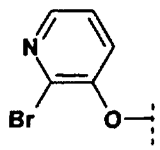 ; 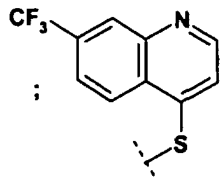 ; 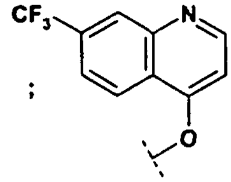 ;

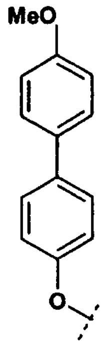 ; 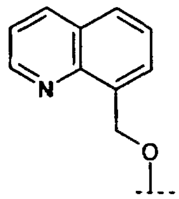 ; 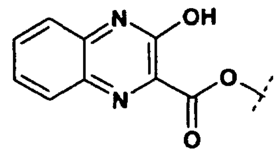 ;

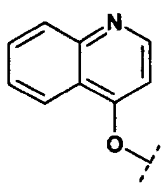 ; 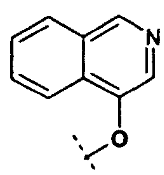 ; 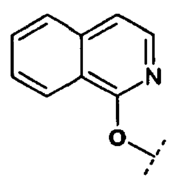 ; 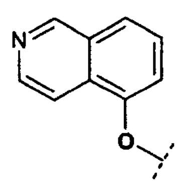 ;

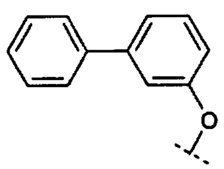 ; 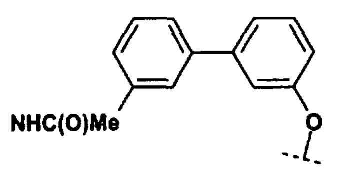 ;

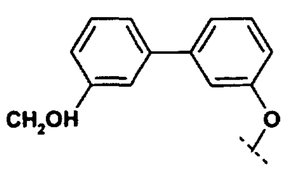 ; 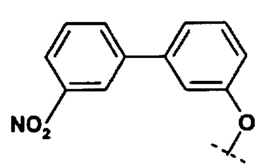 ;

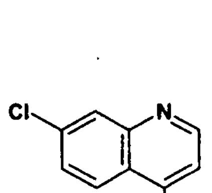 ;  ; 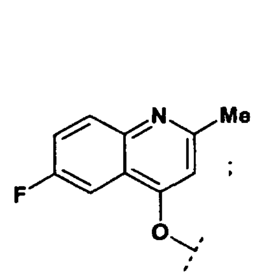 ;

.

**25.** A compound according to claim 24, wherein $\mathbf{R_{13}}$ is O-Bn; $PhCH_2CH_2CH_2$; 1-naphtyloxy; 2-naphtyloxy; 1-naphthalenylmethoxy; 2-naphthalenylmethoxy;

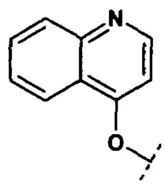 ; 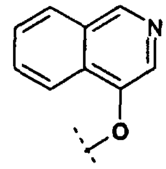 ; 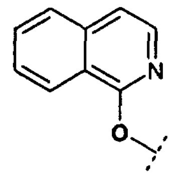 ; 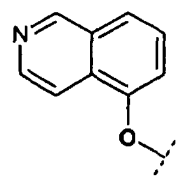 ;

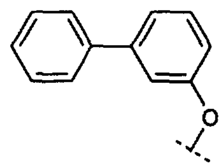 ; 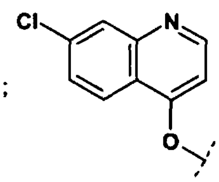 .

**26.** A compound of formula I according to claim 1, wherein

a) **Q** is $CH_2$, **a** is 0, **b** is 0, and **B** is an amide of formula $\mathbf{R_{11a}}N(\mathbf{R_{11b}})$-C(O)- wherein $\mathbf{R_{11a}}$ is $C_{1-6}$ alkyl optionally substituted with carboxyl or $\mathbf{R_{11a}}$ is phenyl, $C_{7-10}$ arylalkyl, 2-tetrahydrofuranylmethyl, or 2-thienylmethyl; and $\mathbf{R_{11b}}$ is ($C_{0-2}$ alkyl)phenyl optionally substituted with carboxyl or ($C_{1-4}$ alkoxy)carbonyl; or $C_{1-6}$ alkyl substituted with carboxyl or ($C_{1-4}$ alkoxy)carbonyl; or $\mathbf{R_{11a}}$ and $\mathbf{R_{11b}}$ are joined to form a piperidine ring optionally substituted with carboxyl or ($C_{1-6}$ alkoxy)carbonyl;
or
b) **Q** is N-**Y**, wherein **Y** is H or $C_{1-6}$ alkyl; **a** is 0 or 1, **b** is 0 or 1, and **B** is an acyl derivative of formula $\mathbf{R_{11}}$-C(O)- wherein $\mathbf{R_{11}}$ is (i) $C_{1-6}$ alkyl, $C_{1-6}$ alkyl substituted with carboxyl, MeC(O)O-, MeO-, EtO-, $MeCH_2CH_2O$- or $Me_3C$-O-; (ii) cyclopentyl or cyclohexyl optionally substituted with carboxyl; (iv) $C_{4-10}$ (alkylcycloalkyl) optionally substituted on the cycloalkyl portion with carboxyl;
(v)

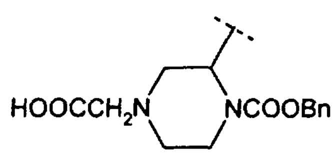

(vi) phenyl, benzyl or phenylethyl;
$\mathbf{R_6}$, when present, is $CH_2COOH$ or $CH_2CH_2COOH$,
$\mathbf{R_5}$, when present, is $C_{1-6}$ alkyl or $CH_2COOH$ or $CH_2CH_2COOH$; or
c) when **Q** is either $CH_2$ or N-**Y**,
$\mathbf{R_4}$ is $C_{1-6}$ alkyl, $C_{3-7}$ cycloalkyl or $C_{4-10}$ (alkylcycloalkyl);
**Z** is oxo or thio;
$\mathbf{R_3}$ is $C_{1-6}$ alkyl; $C_{3-7}$ cycloalkyl or $C_{4-10}$ (alkylcycloalkyl);
$\mathbf{R_{1'}}$ is hydrogen and $\mathbf{R_1}$ is propyl; or $\mathbf{R_{1'}}$ and $\mathbf{R_1}$ together with the carbon atom to which they are attached form a cyclopropyl which may optionally be substituted with ethyl; and
**W** is as defined in claim 1; and
**A** is hydroxy or a pharmaceutically acceptable salt thereof; $C_{1-6}$ alkoxy, or (aryl $C_{1-6}$-alkoxy).

**27.** A compound of formula Ia according to claim 2, wherein **B** is an acyl derivative of formula $\mathbf{R_{11}}$-C(O)- wherein $\mathbf{R_{11}}$ is $C_{1-6}$ alkoxy, $C_{1-10}$ alkyl optionally substituted with carboxyl;$C_{3-7}$ cycloalkyl optionally substituted with carboxyl or benzylcarboxy; or

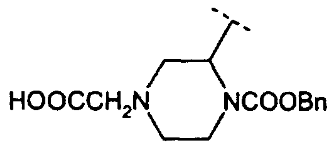

;

**$R_6$** is absent;
**$R_5$** is absent;
**$R_4$** is $C_{1-10}$ alkyl, $C_{3-7}$ cycloalkyl or $C_{4-10}$ (alkylcycloalkyl);
**$R_3$** is $C_{1-10}$ alkyl, $C_{3-7}$ cycloalkyl or $C_{4-10}$ (alkylcycloalkyl);
and
**W** is as defined in claim 1; and
**A** is hydroxy or a pharmaceutically acceptable salt thereof; methoxy, ethoxy, phenoxy, or benzyloxy.

**28.** A compound of formula Ia according to claim 2, wherein **B** is acetyl, 3-carboxypropionyl, 4-carboxylbutyryl, $AcOCH_2C(O)$, $Me_3COC(O)$,

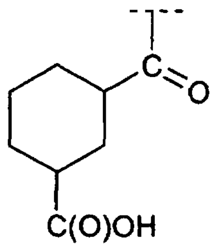
,
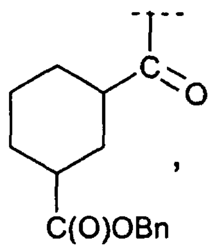
,
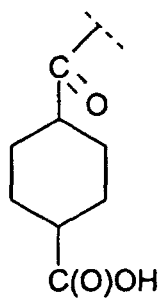
,
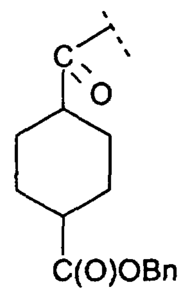
,

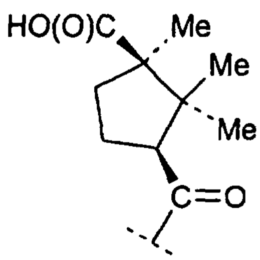
,
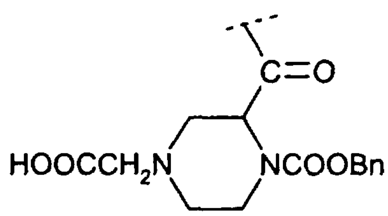
,

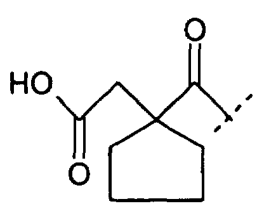
,
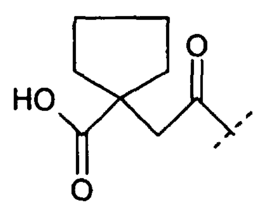

;

**Y** is H or Me, **a** is 0 or 1, **b** is 0 or 1,
**$R_6$**, when present, is the side chain of Asp or Glu,
**$R_5$**, when present, is the side chain of Asp, D-Asp, Glu, D-Glu, Val, D-Val or Tbg,
**$R_4$** is the side chain of Val, Chg, Tbg, Ile or Leu,
**$R_3$** is hydrogen or the side chain of Ile, Chg, Val, Glu;
**W** is group of formula III':

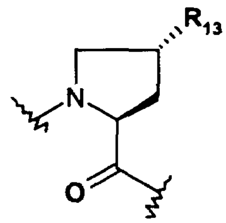

(III')

wherein $\mathbf{R_{13}}$ is Bn, $PhCH_2CH_2$, $PhCH_2CH_2CH_2$, O-Bn, o-tolylmethoxy, m-tolylmethoxy, p-tolylmethoxy, 1-naphthalenylmethoxy, 2-naphthalenylmethoxy, (4-*tert*-butyl)benzyloxy, (3I-Ph)$CH_2O$, (4Br-Ph)O, (2Br-Ph)O, (3Br-Ph)O, (4I-Ph)O, (3Br-Ph)$CH_2O$, (3,5-$Br_2$-Ph)$CH_2O$,

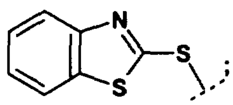 ; 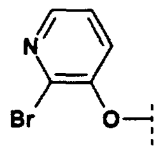 ; 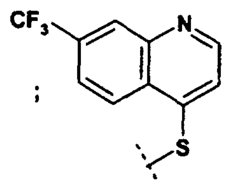 ; 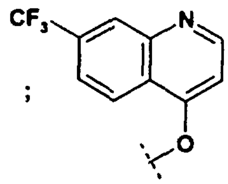 ;

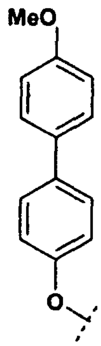 ; 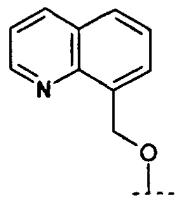 ; 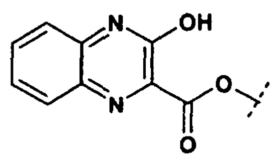 ;

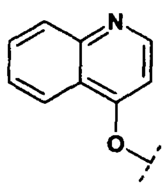 ; 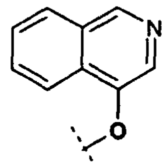 ; 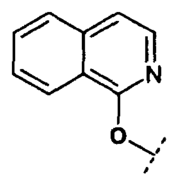 ; 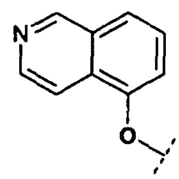 ;

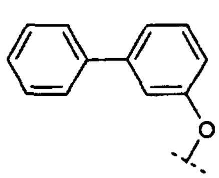 ; 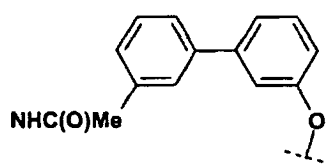 ;

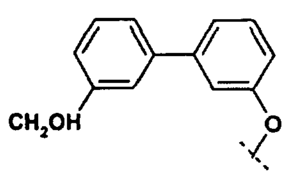 ; 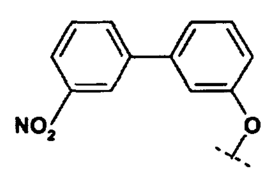 ;

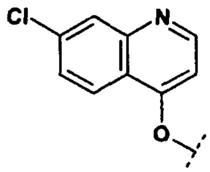 ; 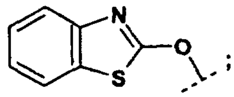 ; 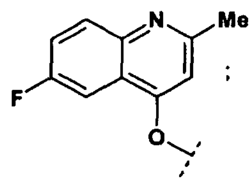 ;

$R_{1'}$ is H and $R_1$ is propyl; or
$R_{1'}$ and $R_1$ together with the carbon atom to which they are attached form a cyclopropyl; and **A** is hydroxyl.

**29.** A compound of formula Ib according to claim 12, wherein **B** is an amide of formula $R_{11a}N(R_{11b})$-C(O)- wherein $R_{11a}$ is $C_{1-6}$ alkyl optionally substituted with carboxyl or $R_{11a}$ is 2-tetrahydrofuranylmethyl;
and $R_{11b}$ is ($C_{0-2}$ alkyl)phenyl optionally substituted with carboxyl or ($C_{1-4}$ alkoxy)carbonyl; or $C_{1-6}$ alkyl substituted with carboxyl or ($C_{1-4}$ alkoxy)carbonyl; or
$R_{11a}$ and $R_{11b}$ are joined to form a piperidine ring optionally substituted with carboxyl or ($C_{1-6}$ alkoxy)carbonyl;
$R_4$ is cyclohexyl,
**Z** is oxo;
$R_3$ is hydrogen or the side chain of Ile, Chg, Val, Glu;
**W** is group of formula III':

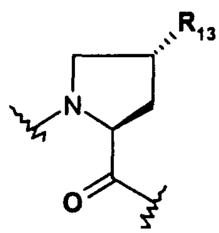 (III')

wherein $R_{13}$ is Bn, $PhCH_2CH_2$, $PhCH_2CH_2CH_2$, O-Bn, o-tolylmethoxy, m-tolylmethoxy, p-tolylmethoxy, 1-naphthalenylmethoxy, 2-naphthalenylmethoxy, (4-*tert*-butyl)methoxy, (3I-Ph)$CH_2O$, (4Br-Ph)O, (2Br-Ph)O, (3Br-Ph)O, (4I-Ph)O, (3Br-Ph)$CH_2O$, (3,5-$Br_2$-Ph)$CH_2O$,

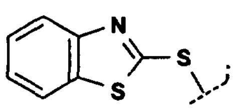 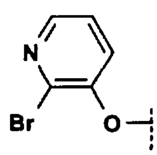 ; 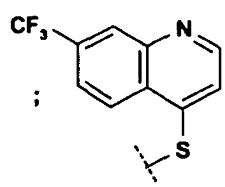 ; 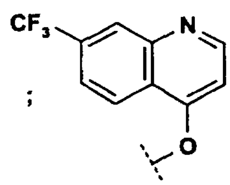 ;

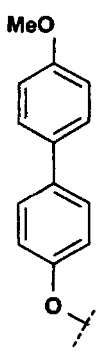 ; 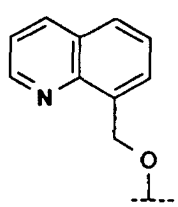 ; 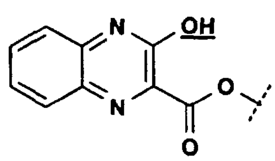 ;

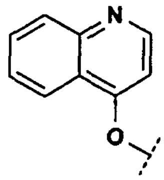 ; 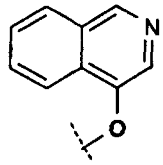 ; 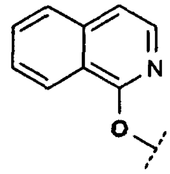 ; 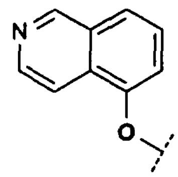 ;

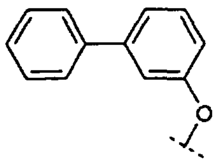 ; 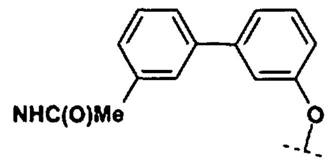 ;

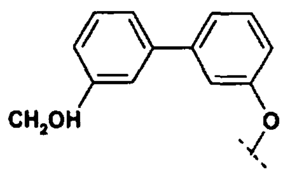 ; 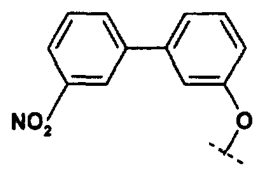 ;

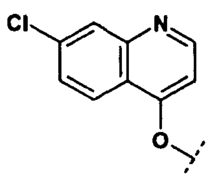 ; 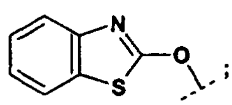 ; 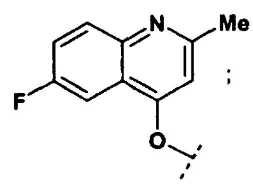 ;

**$R_{1'}$** is H and **$R_1$** is propyl; or
**$R_{1'}$** and **$R_1$** together with the carbon atom to which they are attached form a cyclopropyl; and A is hydroxyl.

**30.** A compound of formula I according to claim 1, wherein **B** is an acyl derivative of formula **$R_{11}$**-C(O)- wherein **$R_{11}$** is $C_{1-10}$ alkyl optionally substituted with carboxyl; $C_{3-7}$ cycloalkyl optionally substituted with carboxyl; or a $C_{4-10}$ (alkylcycloalkyl) optionally substituted on the cycloalkyl portion with carboxyl;
or **$R_{11}$** is $C_6$ or $C_{10}$ aryl or $C_{7-16}$ aralkyl optionally substituted with a $C_{1-6}$ alkyl
**a** is 0 or 1;
**$R_6$,** when present, is $C_{1-6}$ alkyl optionally substituted with carboxyl;
**b** is 0 or 1;
**$R_5$,** when present, is $C_{1-6}$ alkyl optionally substituted with carboxyl;
**Q** is N-**Y**, and **Y** is H or $C_{1-6}$ alkyl;
**$R_4$** is $C_{1-10}$ alkyl, $C_{3-7}$ cycloalkyl or $C_{4-10}$ (alkylcycloalkyl);
**Z** is oxo,
**$R_3$** is $C_{1-10}$ alkyl, $C_{3-7}$ cycloalkyl or $C_{4-10}$ (alkylcycloalkyl);
and
**W**, **$R_1$'** and **$R_1$** are as defined in claim 1; and
**A** is hydroxyl or a pharmaceutically acceptable salt or ester thereof.

**31.** A pharmaceutical composition comprising an anti-hepatitis C virally effective amount of a compound of formula I

of claim 1, or a therapeutically acceptable salt or ester thereof, in admixture with a pharmaceutically acceptable carrier medium or auxiliary agent.

**32.** An *in vitro* method of inhibiting the replication of hepatitis C virus by exposing the virus to a hepatitis C viral NS3 protease inhibiting amount of the compound of formula I of claim 1, or a therapeutically acceptable salt or ester thereof.

**33.** The use of a compound of formula I of claim 1 for the manufacture of a medicament for treatment of a hepatitis C infection in a mammal.

**34.** A compound selected from the group consisting of: a compound of formula

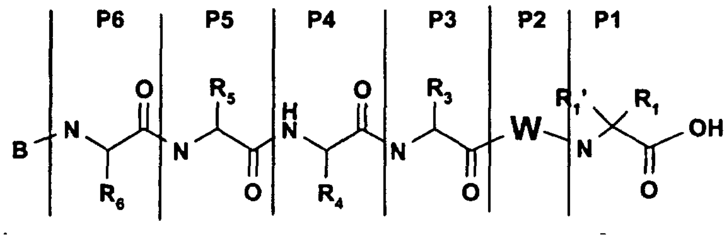

wherein B, P6, P5, P4, P3, W, and P1 are as defined below:

| Cpd #; | B; | P6; | P5; | P4; | P3; | W; | P1; |
|---|---|---|---|---|---|---|---|
| 123; | Ac; | Asp; | Asp; | Ile; | Val; | Pro; | Nva; |
| 126; | Ac; | Asp; | Asp; | Ile; | Val; | Pro; | Acca; |
| 127; | Ac; | Asp; | Asp; | Ile; | Val; | Pip; | Nva; |
| 128; | Ac; | Asp; | D-Glu; | Ile; | Val; | Pro; | Nva; |
| 129; | Ac; | Asp; | Tbg; | Ile; | Val; | Pro; | Nva; |
| 130; | DAD; | ---; | Asp; | Ile; | Val; | Pro; | Nva; |
| 132; | Ac; | Asp; | D-Glu; | Chg; | Glu; | Glu; | Acca; |
| 133; | Ac; | Asp; | Glu; | Chg; | Val; | Glu(OBn); | Acca. |

| Cpd #; | B; | P6; | P5; | P4; | P3; | W; | P1; |
|---|---|---|---|---|---|---|---|
| 303; | Ac; | Asp; | Asp; | Ile; | Val; | 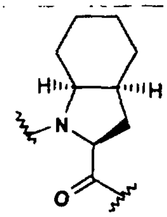 ; | Nva; |
| and 304; | Ac; | ---; | ---; | Chg; | Val; | 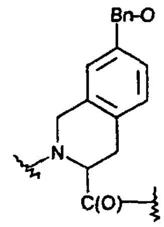 ; | Acca. |

**35.** A compound according to claim 1, selected from the group consisting of: a compound of formula

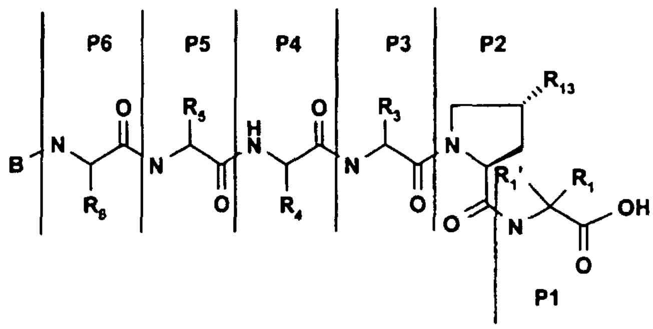

wherein B, P6, P5, P4, P3, $R_{13}$ and $P_1$ are as defined below:

| Cpd #; | B; | P6; | P5; | P4; | P3; | $R_{13}$; | P1; |
|---|---|---|---|---|---|---|---|
| 201; | Ac | Asp | Asp; | Ile; | Val; | O-Bn; | Nva; |
| 202; | Ac | Asp | D-Val; | Ile; | Val; | O-Bn; | Nva; |
| 203; | Ac | Asp | D-Glu; | Ile; | Val; | O-Bn; | Nva; |
| 204; | Ac | Asp | Asp; | Ile; | Val; | *o*-tolyl-methoxy; | Nva; |
| 205; | Ac | Asp | Asp; | Ile; | Val; | *m*-tolyl-methoxy; | Nva; |
| 206; | Ac | Asp | Asp; | Ile; | Val; | *p*-tolyl-methoxy; | Nva; |
| 207; | Ac | Asp | Asp; | Ile; | Val; | 1-$NpCH_2O$; | Nva; |
| 208; | Ac | Asp | Asp; | Ile; | Val; | 2-$NpCH_2O$; | Nva; |
| 209; | Ac | Asp | Asp; | Ile; | Val; | 4-*tert*-butyl-phenyl)-methoxy; | Nva; |
| 211; | Ac | Asp | D-Glu; | Chg; | Val; | O-Bn; | Nva; |
| 212; | Ac | Asp | D-Glu; | Ile; | Val; | O-Bn; | Acca; |
| 213; | Ac | Asp | D-Glu; | Ile; | Val; | 2-$NpCH_2O$; | Nva; |
| 214; | Ac | Asp | D-Glu; | Chg; | Val; | 2-$NpCH_2O$; | Nva; |
| 215; | Ac | Asp | D-Glu; | Chg; | Val; | 1-$NpCH_2O$; | Acca; |
| 216 | Ac | Asp | Asp; | Ile; | Val; | Bn; | Nva; |

| Cpd #; | B; | P6; | P5; | P4; | P3; | $R_{13}$; | P1; |
|---|---|---|---|---|---|---|---|
| 217; | Ac | Asp | Asp; | Ile; | Val; | $Ph(CH_2)_3$; | Nva; |
| 218; | Ac | Asp | D-Glu; | Ile; | Val; | O-Bn; | Nva; |
| 219; | Ac; | ---; | Asp; | Ile; | Val; | 1-$NpCH_2O$; | Nva; |
| 220; | DAD; | ---; | ---; | *N*(Me)Ile; | Val; | 1-$NpCH_2O$; | Nva; |
| 221; | DAD; | ---; | ---; | Ile; | Val; | 1-$NpCH_2O$; | Nva; |
| 222; | DAE; | ---; | ---; | Ile; | Val; | 1-$NpCH_2O$; | Nva; |
| 223; | 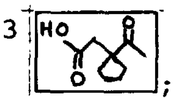; | ---; | ---; | Ile; | Val; | 1-$NpCH_2O$; | Nva; |
| 224; | 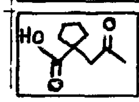; | ---; | ---; | Ile; | Val; | 1-$NpCH_2O$; | Nva; |
| 225; | Ac; | ---; | ---; | Ile; | Val; | 1-$NpCH_2O$; | Nva; |
| 226; | DAE; | ---; | ---; | Chg; | Val | 1-$NpCH_2O$; | Acca; |
| 227; | Ac; | ---; | ---; | Chg; | Val; | 1-$NpCH_2O$; | Acca; |
| 228; | Ac; | ---; | ---; | Chg; | Val; | O-Bn; | 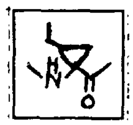; |
| 230; | Ac; | Asp; | Asp; | Ile; | Val; | $Ph(CH_2)_3$; | Nva; |
| 231; | Ac; | ---; | ---; | Chg; | Chg; | 1-$NpCH_2O$; | Acca; |
| 232; | $AcOCH_2$-C(O); | ---; | ---; | Chg; | Chg; | 1-$NpCH_2O$; | Acca; |
| 233; | Ac; | Asp; | Glu; | Ile; | Val; | (3I-Ph)$CH_2O$; | Acca; |
| 234; | Ac; | ---; | ---; | Chg; | Chg; | O-Bn; | Acca; |
| 235; | Boc; | ---; | ---; | Chg; | Chg; | 1-$NpCH_2O$; | Acca; |
| 236; | Ac; | ---; | Gly; | thioxo-Ile; | Val; | 1-$NpCH_2O$; | Nva; |
| 237; | DAE; | ---; | ---; | Ile; | Val; | 1-$NpCH_2O$; | Acca; |
| 238 | Ac; | ---; | ---; | Chg; | Val; | (4Br- | Acca; |

| Cpd #; | B; | P6; | P5; | P4; | P3; | R13; | P1; |
|---|---|---|---|---|---|---|---|
| ; | | | | | | Ph)O; | |
| 239 ; | Ac; | ---; | ---; | Chg; | Val; | (2Br-Ph)O; | Acca; |
| 240 ; | Ac; | ---; | ---; | Chg; | Val; | (3Br-Ph)O; | Acca; |
| 241 ; | Ac; | ---; | ---; | Chg; | Val; | 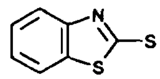 ; | Acca; |
| 242 ; | Ac; | ---; | ---; | Chg; | Val; | (4Br-Ph)S; | Acca; |
| 243 ; | Ac; | ---; | ---; | Chg; | Val; | 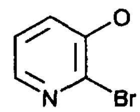 ; | Acca; |
| 244 ; | Ac; | ---; | ---; | Chg; | Val; | 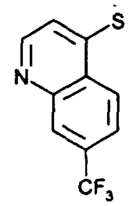 ; | Acca; |
| 245 ; | Ac; | ---; | ---; | Chg; | Val; | 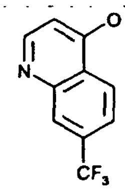 ; | Acca; |
| 246 ; | Ac; | ---; | ---; | Chg; | Val; | 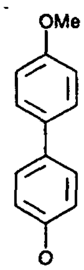 ; | Acca; |
| 247 ; | Ac; | Asp; | Asp; | Ile; | Val; | $Ph(CH_2)_2$; | Nva; |
| 248 ; | Ac; | ---; | ---; | Chg; | Chg; | 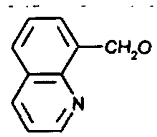 ; | Acca; |
| 249 ; | Ac; | ---; | ---; | Chg; | Val; | (4I-Ph)O; | Acca; |
| 250 ; | Ac; | ---; | ---; | Chg; | Val; | 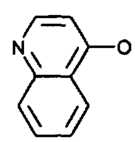 ; | Acca; |

| Cpd #; | B; | P6; | P5; | P4; | P3; | $R_{13}$; | P1; |
|---|---|---|---|---|---|---|---|
| 251 ; | Ac; | ---; | ---; | Chg; | Val; | 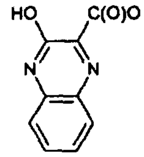 ; | Acca; |
| 252 ; | Ac; | ---; | ---; | Chg; | Val; | 1-NpCH$_2$O; | Nva; |
| 253 ; | Ac; | ---; | ---; | Chg; | Val; | 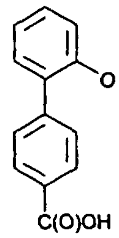 ; | Acca; |
| 254 ; | Ac; | ---; | ---; | Chg; | Val; | 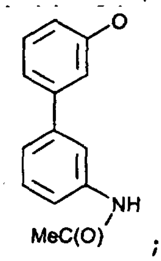 ; | Acca; |
| 255 ; | Ac; | ---; | ---; | Chg; | Val; | 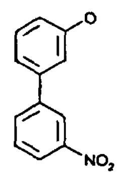 ; | Acca; |
| 256 ; | Ac; | ---; | ---; | Chg; | Val; | 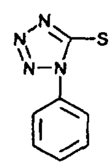 ; | Acca; |
| 257 ; | Ac; | ---; | ---; | Chg; | Val; | 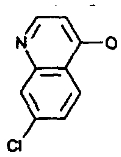 ; | Acca; |
| 258 ; | Ac; | ---; | ---; | Chg; | Val; | 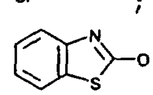 ; | Acca; |
| 259 ; | Ac; | ---; | ---; | Chg; | Val; | 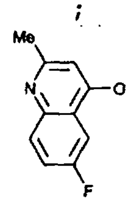 ; | Acca; |
| 262 ; | Ac; | ---; | ---; | Ile; | Val; | 1-NpCH$_2$O; | Acca; |

| Cpd #; | B; | P6; | P5; | P4; | P3; | $R_{13}$; | P1; |
|---|---|---|---|---|---|---|---|
| 263 ; | 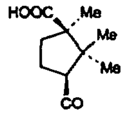 ; | ---; | ---; | Ile; | Val; | 1-NpCH$_2$O; | Acca; |
| 264 ; | 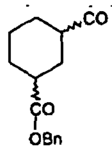 ; | ---; | ---; | Ile; | Val; | 1-NpCH$_2$O; | Acca; |
| 265 ; | 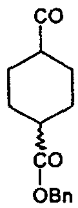 ; | ---; | ---; | Ile; | Val; | 1-NpCH$_2$O; | Acca; |
| 266 ; | 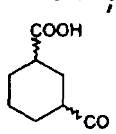 ; | ---; | ---; | Ile; | Val; | 1-NpCH$_2$O; | Acca; |
| 267 ; | 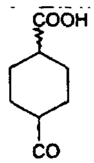 ; | ---; | ---; | Ile; | Val; | 1-NpCH$_2$O; | Acca; |
| 268 ; | Ac; | ---; | ---; | Chg; | Val; | (3Br-Ph) CH$_2$O; | Acca; |
| 269 ; | 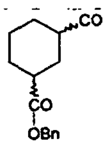 ; | ---; | ---; | Chg; | Val; | 1-NpCH$_2$O; | Acca; |
| 270 ; | 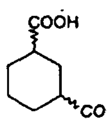 ; | ---; | ---; | Chg; | Val; | 1-NpCH$_2$O; | Acca; |
| 271 ; | 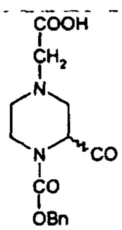 ; | ---; | ---; | Chg; | Val; | 1-NpCH$_2$O; | Acca; |
| 272 ; | Ac; | ---; | ---; | Chg; | Val; | (3,5-Br$_2$-Ph) CH$_2$O; | Acca; |
| 273 ; | Ac; | Asp; | Asp; | Ile; | Val; | H; | Nva; |

| Cpd #; | B; | P6; | P5; | P4; | P3; | R13; | P1; |
|---|---|---|---|---|---|---|---|
| and 275 ; | Ac; | ---; | ---; | Chg; | Val; | 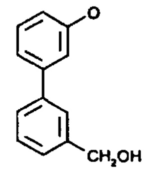 ; | Acca. |

**36.** A compound according to claim 1, selected from the group consisting of: a compound of formula

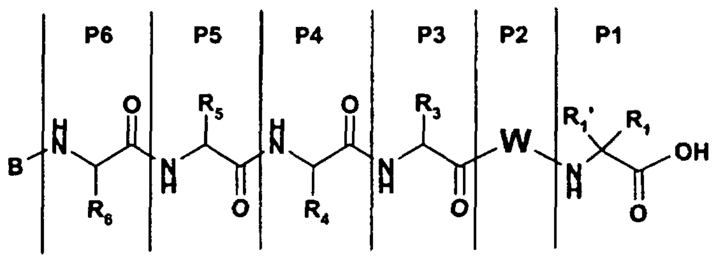

wherein B, P6, P5, P4, P3, W, and P1 are as defined below:

| Entry #; | B; | P6; | P5; | P4; | P3; | W; | P1; |
|---|---|---|---|---|---|---|---|
| 301; | Ac; | Asp; | Asp; | Ile; | Val; | 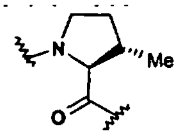 ; | Nva; |
| 302; | Ac; | Asp; | Asp; | Ile; | Val; | 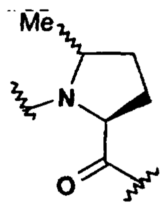 ; | Nva; |

**37.** A compound according to claim 1, selected from the group consisting of: a compound of formula

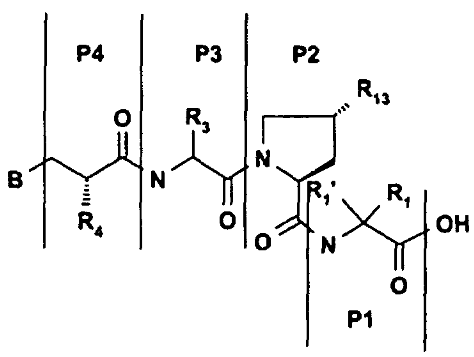

wherein B, $R_4$, P3, $R_{13}$, and P1 are as defined below:

| Cpd; | B; | $R_4$; | P3; | $R_{13}$; | P1; |
|---|---|---|---|---|---|
| 401; | 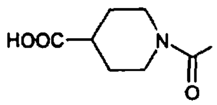 ; | cyclohexyl; | Val; | 1-NpCH$_2$O; | Acca; |
| 402; | 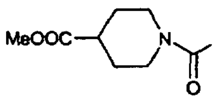 ; | cyclohexyl; | Val; | 1-NpCH$_2$O; | Acca; |
| 403; | 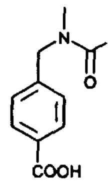 ; | cyclohexyl; | Val; | 1-NpCH$_2$O; | Acca; |
| 404; | 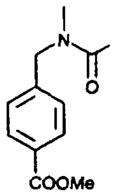 ; | cyclohexyl; | Val; | 1-NpCH$_2$O; | Acca; |
| 405; | HOOC-CH$_2$CH$_2$-N(Me)C(O)-; | cyclohexyl; | Val; | 1-NpCH$_2$O; | Acca; |
| 406; | MeOOC-CH$_2$-CH$_2$-N(Me)C(O)-; | cyclohexyl; | Val; | 1-NpCH$_2$O; | Acca; |
| 407; | HOOC-CH$_2$CH$_2$-N(CH(Me)$_2$)-C(O)-; | cyclohexyl; | Val; | 1-NpCH$_2$O; | Acca; |

| Cpd; | B; | $R_4$; | P3; | $R_{13}$; | P1; |
|---|---|---|---|---|---|
| 408; | MeOOC-$(CH_2)_2$-N(CH(Me)$_2$)-C(O)-; | cyclohexyl; | Val; | 1-$NpCH_2O$; | Acca; |
| 409; | HOOC-$CH_2$-N(CH(Me)$_2$)-C(O)-; | cyclohexyl; | Val; | 1-$NpCH_2O$; | Acca; |
| 410; | EtOOC-$CH_2$-N(CH(Me)$_2$)-C(O)-; | cyclohexyl; | Val; | 1-$NpCH_2O$; | Acca; |
| 411; | HOOC-$(CH_2)_3$-N(CH(Me)$_2$)-C(O); | cyclohexyl; | Val; | 1-$NpCH_2O$; | Acca; |
| 412; | [HOOC-$CH_2$]$_2$-NC(O)-; | cyclohexyl; | Val; | 1-$NpCH_2O$; | Acca; |
| 413; | [HOOC-$(CH_2)_2$]$_2$-NC(O)-; | cyclohexyl; | Val; | 1-$NpCH_2O$; | Acca; |
| 414; | 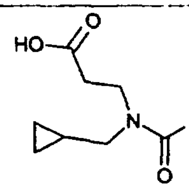 ; | cyclohexyl; | Val; | 1-$NpCH_2O$; | Acca; |
| 415; | 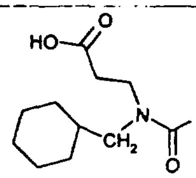 ; | cyclohexyl; | Val; | 1-$NpCH_2O$; | Acca; |
| 416; | 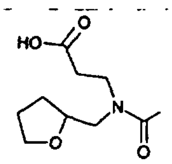 ; | cyclohexyl; | Val; | 1-$NpCH_2O$; | Acca; |
| 417; | 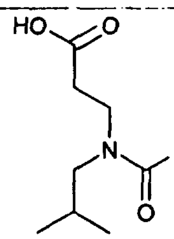 ; | cyclohexyl; | Val; | 1-$NpCH_2O$; | Acca; |

| Cpd; | B; | R4; | P3; | R13; | P1; |
|---|---|---|---|---|---|
| 418; | 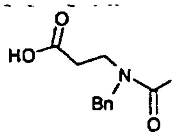 ; | cyclohexyl; | Val; | 1-NpCH2O; | Acca; |
| 419; | 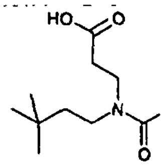 ; | cyclohexyl; | Val; | 1-NpCH2O; | Acca; |
| 420; | 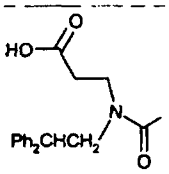 ; | cyclohexyl; | Val; | 1-NpCH2O; | Acca; |
| 421; | 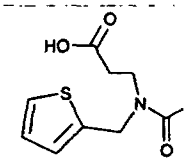 ; | cyclohexyl; | Val; | 1-NpCH2O; | Acca; |
| 422; | 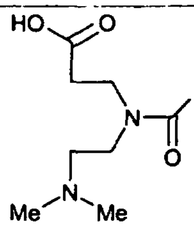 ; | cyclohexyl; | Val; | 1-NpCH2O; | Acca; |
| and 423; | 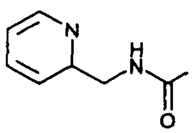 ; | cyclohexyl; | Val; | 1-NpCH2O; | Acca. |

**38.** Use of an intermediate of formula:

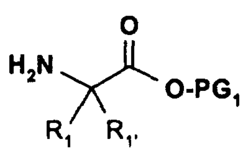

wherein $R_1$ and $R_{1'}$ form a 3 membered-ring optionally substituted with $C_{1-6}$ alkyl, for the synthesis of a compound of formula I according to any one of the preceding claims.

**39.** The use according to claim 38, wherein said carboxyl protecting group (PG1) is selected from the group consisting of: alkyl esters, aralkyl esters, and esters being cleavable by mild base treatment or mild reductive means.

## Patentansprüche

**1.** Verbindung der Formel (I):

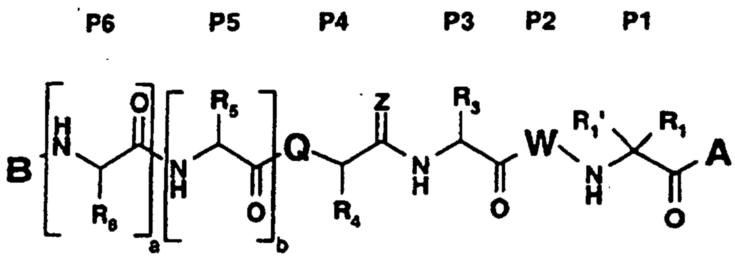

(I)

worin Q $CH_2$ oder N-Y ist, worin Y H oder $C_{1-6}$-Alkyl ist;

a) wenn Q $CH_2$ ist, a 0 ist, b 0 ist und B ein Amidderivat der Formel $R_{11a}N(R_{11b})$-C(O) ist, worin $R_{11a}$ H; $C_{1-10}$-Alkyl, gegebenenfalls substituiert mit Carboxyl oder Di($C_{1-6}$-alkyl)amino; $C_{3-7}$-Cycloalkyl; $C_6$-Aryl; $C_{7-10}$-Alkylaryl; ($C_{3-7}$-Cycloalkyl)-($C_{1-6}$-alkyl); Heterocyclus-$C_{1-6}$-alkyl ist;
und $R_{11b}$ $C_{1-6}$-Alkyl, substituiert mit Carboxyl, ($C_{1-6}$-Alkoxy)carbonyl oder Phenylmethoxycarbonyl; oder $C_{7-16}$-Aralkyl, substituiert am aromatischen Teil mit Carboxyl, ($C_{1-6}$-Alkoxy)carbonyl, Phenylmethoxycarbonyl oder Heterocyclus-$C_{1-6}$-alkyl ist;
oder $R_{11a}$ und $R_{11b}$ unter Bildung eines 3- bis 7-gliedrigen, Stickstoff enthaltenden Rings, gegebenenfalls substituiert mit Carboxyl oder ($C_{1-6}$-Alkoxy)-carbonyl, verbunden sind;
oder
b) wenn Q N-Y ist; a 0 oder 1 ist, b 0 oder 1 ist und B ein Acylderivat der Formel $R_{11}$-C(O)- ist, worin $R_{11}$ (i) $C_{1-10}$-Alkyl, gegebenenfalls substituiert mit Carboxyl, $C_{1-6}$-Alkanoyloxy (z.B. $AcOCH_2$) oder $C_{1-6}$-Alkoxy (z.B. Boc); ii) $C_{3-7}$-Cycloalkyl, gegebenenfalls substituiert mit Carboxyl, ($C_{1-6}$-Alkoxy)carbonyl oder Phenylmethoxycarbonyl; (iii) $C_{3-7}$-Cycloalkyl, substituiert mit Carboxyl und einem bis drei $C_{1-6}$-Alkylsubstituenten; (iv) $C_{4-10}$-(Alkylcycloalkyl), gegebenenfalls substituiert am Cycloalkylteil mit Carboxy, ($C_{1-6}$-Alkoxy)carbonyl oder Phenylmethoxycarbonyl; (v)

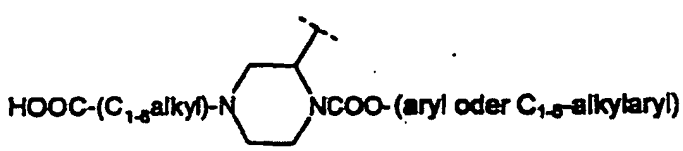

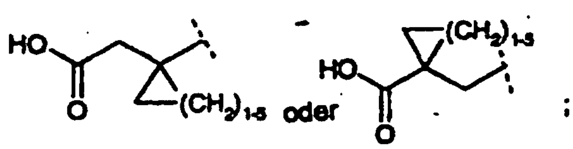

(vi) $C_6$- oder $C_{10}$-Aryl oder $C_{7-16}$-Aralkyl, gegebenenfalls substituiert mit $C_{1-6}$-Alkyl, ist;
$R_6$, wenn vorhanden, $C_{1-6}$-Alkyl, substituiert mit Carboxyl ist; und
$R_5$, wenn vorhanden, $C_{1-6}$-Alkyl, gegebenenfalls substituiert mit Carboxyl ist;
oder
c) wenn Q entweder $CH_2$ oder N-Y ist;
$R_4$ $C_{1-10}$-Alkyl, $C_{3-7}$-Cycloalkyl oder $C_{4-10}$-(Alkylcycloalkyl) ist;
Z Oxo oder Thioxo ist;
$R_3$ $C_{1-10}$-Alkyl, gegebenenfalls substituiert mit Carboxyl, $C_{3-7}$-Cycloalkyl oder $C_{4-10}$-(Alkylcycloalkyl) ist;
W eine Gruppe der Formel III' ist:

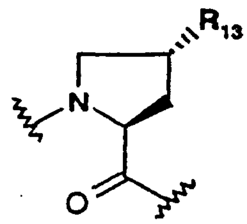

Formel III'

worin $R_{13}$ OH; SH; $NH_2$; Carboxyl; $R_{12}$; $OR_{12}$, $SR_{12}$, $NHR_{12}$ oder $NR_{12}R_{12}'$ ist, worin $R_{12}$ und $R_{12}'$ unabhängig sind: cyclisches $C_{3-16}$-Alkyl oder acyclisches $C_{1-16}$-Alkyl oder cyclisches $C_{3-16}$-Alkenyl oder acyclisches $C_{2-16}$-Alkenyl, wobei das Alkyl oder Alkenyl gegebenenfalls substituiert sind mit $NH_2$, OH, SH, Halogen oder Carboxyl; wobei Alkyl oder Alkenyl gegebenenfalls mindestens ein Heteroatom enthalten, das unabhängig ausgewählt ist aus der Gruppe bestehend aus: O, S und N;
oder
$R_{12}$ und $R_{12}'$ unabhängig $C_6$- oder $C_{10}$-Aryl oder $C_{7-16}$-Aralkyl, gegebenenfalls substituiert mit $C_{1-6}$-Alkyl, $NH_2$, OH, SH, Halogen, Carboxyl oder Carboxy-$C_{1-6}$alkyl sind; wobei das Aryl oder Aralkyl gegebenenfalls mindestens ein Heteroatom enthalten, das unabhängig ausgewählt ist aus der Gruppe bestehend aus: O, S und N;
wobei das cyclische Alkyl, cyclische Alkenyl, Aryl oder Aralkyl gegebenenfalls mit einem zweiten 5-, 6- oder 7-gliedrigen Ring kondensiert sind, um ein cyclisches System oder ein heterocyclisches System zu bilden, wobei der zweite Ring gegebenenfalls substituiert ist mit $NH_2$, OH, SH, Halogen, Carboxyl oder Carboxy-$C_{1-6}$-alkyl; wobei der zweite Ring gegebenenfalls mindestens ein Heteroatom enthält, das unabhängig ausgewählt ist aus der Gruppe bestehend aus: O, S und N;
$R_1'$ Wasserstoff ist und $R_1$ Propyl ist; oder
$R_1'$ und $R_1$ zusammen einen 3-gliedrigen Ring gegebenenfalls substituiert mit $C_{1-6}$-Alkyl bilden; und
A Hydroxy oder ein pharmazeutisch annehmbares Salz oder ein Ester davon ist.

2. Verbindung nach Anspruch 1 der Formel (Ia):

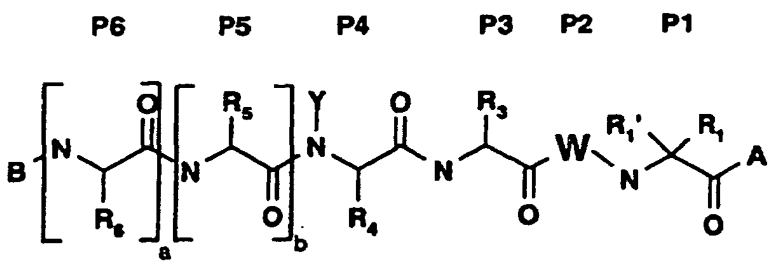

(Ia)

worin Y H oder $C_{1-6}$-Alkyl ist;
a 0 oder 1 ist;
b 0 oder 1 ist;
B ein Acylderivat der Formel $R_{11}$-C(O)- ist, worin $R_3$, $R_4$, $R_5$, $R_6$, $R_{11}$, W, $R_1$, $R_1'$ und A wie in Anspruch 1 definiert sind.

3. Verbindung der Formel Ia nach Anspruch 2, worin B ein Acylderivat der Formel $R_{11}C(O)$- ist, worin $R_{11}$:

$C_{1-6}$-Alkyl, gegebenenfalls substituiert mit Carboxyl, $C_{1-6}$-Alkanoyloxy oder $C_{1-6}$-Alkoxy;
$C_{3-7}$-Cycloalkyl, gegebenenfalls substituiert mit Carboxyl, MeOC(O), EtOC(O) oder BnOC(O);
3-Carboxypropionyl (DAD) oder 4-Carboxybutyryl (DAE); oder

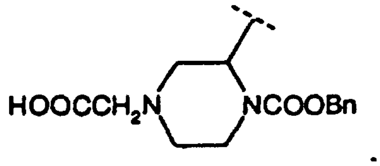

.

4. Verbindung der Formel Ia nach Anspruch 3, worin B Acetyl, 3-Carboxypropionyl, 4-Carboxybutyryl, $AcOCH_2C(O)$, $Me_3COC(O)$,

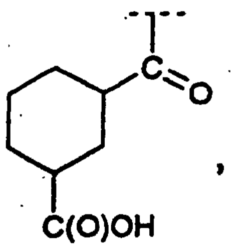

,

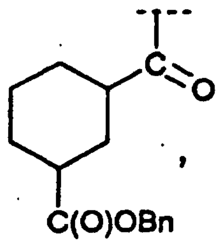

,

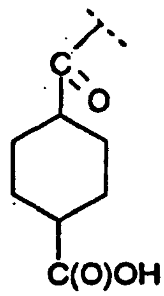

,

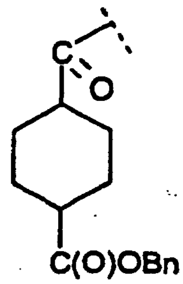

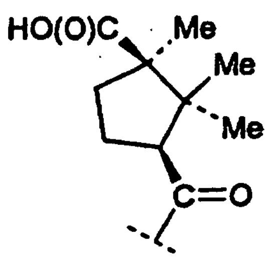

,

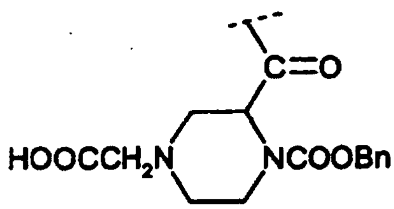

,

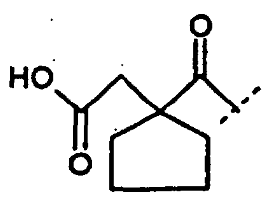

,

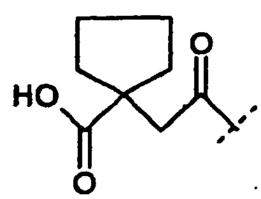

ist.

5. Verbindung der Formel Ia nach Anspruch 4, worin B Acetyl, 3-Carboxypropionyl (DAD), 4-Carboxybutyryl (DAE), $AcOCH_2C(O)$

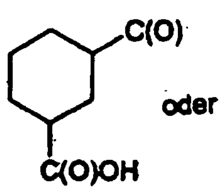

oder

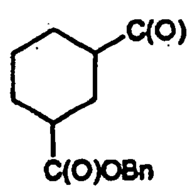

ist.

**6.** Verbindung der Formel Ia nach Anspruch 5, worin B Acetyl ist.

**7.** Verbindung der Formel Ia nach Anspruch 2, worin $R_6$, falls vorhanden, die Seitenkette von Asp oder Glu ist.

**8.** Verbindung der Formel Ia nach Anspruch 7, worin $R_6$, falls vorhanden, die Seitenkette von Asp ist.

**9.** Verbindung der Formel Ia nach Anspruch 2, worin $R_5$, falls vorhanden, die Seitenkette einer Aminosäure ist, die ausgewählt ist aus der Gruppe bestehend aus D-Asp, Asp, D-Glu, Glu, D-Val, Val, D-Tbg und Tbg.

**10.** Verbindung der Formel Ia nach Anspruch 9, worin $R_5$, falls vorhanden, die Seitenkette von D-Asp, D-Val oder D-Glu ist.

**11.** Verbindung der Formel Ia nach Anspruch 10, worin $R_5$, falls vorhanden, die Seitenkette von D-Glu ist.

**12.** Verbindung nach Anspruch 1 der Formel (Ib):

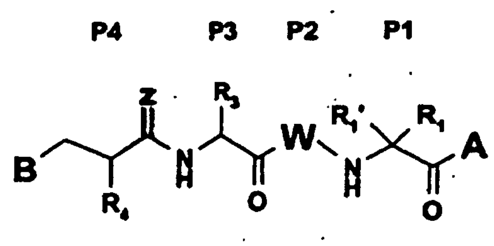

(Ib)

worin B ein Amid der Formel $R_{11a}N(R_{11b})C(O)$- ist, worin $R_{11a}$ $C_{1-6}$-Alkyl, gegebenenfalls substituiert mit Carboxy ist oder $R_{11a}$ $C_6$-Aryl, $C_{7-10}$-Arylalkyl, 2-Tetrahydrofuranylmethyl oder 2-Thiazolidylmethyl ist; und $R_{11b}$ $C_{1-6}$-Alkyl substituiert mit Carboxyl ist.

**13.** Verbindung der Formel (Ib) nach Anspruch 12, worin $R_{11a}$ Isopropyl, Carboxyethyl, Benzylmethyl, Benzyl oder 2-Tetrahydrofuranylmethyl ist.

**14.** Verbindung der Formel (Ib) nach Anspruch 13, worin $R_{11b}$ $C_{1-4}$-Alkyl substituiert mit Carboxyl ist.

**15.** Verbindung der Formel (Ib) nach Anspruch 14, worin $R_{11b}$ Ethylcarboxyl ist.

**16.** Verbindung der Formel I nach Anspruch 1, worin $R_4$ ausgewählt ist aus der Gruppe bestehend aus: Isopropyl, Cyclopropyl, tert.-Butyl, 1-Methylpropyl oder 2-Methylpropyl.

**17.** Verbindung der Formel I nach Anspruch 16, worin $R_4$ Cyclopropyl oder 1-Methylpropyl ist.

**18.** Verbindung der Formel Ia nach Anspruch 17, worin $R_4$ Cyclopropyl ist.

**19.** Verbindung der Formel I nach Anspruch 1, worin Z Oxo ist.

**20.** Verbindung der Formel I nach Anspruch 1, worin $R_3$ die Seitenkette von Ile, allo-Ile, Chg, Cha, Val, Tbg oder Glu ist.

**21.** Verbindung der Formel I nach Anspruch 20, worin $R_3$ die Seitenkette von Val, Tbg oder Chg ist.

**22.** Verbindung der Formel I nach Anspruch 21, worin $R_3$ die Seitenkette von Val ist.

**23.** Verbindung der Formel I nach Anspruch 1, worin $R_{13}$ $OR_{12}$ oder $SR_{12}$ ist, worin $R_{12}$ ein $C_6$- oder $C_{10}$-Aryl oder $C_{7-16}$-Aralkyl ist, wobei das genannte erste Aryl oder Aralkyl gegebenenfalls substituiert ist mit $C_{1-6}$-Alkyl, $C_{3-7}$-Cycloalkyl, $NH_2$, OH, SH, Halogen, $C_{1-6}$-Alkoxy, Carboxyl, Carboxy-$C_{1-6}$-alkyl oder einem zweiten Aryl oder Aralkyl;

wobei das genannte erste und zweite Aryl oder Aralkyl gegebenenfalls mindestens ein Heteroatom enthalten, das unabhängig ausgewählt ist aus der Gruppe bestehend aus: O, S und N.

**24.** Verbindung nach Anspruch 23, worin $R_{13}$ Bn; $PhCH_2CH_2$; $PhCH_2CH_2CH_2$; o-Bn; o-Tolylmethoxy; m-Tolylmethoxy; p-Tolylmethoxy; 1-Naphthyloxy; 2-Naphtyloxy; 1-Naphthalinylmethoxy; 2-Naphthalinylmethoxy; (4-tert.-Butyl)-methoxy; (3I-Ph)$CH_2O$; (4Br-Ph)O; (2Br-Ph)O; (3Br-Ph)O; (4I-Ph)O; (3Br-Ph)$CH_2O$; (3,5-$Br_2$-Ph)$CH_2O$;

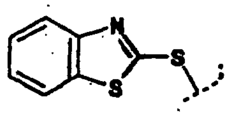 ; 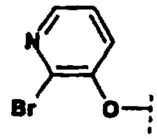 ; 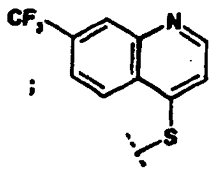 ; 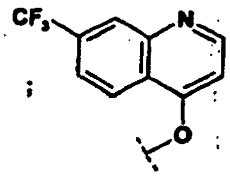 ;

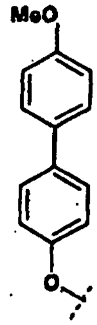 ; 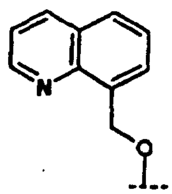 ; 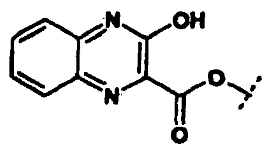 ;

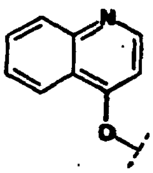 ; 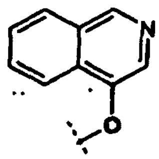 ; 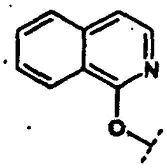 ; 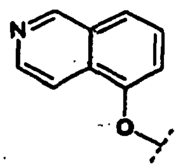 ;

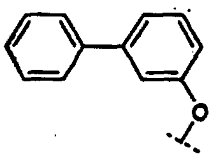 ; 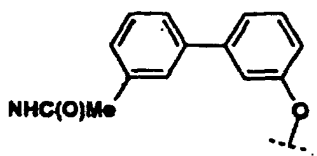 ;

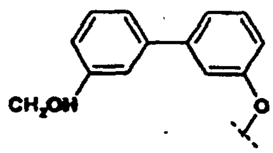 ; 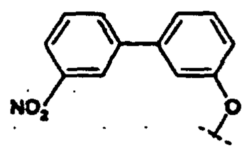 ;

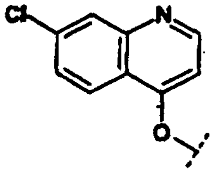 ; 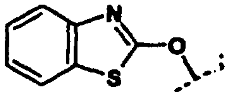 ; 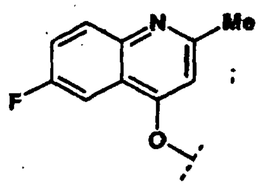 ;

ist.

**25.** Verbindung nach Anspruch 24, worin $R_{13}$ O-Bn; $PhCH_2CH_2CH_2$; 1-Naphthyloxy; 2-Naphthyloxy; 1-Naphthalinylmethoxy; 2-Naphthalinylmethoxy;

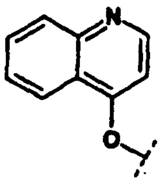 ; 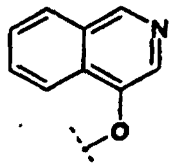 ; 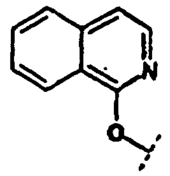 ; 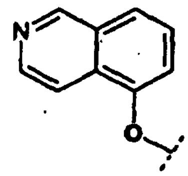 ;

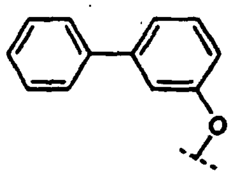 ; 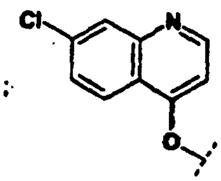

ist.

**26.** Verbindung der Formel I nach Anspruch 1, worin

a) Q $CH_2$ ist, a 0 ist, b 0 ist und B ein Amid der Formel $R_{11a}N(R_{11b})$-C(O)- ist, worin $R_{11a}$ $C_{1\text{-}6}$-Alkyl, gegebenenfalls substituiert mit Carboxyl ist oder $R_{11a}$ Phenyl, $C_{7\text{-}10}$-Arylalkyl, 2-Tetrahydrofuranylmethyl oder 2-Thienylmethyl ist;
und $R_{11b}$ ($C_{0\text{-}2}$-Alkyl)phenyl, gegebenenfalls substituiert mit Carboxyl oder ($C_{1\text{-}4}$-Alkoxy)carbonyl; oder $C_{1\text{-}6}$-Alkyl substituiert mit Carboxyl oder ($C_{1\text{-}4}$-Alkoxy)carbonyl ist; oder $R_{11a}$ und $R_{11b}$ unter Bildung eines Piperidinrings, gegebenenfalls substituiert mit Carboxyl oder ($C_{1\text{-}6}$-Alkoxy)carbonyl, verbunden sind;
oder
b) Q N-Y ist, worin Y H oder $C_{1\text{-}6}$-Alkyl ist; a 0 oder 1 ist, b 0 oder 1 ist und B ein Acylderivat der Formel $R_{11}$-C(O)- ist, worin $R_{11}$ (i) $C_{1\text{-}6}$-Alkyl, $C_{1\text{-}6}$-Alkyl substituiert mit Carboxyl, MeC(O)O-, MeO-, EtO-, $MeCH_2CH_2O$- oder $Me_3C$-O-; (ii) Cyclopentyl oder Cyclohexyl, gegebenenfalls substituiert mit Carboxyl; (iv) $C_{4\text{-}10}$-(Alkylcycloalkyl), gegebenenfalls substituiert am Cycloalkylteil mit Carboxyl;
(v)

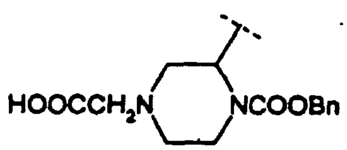

(vi) Phenyl, Benzyl oder Phenylethyl ist;
$R_6$, falls vorhanden, $CH_2COOH$ oder $CH_2CH_2COOH$ ist,
$R_5$, falls vorhanden, $C_{1\text{-}6}$-Alkyl oder $CH_2COOH$ oder $CH_2CH_2COOH$ ist; oder
c) wenn Q entweder $CH_2$ oder N-Y ist,
$R_4$ $C_{1\text{-}6}$-Alkyl, $C_{3\text{-}7}$-Cycloalkyl oder $C_{4\text{-}10}$-(Alkylcycloalkyl) ist;
Z Oxo oder Thio ist;
$R_3$ $C_{1\text{-}6}$-Alkyl; $C_{3\text{-}7}$-Cycloalkyl oder $C_{4\text{-}10}$-(Alkylcycloalkyl) ist;
$R_{1'}$ Wasserstoff ist und $R_1$ Propyl ist; oder $R_{1'}$ und $R_1$ zusammen mit dem Kohlenstoffatom, an dem sie gebunden sind, ein Cyclopropyl bilden, das gegebenenfalls mit Ethyl substituiert sein kann; und
W wie in Anspruch 1 definiert ist; und
A Hydroxy oder ein pharmazeutisch annehmbares Salz davon; $C_{1\text{-}6}$-Alkoxy oder (Aryl-$C_{1\text{-}6}$-alkoxy) ist.

**27.** Verbindung der Formel Ia nach Anspruch 2, worin B ein Acylderivat der Formel $R_{11}$-C(O)- ist, worin $R_{11}$ $C_{1-6}$-Alkoxy, $C_{1-10}$-Alkyl, gegebenenfalls substituiert mit Carboxyl; $C_{3-7}$-Cycloalkyl, gegebenenfalls substituiert mit Carboxyl oder Benzylcarboxy; oder

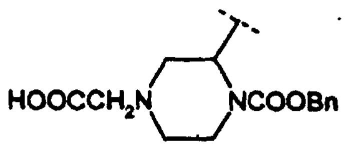

ist;
$R_6$ abwesend ist;
$R_5$ abwesend ist;
$R_4$ $C_{1-10}$-Alkyl, $C_{3-7}$-Cycloalkyl oder $C_{4-10}$-(Alkylcycloalkyl) ist;
$R_3$ $C_{1-10}$-Alkyl, $C_{3-7}$-Cycloalkyl oder $C_{4-10}$-(Alkylcycloalkyl) ist;
und
W wie in Anspruch 1 definiert ist; und
A Hydroxy oder ein pharmazeutisch annehmbares Salz davon; Methoxy, Ethoxy, Phenoxy oder Benzyloxy ist.

**28.** Verbindung der Formel Ia nach Anspruch 2, worin B Acetyl, 3-Carboxypropionyl, 4-Carboxylbutyryl, $AcOCH_2C(O)$, $Me_3COC(O)$,

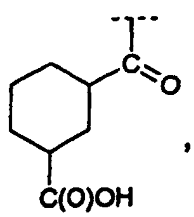 , 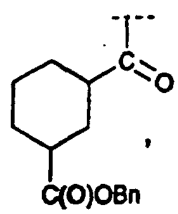 , 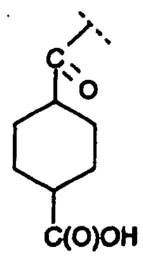 , 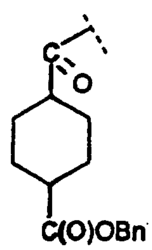

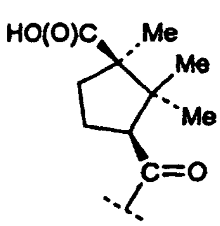 , 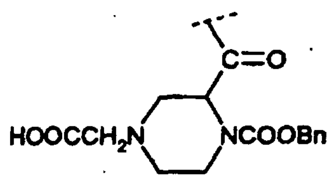 ,

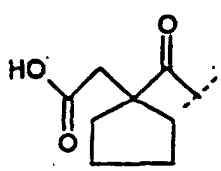 , 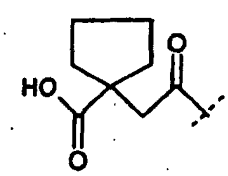

ist;
Y H oder Me ist, a 0 oder 1 ist, b 0 oder 1 ist,
$R_6$, falls vorhanden, die Seitenkette von Asp oder Glu ist,
$R_5$, falls vorhanden, die Seitenkette von Asp, D-Asp, Glu, D-Glu, Val, D-Val oder Tbg ist,
$R_4$ die Seitenkette von Val, Chg, Tbg, Ile oder Leu ist,
$R_3$ Wasserstoff oder die Seitenkette von Ile, Chg, Val, Glu ist;
W eine Gruppe der Formel III' ist:

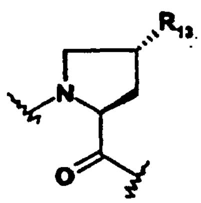

(III')

worin $R_{13}$ Bn, $PhCH_2CH_2$, $PhCH_2CH_2CH_2$, O-Bn, o-Tolylmethoxy, m-Tolylmethoxy, p-Tolylmethoxy, 1-Naphthalinylmethoxy, 2-Naphthalinylmethoxy, (4-tert.-Butyl)benzyloxy, (3I-Ph)$CH_2O$, (4Br-Ph)O, (2Br-Ph)O, (3Br-Ph)O, (4I-PH)O, (3Br-Ph)$CH_2O$, (3,5-$Br_2$-Ph)$CH_2O$,

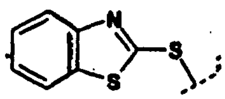 ; 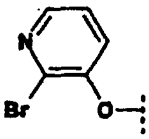 ; 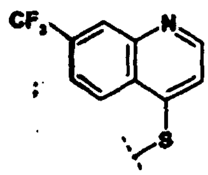 ; 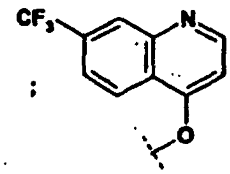 ;

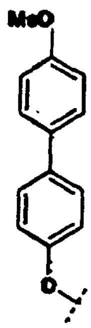 ; 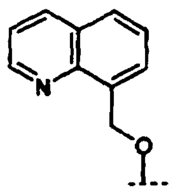 ; 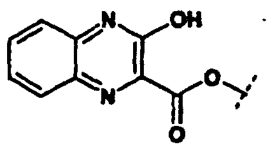 ;

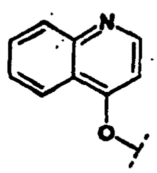 ; 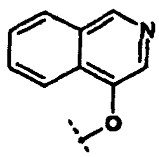 ; 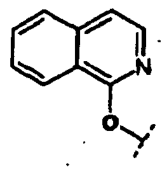 ; 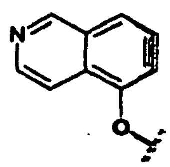 ;

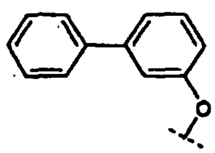 ; 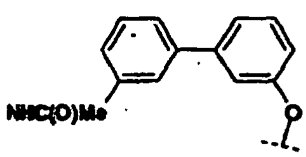 ;

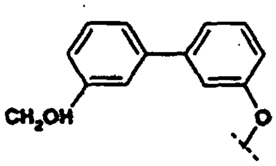 ; 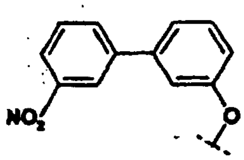 ;

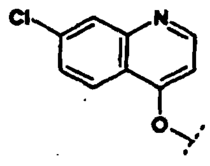 ; 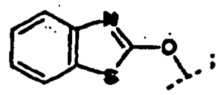 ; 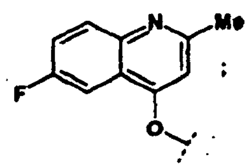 ;

ist;

$R_{1'}$ H ist und $R_1$ Propyl ist; oder

$R_{1'}$ und $R_1$ zusammen mit dem Kohlenstoffatom, an dem sie gebunden sind, ein Cyclopropyl bilden; und A Hydroxyl ist.

**29.** Verbindung der Formel Ib nach Anspruch 12, worin B ein Amid der Formel $R_{11a}N(R_{11b})$-C(O)- ist, worin $R_{11a}$ $C_{1-6}$-Alkyl, gegebenenfalls substituiert mit Carboxyl ist oder $R_{11a}$ 2-Tetrahydrofuranylmethyl ist; und $R_{11b}$ ($C_{0-2}$-Alkyl)-phenyl, gegebenenfalls substituiert mit Carboxyl oder ($C_{1-4}$-Alkoxy)carbonyl; oder $C_{1-6}$-Alkyl substituiert mit Carboxyl oder ($C_{1-4}$-Alkoxy)carbonyl ist; oder $R_{11a}$ und $R_{11b}$ unter Bildung eines Piperidinrings, gegebenenfalls substituiert mit Carboxyl oder ($C_{1-6}$-Alkoxy)carbonyl verbunden sind;

$R_4$ Cyclohexyl ist,

Z Oxo ist;

$R_3$ Wasserstoff oder die Seitenkette von Ile, Chg, Val, Glu ist;

W eine Gruppe der Formel III' ist:

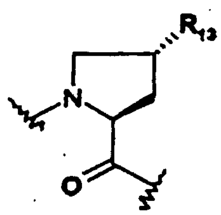 (III')

worin $R_{13}$ Bn, $PhCH_2CH_2$, $PhCH_2CH_2CH_2$, O-Bn, o-Tolylmethoxy, m-Tolylmethoxy, p-Tolylmethoxy, 1-Naphthalinylmethoxy, 2-Naphthalinylmethoxy, (4-tert.-Butyl)methoxy, (3I-Ph)$CH_2O$, (4Br-Ph)O, (2Br-Ph)O, (3Br-Ph)O, (4I-Ph)O, (3Br-Ph)$CH_2O$, (3,5-$Br_2$-Ph)$CH_2O$,

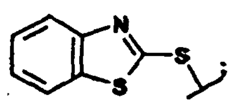 ; 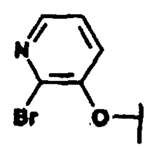 ; 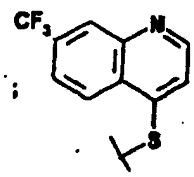 ; 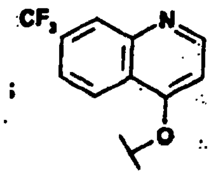 ;

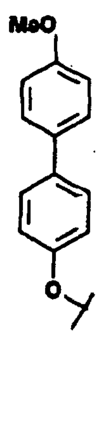 ; 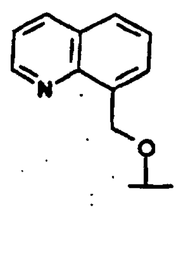 ; 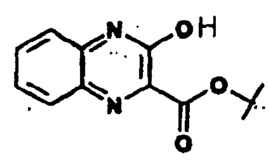 ;

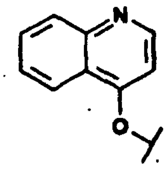 ; 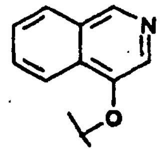 ; 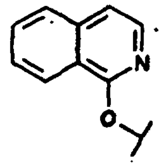 ; 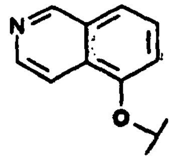

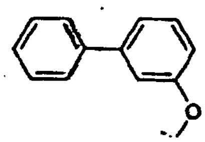 ; 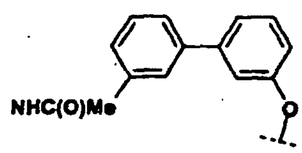 ;

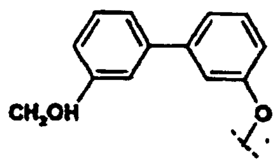 ; 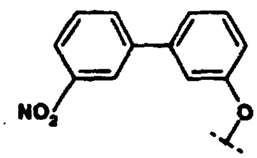 ;

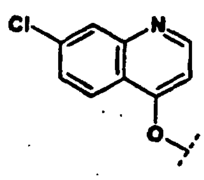 ; 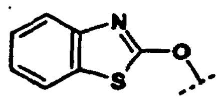 ; 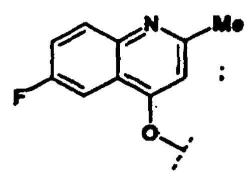 ;

ist;
$R_{1'}$ H ist und $R_1$ Propyl ist; oder
$R_{1'}$ und $R_1$ zusammen mit dem Kohlenstoffatom, an dem sie gebunden sind, ein Cyclopropyl bilden; und A Hydroxyl ist.

**30.** Verbindung der Formel I nach Anspruch 1, worin B ein Acylderivat der Formel $R_{11}$-C(O)- ist, worin $R_{11}$ $C_{1-10}$-Alkyl, gegebenenfalls substituiert mit Carboxyl; $C_{3-7}$-Cycloalkyl, gegebenenfalls substituiert mit Carboxyl; oder ein $C_{4-10}$-(Alkylcycloalkyl), gegebenenfalls substituiert am Cycloalkylteil mit Carboxyl, ist; oder $R_{11}$ $C_6$- oder $C_{10}$-Aryl oder $C_{7-16}$-Aralkyl, gegebenenfalls substituiert mit einem $C_{1-6}$-Alkyl ist;
a 0 oder 1 ist;
$R_6$, falls vorhanden, $C_{1-6}$-Alkyl, gegebenenfalls substituiert mit Carboxyl ist;
b 0 oder 1 ist;
$R_5$, falls vorhanden, $C_{1-6}$-Alkyl, gegebenenfalls substituiert mit Carboxyl ist;
Q N-Y ist und Y H oder $C_{1-6}$-Alkyl ist;
$R_4$ $C_{1-10}$-Alkyl, $C_{3-7}$-Cycloalkyl oder $C_{4-10}$-(Alkylcycloalkyl) ist;
Z Oxo ist,
$R_3$ $C_{1-10}$-Alkyl, $C_{3-7}$-Cycloalkyl oder $C_{4-10}$-(Alkylcycloalkyl) ist;
und
W, $R_1$' und $R_1$ wie in Anspruch 1 definiert sind; und
A Hydroxyl oder ein pharmazeutisch annehmbares Salz oder ein Ester davon ist.

**31.** Pharmazeutische Zusammensetzung, umfassend eine bezüglich Hepatitis C antiviral wirksame Menge einer Verbindung der Formel I nach Anspruch 1 oder eines therapeutisch annehmbaren Salzes oder Esters davon in Mischung mit einem pharmazeutisch annehmbaren Trägermedium oder Hilfsmittel.

**32.** In-vitro-Verfahren zur Inhibierung der Replikation des Hepatitis C-Virus, in dem der Virus einer Hepatitis C-Virus-NS3-Protease inhibierenden Menge der Verbindung der Formel I nach Anspruch 1 oder eines therapeutisch annehmbaren Salzes oder Esters davon ausgesetzt wird.

**33.** Verwendung einer Verbindung der Formel I nach Anspruch 1 zur Herstellung eines Arzneimittels zur Behandlung einer Hepatitis C-Infektion in einem Säuger.

**34.** Verbindung, ausgewählt aus der Gruppe bestehend aus einer Verbindung der Formel

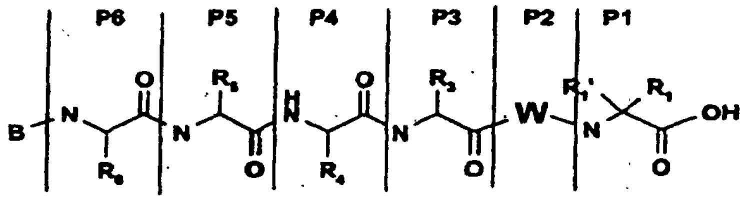

worin B, P6, P5, P4, P3, W und P1 wie nachstehend definiert sind:

| Vbdg. Nr. ; | B; | P6; | P5; | P4; | P3; | W; | P1; |
|---|---|---|---|---|---|---|---|
| 123; | Ac; | Asp; | Asp; | Ile; | Val; | Pro; | Nva; |
| 126; | Ac; | Asp; | Asp; | Ile; | Val; | Pro; | Acca; |
| 127; | Ac; | Asp; | Asp; | Ile; | Val; | Pip; | Nva; |
| 128; | Ac; | Asp; | D-Glu; | Ile; | Val; | Pro; | Nva; |
| 129; | Ac; | Asp; | Tbg; | Ile; | Val; | Pro; | Nva; |
| 130; | DAD; | ---; | Asp; | Ile; | Val; | Pro; | Nva; |
| 132; | Ac; | Asp; | D-Glu; | Chg; | Glu; | Glu; | Acca; |
| 133; | Ac; | Asp; | Glu; | Chg; | Val; | Glu(OBn); | Acca. |

| Vbdg. Nr. ; | B; | P6; | P5; | P4; | P3; | W; | P1; |
|---|---|---|---|---|---|---|---|
| 303; | Ac; | Asp; | Asp; | Ile; | Val; | 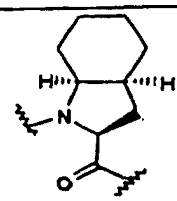 ; | Nva; |
| und 304; | Ac; | ---; | ---; | Chg; | Val; | 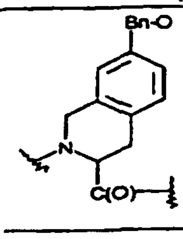 ; | Acca. |

**35.** Verbindung nach Anspruch 1, ausgewählt aus der Gruppe bestehend aus einer Verbindung der Formel

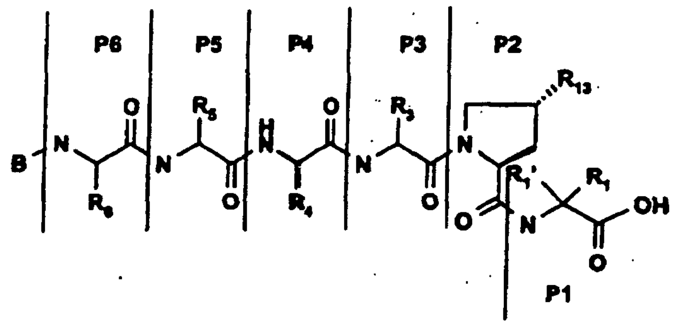

worin B, P6, P5, P4, P3, $R_{13}$ und $P_1$ wie nachstehend definiert sind:

| Vbdg. Nr.; | B; | P6; | P5; | P4; | P3; | $R_{13}$; | P1; |
|---|---|---|---|---|---|---|---|
| 201; | Ac | Asp | Asp; | Ile; | Val; | O-Bn; | Nva; |
| 202; | Ac | Asp | D-Val; | Ile; | Val; | O-Bn; | Nva; |
| 203; | Ac | Asp | D-Glu; | Ile; | Val; | O-Bn; | Nva; |
| 204; | Ac | Asp | Asp; | Ile; | Val; | o-Tolyl-methoxy; | Nva; |
| 205; | Ac | Asp | Asp; | Ile; | Val; | m-Tolyl-methoxy; | Nva; |
| 206; | Ac | Asp | Asp; | Ile; | Val; | p-Tolyl-methoxy; | Nva; |
| 207; | Ac | Asp | Asp; | Ile; | Val; | 1-NpCH$_2$O; | Nva; |
| 208; | Ac | Asp | Asp; | Ile; | Val; | 2-NpCH$_2$O; | Nva; |

| Vbdg. Nr.; | B; | P6; | P5; | P4; | P3; | $R_{13}$; | P1; |
|---|---|---|---|---|---|---|---|
| 209; | Ac | Asp | Asp; | Ile; | Val; | 4-tert.-Butyl-phenyl-methoxy | Nva; |
| 211; | Ac | Asp | D-Glu; | Chg; | Val; | O-Bn; | Nva; |
| 212; | Ac | Asp | D-Glu; | Ile; | Val; | O-Bn; | Acca; |
| 213; | Ac | Asp | D-Glu; | Ile; | Val; | 2-$NpCH_2O$; | Nva; |
| 214; | Ac | Asp | D-Glu; | Chg; | Val; | 2-$NpCH_2O$; | Nva; |
| 215; | Ac | Asp | D-Glu; | Chg; | Val; | 1-$NpCH_2O$; | Acca; |
| 216; | Ac | Asp | Asp; | Ile; | Val; | Bn; | Nva; |
| 217; | Ac | Asp | Asp; | Ile; | Val; | $Ph(CH_2)_3$; | Nva; |
| 218; | Ac | Asp | D-Glu; | Ile; | Val; | O-Bn; | Nva; |
| 219; | Ac; | ---; | Asp; | Ile; | Val; | 1-$NpCH_2O$; | Nva; |
| 220; | DAD; | ---; | ---; | *N*(Me)Ile; | Val; | 1-$NpCH_2O$; | Nva; |
| 221; | DAD; | ---; | ---; | Ile; | Val; | 1-$NpCH_2O$; | Nva; |
| 222; | DAE; | ---; | ---; | Ile; | Val; | 1-$NpCH_2O$; | Nva; |
| 223; | 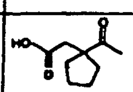 ; | ---; | ---; | Ile; | Val; | 1-$NpCH_2O$; | Nva; |
| 224; | 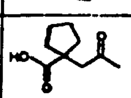 ; | ---; | ---; | Ile; | Val; | 1-$NpCH_2O$; | Nva; |
| 225; | Ac; | ---; | ---; | Ile; | Val; | 1-$NpCH_2O$; | Nva; |
| 226; | DAE; | ---; | ---; | Chg; | Val | 1-$NpCH_2O$; | Acca; |
| 227; | Ac; | ---; | ---; | Chg; | Val; | 1-$NpCH_2O$; | Acca; |
| 228; | Ac; | ---; | ---; | Chg; | Val; | O-Bn; | 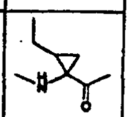 ; |
| 230; | Ac; | Asp; | Asp; | Ile; | Val; | $Ph(CH_2)_3$; | Nva; |
| 231; | Ac; | ---; | ---; | Chg; | Chg; | 1-$NpCH_2O$; | Acca; |

| Vbdg. Nr.; | B; | P6; | P5; | P4; | P3; | $R_{13}$; | P1; |
|---|---|---|---|---|---|---|---|
| 232; | $AcOCH_2$-C(O); | ---; | ---; | Chg; | Chg; | 1-$NpCH_2O$; | Acca; |
| 233; | Ac; | Asp; | Glu; | Ile; | Val; | (3I-Ph)$CH_2O$; | Acca; |
| 234; | Ac; | ---; | ---; | Chg; | Chg; | O-Bn; | Acca; |
| 235; | Boc; | ---; | ---; | Chg; | Chg; | 1-$NpCH_2O$; | Acca; |
| 236; | Ac; | ---; | Gly; | thioxo-Ile; | Val; | 1-$NpCH_2O$; | Nva; |
| 237; | DAE; | ---; | ---; | Ile; | Val; | 1-$NpCH_2O$; | Acca; |
| 238; | Ac; | ---; | ---; | Chg; | Val; | (4Br-Ph)O; | Acca; |
| 239; | Ac; | ---; | ---; | Chg; | Val; | (2Br-Ph)O; | Acca; |
| 240; | Ac; | ---; | ---; | Chg; | Val; | (3Br-Ph)O; | Acca; |
| 241; | Ac; | ---; | ---; | Chg; | Val; | 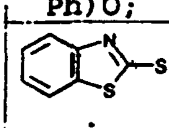 ; | Acca; |
| 242; | Ac; | ---; | ---; | Chg; | Val; | (4Br-Ph)S; | Acca; |
| 243; | Ac; | ---; | ---; | Chg; | Val; | 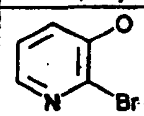 ; | Acca; |
| 244; | Ac; | ---; | ---; | Chg; | Val; | 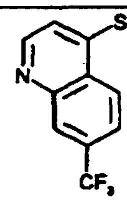 ; | Acca; |
| 245; | Ac; | ---; | ---; | Chg; | Val; | 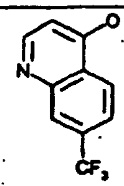 ; | Acca; |
| 246; | Ac; | ---; | ---; | Chg; | Val; | 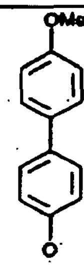 ; | Acca; |

| Vbdg. Nr.; | B; | P6; | P5; | P4; | P3; | R13; | P1; |
|---|---|---|---|---|---|---|---|
| 247; | Ac; | Asp; | Asp; | Ile; | Val; | Ph(CH$_2$)$_2$; | Nva; |
| 248; | Ac; | ---; | ---; | Chg; | Chg; | 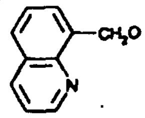 ; | Acca; |
| 249; | Ac; | ---; | ---; | Chg; | Val; | (4I-Ph)O; | Acca; |
| 250; | Ac; | ---; | ---; | Chg; | Val; | 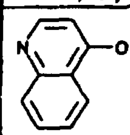 ; | Acca; |
| 251; | Ac; | ---; | ---; | Chg; | Val; | 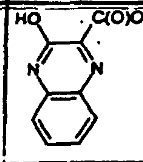 ; | Acca; |
| 252; | Ac; | ---; | ---; | Chg; | Val; | 1-NpCH$_2$O; | Nva; |
| 253; | Ac; | ---; | ---; | Chg; | Val; | 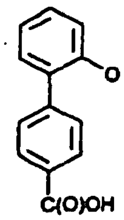 ; | Acca; |
| 254; | Ac; | ---; | ---; | Chg; | Val; | 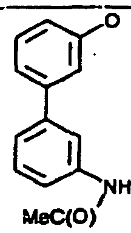 ; | Acca; |
| 255; | Ac; | ---; | ---; | Chg; | Val; | 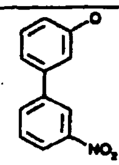 ; | Acca; |
| 256; | Ac; | ---; | ---; | Chg; | Val; | 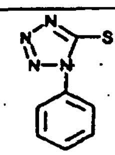 ; | Acca; |
| 257; | Ac; | ---; | ---; | Chg; | Val; | 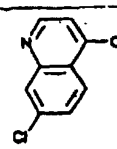 ; | Acca; |

| Vbdg. Nr. ; | B; | P6; | P5; | P4; | P3; | R13; | P1; |
|---|---|---|---|---|---|---|---|
| 258 ; | Ac; | ---; | ---; | Chg; | Val; | 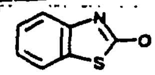 ; | Acca; |
| 259 ; | Ac; | ---; | ---; | Chg; | Val; | 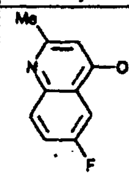 ; | Acca; |
| 262 ; | Ac; | ---; | ---; | Ile; | Val; | 1-NpCH$_2$O; | Acca; |
| 263 ; | 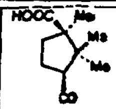 ; | ---; | ---; | Ile; | Val; | 1-NpCH$_2$O; | Acca; |
| 264 ; | 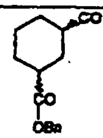 ; | ---; | ---; | Ile; | Val; | 1-NpCH$_2$O; | Acca; |
| 265 ; | 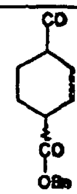 ; | ---; | ---; | Ile; | Val; | 1-NpCH$_2$O; | Acca; |
| 266 ; | 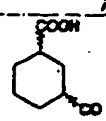 ; | ---; | ---; | Ile; | Val; | 1-NpCH$_2$O; | Acca; |
| 267 ; | 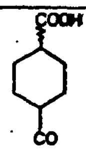 ; | ---; | ---; | Ile; | Val; | 1-NpCH$_2$O; | Acca; |
| 268 ; | Ac; | ---; | ---; | Chg; | Val; | (3Br-Ph) CH$_2$O; | Acca; |
| 269 ; | 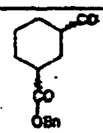 ; | ---; | ---; | Chg; | Val; | 1-NpCH$_2$O; | Acca; |
| 270 ; | 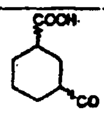 ; | ---; | ---; | Chg; | Val; | 1-NpCH$_2$O; | Acca; |
| 271 ; | 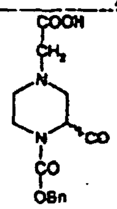 ; | ---; | ---; | Chg; | Val; | 1-NpCH$_2$O; | Acca; |

| Vbdg. Nr. ; | B; | P6; | P5; | P4; | P3; | $R_{13}$; | P1; |
|---|---|---|---|---|---|---|---|
| 272 ; | Ac; | ---; | ---; | Chg; | Val; | (3,5-$Br_2$-Ph)$CH_2O$; | Acca; |
| 273 ; | Ac; | Asp; | Asp; | Ile; | Val; | H; | Nva; |
| und 275 ; | Ac; | ---; | ---; | Chg; | Val; | 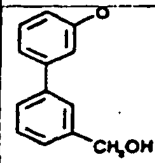 ; | Acca. |

**36.** Verbindung nach Anspruch 1, ausgewählt aus der Gruppe bestehend aus einer Verbindung der Formel

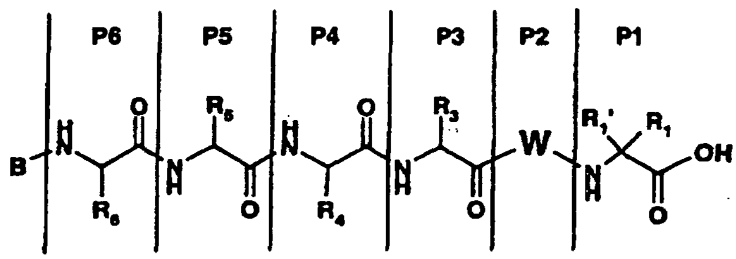

worin B, P6, P5, P4, P3, W und P1 wie nachstehend definiert sind:

| Eintrag Nr. | B; | P6; | P5; | P4; | P3; | W; | P1; |
|---|---|---|---|---|---|---|---|
| 301; | Ac; | Asp; | Asp; | Ile; | Val; | 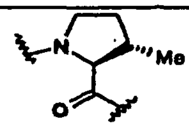 ; | Nva; |
| 302; | Ac; | Asp; | Asp; | Ile; | Val; | 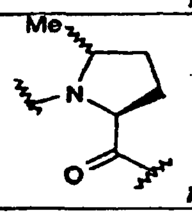 ; | Nva; |

**37.** Verbindung nach Anspruch 1, ausgewählt aus der Gruppe bestehend aus einer Verbindung der Formel

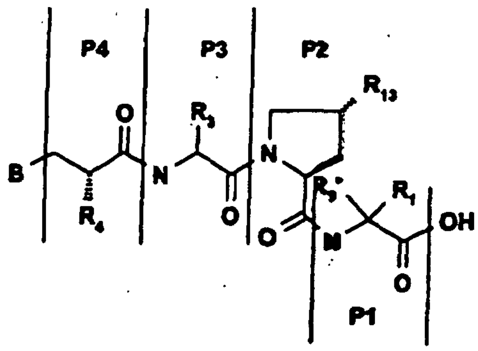

worin B, $R_4$, P3, $R_{13}$ und P1 wie nachstehend definiert sind:

| Vbdg.; | B; | $R_4$; | P3; | $R_{13}$; | P1; |
|---|---|---|---|---|---|
| 401; | 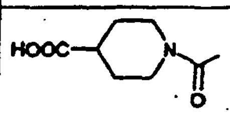 ; | Cyclohexyl; | Val; | 1-$NpCH_2O$; | Acca; |
| 402; | 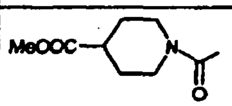 ; | Cyclohexyl; | Val; | 1-$NpCH_2O$; | Acca; |
| 403; | 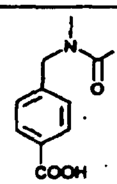 ; | Cyclohexyl; | Val; | 1-$NpCH_2O$; | Acca; |
| 404; | 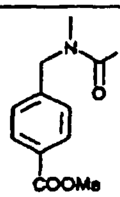 ; | Cyclohexyl; | Val; | 1-$NpCH_2O$; | Acca; |
| 405; | $HOOC-CH_2CH_2-N(Me)C(O)-$; | Cyclohexyl; | Val; | 1-$NpCH_2O$; | Acca; |
| 406; | $MeOOC-CH_2-CH_2-N(Me)C(O)-$; | Cyclohexyl; | Val; | 1-$NpCH_2O$; | Acca; |
| 407; | $HOOC-CH_2CH_2-N(CH(Me)_2)-C(O)-$; | Cyclohexyl; | Val; | 1-$NpCH_2O$; | Acca; |

| Vbdg.; | B; | $R_4$; | P3; | $R_{13}$; | P1; |
|---|---|---|---|---|---|
| 408; | MeOOC-$(CH_2)_2$-N(CH(Me)$_2$)-C(O)-; | Cyclo-hexyl; | Val; | 1-NpCH$_2$O; | Acca; |
| 409; | HOOC-$CH_2$-N(CH(Me)$_2$)-C(O)-; | Cyclo-hexyl; | Val; | 1-NpCH$_2$O; | Acca; |
| 410; | EtOOC-$CH_2$-N(CH(Me)$_2$)-C(O)-; | Cyclo-hexyl; | Val; | 1-NpCH$_2$O; | Acca; |
| 411; | HOOC-$(CH_2)_3$-N(CH(Me)$_2$)-C(O); | Cyclo-hexyl; | Val; | 1-NpCH$_2$O; | Acca; |
| 412; | [HOOC-$CH_2$]$_2$-NC(O)-; | Cyclo-hexyl; | Val; | 1-NpCH$_2$O; | Acca; |
| 413; | [HOOC-$(CH_2)_2$]$_2$-NC(O)-; | Cyclo-hexyl; | Val; | 1-NpCH$_2$O; | Acca; |
| 414; | 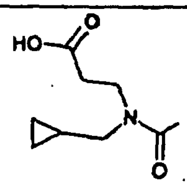 ; | Cyclo-hexyl; | Val; | 1-NpCH$_2$O; | Acca; |
| 415; | 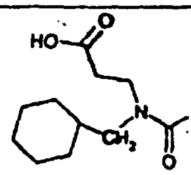 ; | Cyclo-hexyl; | Val; | 1-NpCH$_2$O; | Acca; |
| 416; | 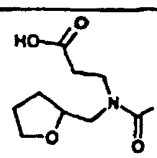 ; | Cyclo-hexyl; | Val; | 1-NpCH$_2$O; | Acca; |
| 417; | 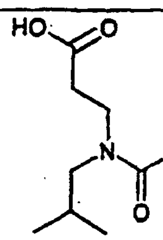 ; | Cyclo-hexyl; | Val; | 1-NpCH$_2$O; | Acca; |

| Vbdg. ; | B; | $R_4$; | P3; | $R_{13}$; | P1; |
|---|---|---|---|---|---|
| 418; | 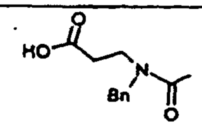 ; | Cyclo-hexyl; | Val ; | 1-$NpCH_2O$; | Acca; |
| 419; | 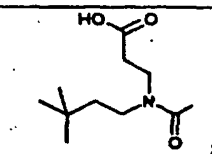 ; | Cyclo-hexyl; | Val ; | 1-$NpCH_2O$; | Acca; |
| 420; | 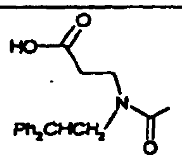 ; | Cyclo-hexyl; | Val ; | 1-$NpCH_2O$; | Acca; |
| 421; | 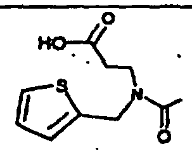 ; | Cyclo-hexyl; | Val ; | 1-$NpCH_2O$; | Acca; |
| 422; | 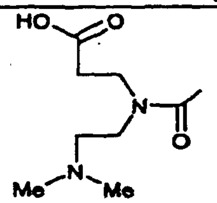 ; | Cyclo-hexyl; | Val ; | 1-$NpCH_2O$; | Acca; |
| und 423; | 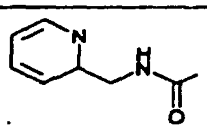 ; | Cyclo-hexyl; | Val ; | 1-$NpCH_2O$; | Acca. |

**38.** Verwendung eines Zwischenprodukts der Formel:

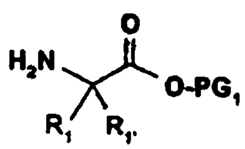

worin $R_1$ und $R_{1'}$ einen 3-gliedrigen Ring gegebenenfalls substituiert mit $C_{1-6}$-Alkyl bilden, zur Synthese einer Verbindung der Formel I nach irgendeinem der vorhergehenden Ansprüche.

**39.** Verwendung nach Anspruch 38, worin die Carboxyl-Schutzgruppe (PG1) ausgewählt ist aus der Gruppe bestehend aus Alkylestern, Aralkylestern und Estern, die durch milde Basenbehandlung oder milde reduktive Mittel gespalten werden können.

**Revendications**

**1.** Composé de formule (I) :

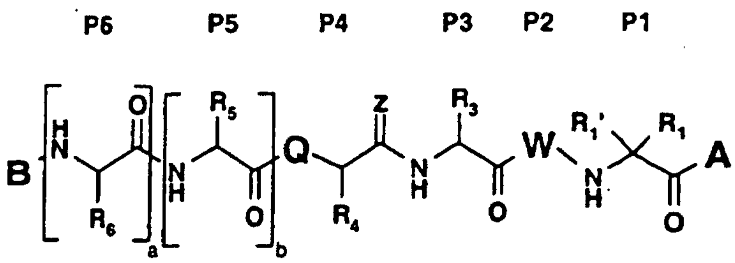

(I)

où **Q** est $CH_2$ ou N-**Y**, où **Y** est H ou $C_{1-6}$ alkyle ;

a) quand **Q** est $CH_2$, **a** est 0, **b** est 0 et **B** est un dérivé amide de formule $\mathbf{R_{11a}}$N($\mathbf{R_{11b}}$)-C(O)- où $\mathbf{R_{11a}}$ est H ; $C_{1-10}$ alkyle éventuellement substitué avec carboxyle ou di($C_{1-6}$ alkyl)amino ; $C_{3-7}$ cycloalkyle ; $C_6$ aryle ; $C_{7-10}$ alkylaryle ; ($C_{3-7}$cycloalkyl)-($C_{1-6}$alkyle) ; hétérocycle- $C_{1-6}$ alkyle ;
et $\mathbf{R_{11b}}$ est $C_{1-6}$ alkyle substitué avec carboxyle, ($C_{1-6}$ alcoxy)carbonyle ou phénylméthoxycarbonyle ; ou $C_{7-16}$ aralkyle substitué sur la partie aromatique avec carboxyle, ($C_{1-6}$ alcoxy)carbonyle, phénylméthoxycarbonyle, ou hétérocycle-$C_{1-6}$alkyle ;
ou $\mathbf{R_{11a}}$ et $\mathbf{R_{11b}}$ sont liés pour former un cycle contenant de l'azote à 3 à 7 chaînons éventuellement substitué avec carboxyle ou ($C_{1-6}$ alcoxy)carbonyle ;
ou
b) quand **Q** est N-**Y** ; **a** est 0 ou 1, **b** est 0 ou 1, et **B** est un dérivé acyle de formule $\mathbf{R_{11}}$-C(O)- où $\mathbf{R_{11}}$ est (i) $C_{1-10}$ alkyle éventuellement substitué avec carboxyle, $C_{1-6}$ alcanoyloxy (par exemple $AcOCH_2$) ou $C_{1-6}$ alcoxy (par exemple Boc) ; (ii) $C_{3-7}$ cycloalkyle éventuellement substitué avec carboxyle, ($C_{1-6}$ alcoxy)carbonyle ou phénylméthoxycarbonyle ; (iii) $C_{3-7}$ cycloalkyle substitué avec carboxyle et un à trois substituants $C_{1-6}$ alkyle (iv) $C_{4-10}$ (alkylcycloalkyle) éventuellement substitué sur la partie cycloalkyle avec carboxy, ($C_{1-6}$ alcoxy)carbonyle ou phénylméthyloxycarbonyle ; (v)

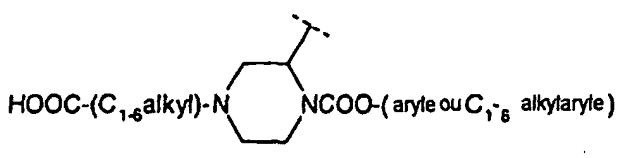

,

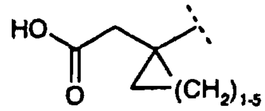

ou

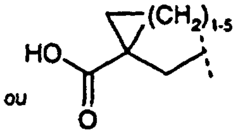

;

(vi) $C_6$ ou $C_{10}$ aryle ou $C_{7-16}$ aralkyle éventuellement substitué avec $C_{1-6}$ alkyle ;
$\mathbf{R_6}$, quand il est présent, est $C_{1-6}$ alkyle substitué avec carboxyle ; et
$\mathbf{R_5}$, quand il est présent, est $C_{1-6}$ alkyle éventuellement substitué avec carboxyle ;
ou
c) quand **Q** est $CH_2$ ou N-**Y** ;
$\mathbf{R_4}$ est $C_{1-10}$ alkyle, $C_{3-7}$ cycloalkyle ou $C_{4-10}$ (alkylcycloalkyle) ;
**Z** est oxo ou thioxo ;
$\mathbf{R_3}$ est $C_{1-10}$ alkyle éventuellement substitué avec carboxyle, $C_{3-7}$ cycloalkyle ou $C_{4-10}$ (alkylcycloalkyle) ;
**W** est un groupe de formule III' :

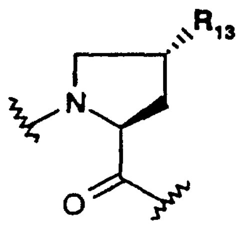

formule III'

où $\mathbf{R_{13}}$ est OH ; SH ; $NH_2$ ; carboxyle ; $\mathbf{R_{12}}$ ; O$\mathbf{R_{12'}}$ S$\mathbf{R_{12'}}$ NH$\mathbf{R_{12}}$ ou N$\mathbf{R_{12}R_{12'}}$ où $\mathbf{R_{12}}$ et $\mathbf{R_{12'}}$ sont indépendamment :

$C_{3-16}$ alkyle cyclique ou $C_{1-16}$ alkyle acyclique ou $C_{3-16}$ alcényle cyclique ou $C_{2-16}$ alcényle acyclique, ledit alkyle ou alcényle éventuellement substitué avec $NH_2$, OH, SH, halogéno ou carboxyle; ledit alkyle ou alcényle contenant éventuellement au moins un hétéroatome choisi indépendamment dans le groupe consistant en : O, S et N ;

ou

$\mathbf{R_{12}}$ et $\mathbf{R_{12'}}$ sont indépendamment $C_6$ ou $C_{10}$ aryle ou $C_{7-16}$ aralkyle éventuellement substitué avec $C_{1-6}$ alkyle, $NH_2$, OH, SH, halogène, carboxyle ou carboxy $C_{1-6}$ alkyle ; ledit aryle ou aralkyle contenant éventuellement au moins un hétéroatome choisi indépendamment dans le groupe consistant en : O, S et N ;
ledit alkyle cyclique, alcényle cyclique, aryle ou aralkyle étant éventuellement condensé avec un second cycle à 5, 6 ou 7 chaînons pour former un système cyclique ou un système hétérocyclique, ledit second cycle étant éventuellement substitué avec $NH_2$, OH, SH, halogène, carboxyle ou carboxy $C_{1-6}$ alkyle ; ledit second cycle contenant éventuellement au moins un hétéroatome choisi indépendamment dans le groupe consistant en : O, S et N ;
$\mathbf{R_{1'}}$ est l'hydrogène, et $\mathbf{R_1}$ est propyle ; ou
$\mathbf{R_{1'}}$ et $\mathbf{R_1}$ forment ensemble un cycle à 3 chaînons éventuellement substitué avec $C_{1-6}$ alkyle ; et
**A** est hydroxyle ou sel ou ester pharmaceutiquement acceptable de celui-ci.

2. Composé selon la revendication 1 de formule (Ia) :

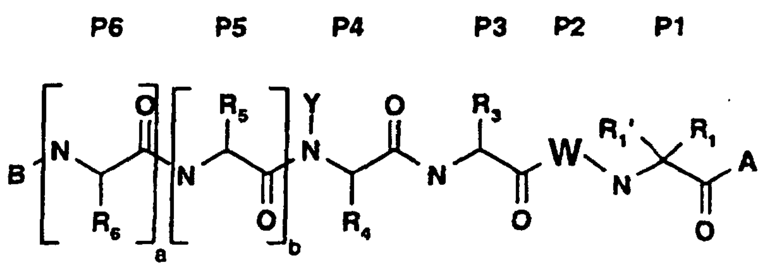

(Ia)

où **Y** est H ou $C_{1-6}$ alkyle ;
**a** est 0 ou 1 ;
**b** est 0 ou 1 ;
**B** est un dérivé acyle de formule $\mathbf{R_{11}}$-C(O)- où $\mathbf{R_3}$, $\mathbf{R_4}$, $\mathbf{R_5}$, $\mathbf{R_6}$, $\mathbf{R_{11}}$, **W**, $\mathbf{R_1}$, $\mathbf{R_1'}$ et **A** sont définis comme dans la revendication 1.

3. Composé de formule Ia selon la revendication 2 où **B** est un dérivé acyle de formule $\mathbf{R_{11}}$C(O)- où $\mathbf{R_{11}}$ est :

$C_{1-6}$ alkyle éventuellement substitué avec carboxyle, $C_{1-6}$ alcanoyloxy ou $C_{1-6}$ alcoxy ;

$C_{3-7}$ cycloalkyle éventuellement substitué avec carboxyle, MeOC(O), EtOC(O) ou BnOC(O) ;
3-carboxypropionyle (DAD) ou 4-carboxybutyryle (DAE) ;

ou

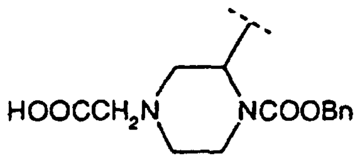

.

4. Composé de formule Ia selon la revendication 3 où **B** est acétyle, 3-carboxypropionyle, 4-carboxybutyryle, $AcOCH_2C(O)$, $Me_3COC(O)$,

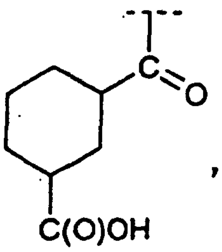 , 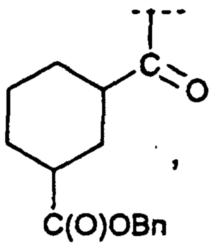 , 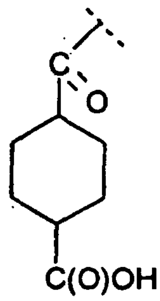 , 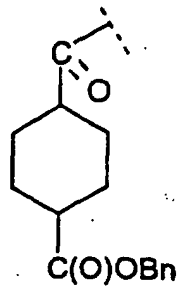 ,

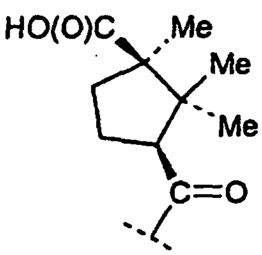 , 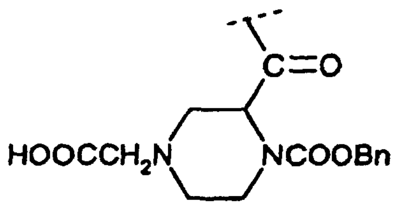 ,

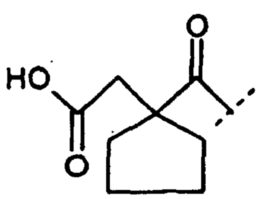 , 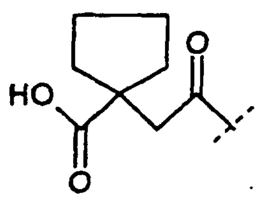 .

5. Composé de formule Ia selon la revendication 4 où **B** est acétyle, 3-carboxypropionyle (DAD), 4-carboxybutyryle (DAE), $AcOCH_2C(O)$,

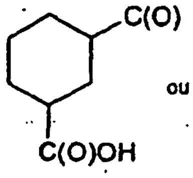

ou

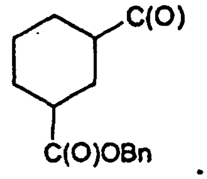

6. Composé de formule Ia selon la revendication 5 où **B** est acétyle.

7. Composé de formule Ia selon la revendication 2 où $\mathbf{R_6}$, quand il est présent, est la chaîne latérale de Asp ou Glu.

8. Composé de formule Ia selon la revendication 7 où $\mathbf{R_6}$, quand il est présent, est la chaîne latérale de Asp.

9. Composé de formule la selon la revendication 2 où $\mathbf{R_5}$, quand il est présent, est la chaîne latérale d'un aminoacide choisi dans le groupe consistant en D-Asp, Asp, D-Glu, Glu, D-Val, Val, D-Tbg et Tbg.

10. Composé de formule Ia selon la revendication 9 où $\mathbf{R_5}$, quand il est présent, est la chaîne latérale de D-Asp, D-Val ou D-Glu.

11. Composé de formule Ia selon la revendication 10 où $\mathbf{R_5}$, quand il est présent, est la chaîne latérale de D-Glu.

12. Composé selon la revendication 1 de formule (Ib) :

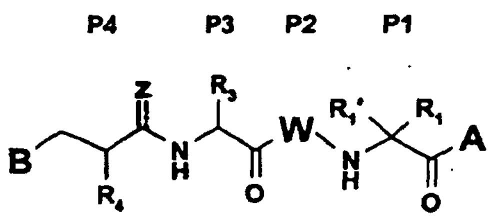

(Ib)

où **B** est un amide de formule $\mathbf{R_{11a}}N(\mathbf{R_{11b}})C(O)$- où $\mathbf{R_{11a}}$ est $C_{1-6}$ alkyle éventuellement substitué avec carboxy ou $\mathbf{R_{11a}}$ est $C_6$ aryle, $C_{7-10}$ arylalkyle, 2-tétrahydrofuranylméthyle ou 2-thiazolidylméthyle ; et $\mathbf{R_{11b}}$ est $C_{1-6}$ alkyle substitué avec carboxyle.

13. Composé de formule (Ib) selon la revendication 12 où $\mathbf{R_{11a}}$ est isopropyle, carboxyéthyle, benzylméthyle, benzyle ou 2-tétrahydrofuranylméthyle.

14. Composé de formule (Ib) selon la revendication 13 où $\mathbf{R_{11b}}$ est $C_{1-4}$ alkyle substitué avec carboxyle.

15. Composé de formule (1b) selon la revendication 14 où $\mathbf{R_{11b}}$ est éthyl carboxyle.

16. Composé de formule I selon la revendication 1 où $\mathbf{R_4}$ est choisi dans le groupe consistant en : isopropyle, cyclopropyle, tert-butyle, 1-méthylpropyle ou 2-méthylpropyle.

17. Composé de formule I selon la revendication 16 où $\mathbf{R_4}$ est cyclopropyle ou 1-méthylpropyle.

18. Composé de formule Ia selon la revendication 17 où $\mathbf{R_4}$ est cyclopropyle.

19. Composé de formule I selon la revendication 1 où **Z** est oxo.

**20.** Composé de formule I selon la revendication 1 où $\mathbf{R_3}$ est la chaîne latérale de Ile, allo-Ile, Chg, Cha, Val, Tbg ou Glu.

**21.** Composé de formule I selon la revendication 20 où $\mathbf{R_3}$ est la chaîne latérale de Val, Tbg ou Chg.

**22.** Composé de formule I selon la revendication 21 où $\mathbf{R_3}$ est la chaîne latérale de Val.

**23.** Composé de formule I selon la revendication 1 où $\mathbf{R_{13}}$ est O$\mathbf{R_{12}}$ ou $\mathbf{SR_{12}}$ où $\mathbf{R_{12}}$ est un $C_6$ ou $C_{10}$ aryle ou $C_{7-16}$ aralkyle, ledit premier aryle ou aralkyle éventuellement substitué avec $C_{1-6}$ alkyle, $C_{3-7}$ cycloalkyle, $NH_2$, OH, SH, halogène, $C_{1-6}$ alcoxy, carboxyle, carboxy $C_{1-6}$ alkyle, ou un second aryle ou aralkyle ; lesdits premier et second aryle ou aralkyle contenant éventuellement au moins un hétéroatome choisi indépendamment dans le groupe consistant en : O, S et N.

**24.** Composé selon la revendication 23 où $\mathbf{R_{13}}$ est Bn ; $PhCH_2CH_2$ ; $PhCH_2CH_2CH_2$; O-Bn ; o-tolylméthoxy ; m-tolylméthoxy ; p-tolylméthoxy ; 1-naphtyloxy ; 2-naphtyloxy ; 1-naphtalénylméthoxy ; 2-naphtalénylméthoxy ; (4-tert-butyl)méthoxy; (3I-Ph)$CH_2$O ; (4Br-Ph)O ; (2Br-Ph)O ; (3Br-Ph)O ; (4I-Ph)O ; (3Br-Ph)$CH_2$O ; (3,5-$Br_2$-Ph) $CH_2$O ;

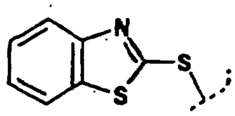 ; 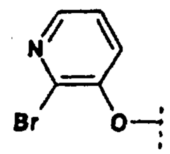 ; 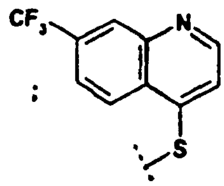 ; 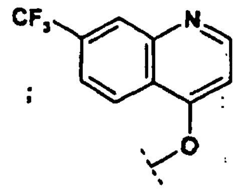 ;

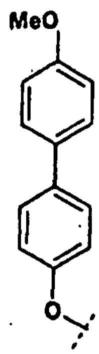 ; 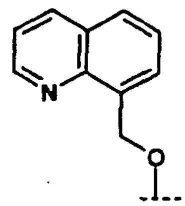 ; 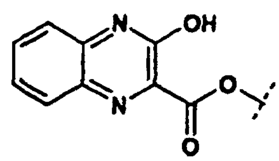 ;

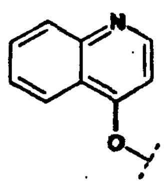 ; 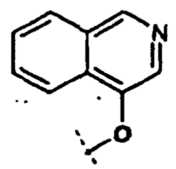 ; 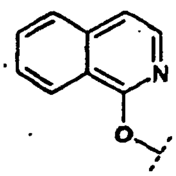 ; 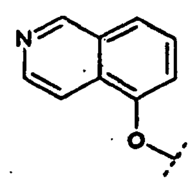 ;

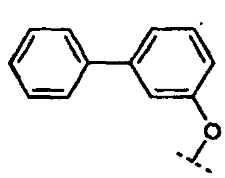 ; 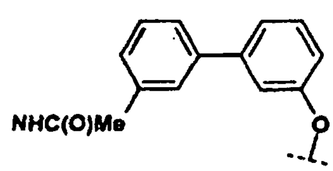 ;

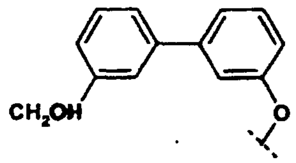 ; 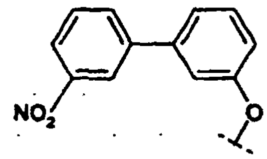 ;

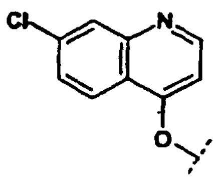 ; 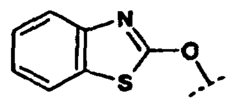 ; 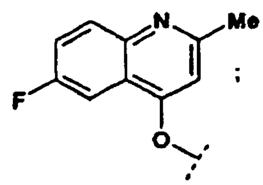 ;

25. Composé selon la revendication 24 où $\mathbf{R_{13}}$ est O-Bn ; $PhCH_2CH_2CH_2$ ; 1-naphtyloxy ; 2-naphtyloxy ; 1-naphtalénylméthoxy ; 2-naphtalénylméthoxy ;

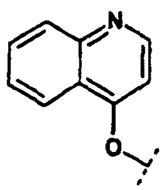 ; 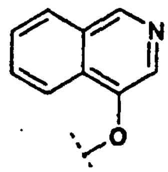 ; 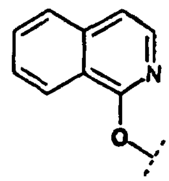 ; 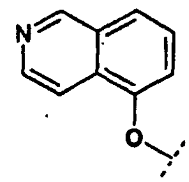 ;

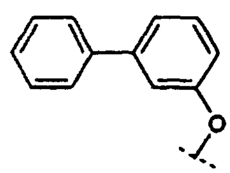 ; 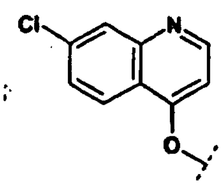

26. Composé de formule 1 selon la revendication 1 où

a) **Q** est $CH_2$, **a** est 0, **b** est 0 et **B** est un amide de formule $\mathbf{R_{11a}}N(\mathbf{R_{11b}})$-C(O)- où $\mathbf{R_{11a}}$ est $C_{1-6}$ alkyle éventuellement substitué avec carboxyle ou $\mathbf{R_{11a}}$ est phényle, $C_{7-10}$ arylalkyle, 2-tétrahydrofuranylméthyle ou 2-thiénylméthyle ;

et $\mathbf{R_{11b}}$ est ($C_{0-2}$ alkyl)phényle éventuellement substitué avec carboxyle ou ($C_{1-4}$ alcoxy)carbonyle ; ou $C_{1-6}$ alkyle substitué avec carboxyle ou ($C_{1-4}$ alcoxy)carbonyle ;

ou $\mathbf{R_{11a}}$ et $\mathbf{R_{11b}}$ sont liés pour former un cycle pipéridine éventuellement substitué avec carboxyle ou ($C_{1-6}$ alcoxy)carbonyle ;

ou

b) Q est N-**Y**, où **Y** est H ou $C_{1-6}$ alkyle ; **a** est 0 ou 1, **b** est 0 ou 1, et **B** est un dérivé acyle de formule $\mathbf{R_{11}}$-C(O)- où $\mathbf{R_{11}}$ est (i) $C_{1-6}$ alkyle, $C_{1-6}$ alkyle substitué avec carboxyle, MeC(O)O-, MeO-, EtO-, $MeCH_2CH_2O$- ou $Me_3C$-O- ; (ii) cyclopentyle ou cyclohexyle éventuellement substitué avec carboxyle ; (iv) $C_{4-10}$ (alkylcycloalkyle) éventuellement substitué sur la partie cycloalkyle avec carboxyle ;

(v)

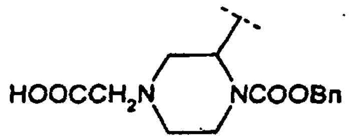

(vi) phényle, benzyle ou phényléthyle ;
**$R_6$,** quand il est présent, est $CH_2COOH$ ou $CH_2CH_2COOH$,
**$R_5$,** quand il est présent, est $C_{1-6}$ alkyle ou $CH_2COOH$ ou $CH_2CH_2COOH$ ;
ou
c) quand **Q** est $CH_2$ ou N-**Y** ;
**$R_4$** est $C_{1-6}$ alkyle, $C_{3-7}$ cycloalkyle ou $C_{4-10}$ (alkylcycloalkyle) ;
**Z** est oxo ou thio ;
**$R_3$** est $C_{1-6}$ alkyle ; $C_{3-7}$ cycloalkyle ou $C_{4-10}$ (alkylcycloalkyle) ;
**$R_{1'}$** est l'hydrogène et **$R_1$** est propyle ; ou **$R_{1'}$** et **$R_1$** forment avec l'atome de carbone auquel ils sont liés un cyclopropyle qui peut éventuellement être substitué avec éthyle ; et
**W** est défini comme dans la revendication 1 ; et
**A** est hydroxyle ou un sel pharmaceutiquement acceptable de celui-ci ; $C_{1-6}$ alcoxy ou (aryl $C_{1-6}$ alcoxy).

**27.** Composé de formule Ia selon la revendication 2 où **B** est un dérivé acyle de formule **$R_{11}$**-C(O)- où **$R_{11}$** est $C_{1-6}$ alcoxy, $C_{1-10}$ alkyle éventuellement substitué avec carboxyle ; $C_{3-7}$ cycloalkyle éventuellement substitué avec carboxyle ou benzylcarboxy ; ou

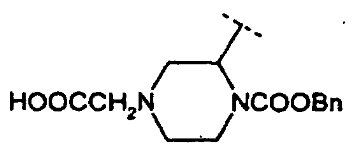

;

**$R_6$** est absent ;
**$R_5$** est absent ;
**$R_4$** est $C_{1-10}$ alkyle, $C_{3-7}$ cycloalkyle ou $C_{4-10}$ (alkylcycloalkyle) ;
**$R_3$** est $C_{1-10}$ alkyle, $C_{3-7}$ cycloalkyle ou $C_{4-10}$ (alkylcycloalkyle) ;
et
**W** est défini comme dans la revendication 1 ; et
**A** est hydroxy ou un sel pharmaceutiquement acceptable de celui-ci ; méthoxy, éthoxy, phénoxy ou benzyloxy.

**28.** Composé de formule Ia selon la revendication 2 où **B** est acétyle, 3-carboxypropionyle, 4-carboxybutyryle, $AcOCH_2C(O)$, $Me_3COC(O)$,

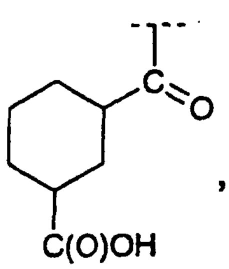 , 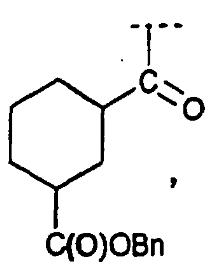 , 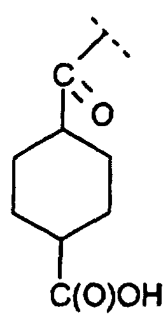 , 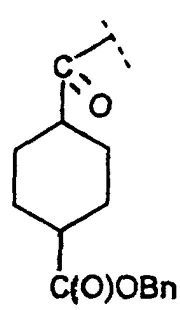 ,

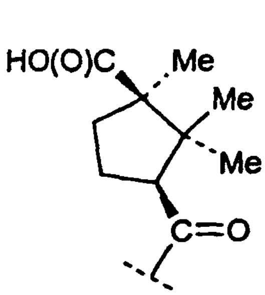 , 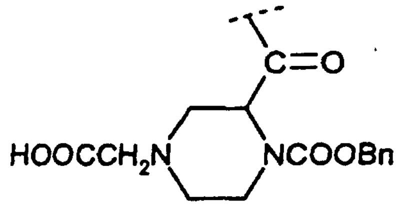 ,

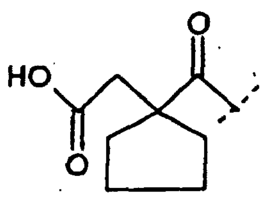 , 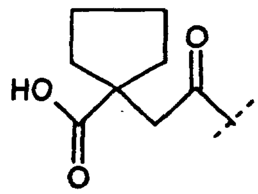 ;

**Y** est H ou Me, **a** est 0 ou 1, **b** est 0 ou 1,
$\mathbf{R_6}$, quand il est présent, est la chaîne latérale de Asp ou Glu,
$\mathbf{R_5}$, quand il est présent, est la chaîne latérale de Asp, D-Asp, Glu, D-Glu, Val, D-Val ou Tbg,
$\mathbf{R_4}$ est la chaîne latérale de Val, Chg, Tbg, Ile ou Leu,
$\mathbf{R_3}$ est l'hydrogène ou la chaîne latérale de Ile, Chg, Val, Glu ;
**W** est un groupe de formule III' :

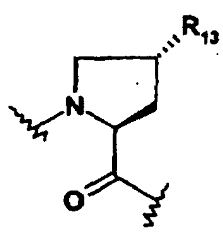 (III')

où $\mathbf{R_{13}}$ est Bn, $PhCH_2CH_2$, $PhCH_2CH_2CH_2$, O-Bn, o-tolylméthoxy, m-tolylméthoxy, p-tolylméthoxy, 1-naphtalénylméthoxy, 2-naphtalénylméthoxy, (4-tert-butyl)benzyloxy, $(3I\text{-}Ph)CH_2O$, (4Br-Ph)O, (2Br-Ph)O, (3Br-Ph)O, (4I-Ph)O, $(3Br\text{-}Ph)CH_2O$, $(3,5\text{-}Br_2\text{-}Ph)CH_2O$,

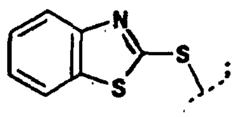 ; 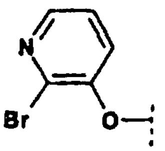 ; 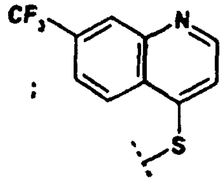 ; 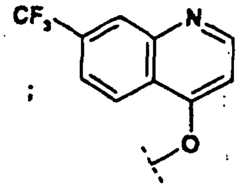 ;

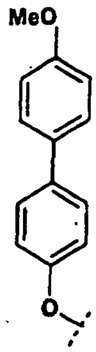 ; 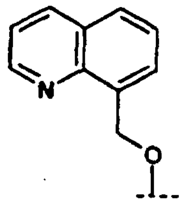 ; 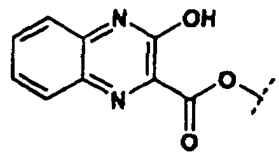 ;

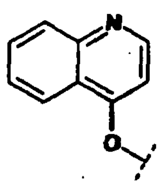 ; 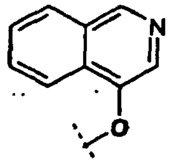 ; 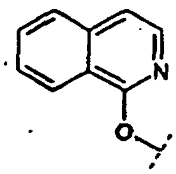 ; 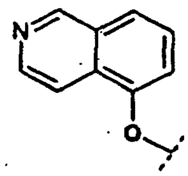 ;

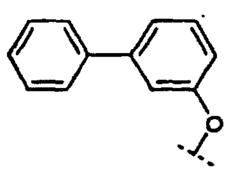 ; 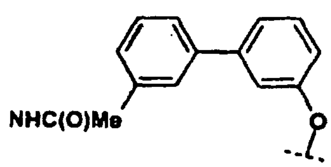 ;

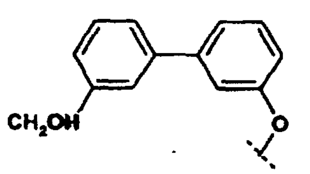 ; 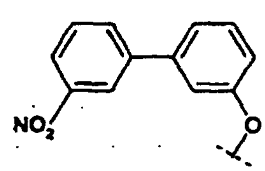 ;

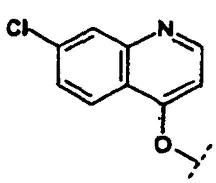 ; 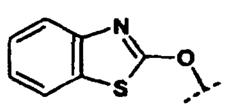 ; 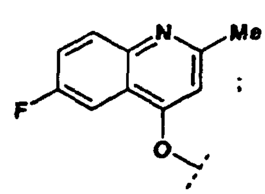 ;

**R$_{1'}$** est H et **R$_1$** est propyle ; ou
**R$_{1'}$** et **R$_1$** forment avec l'atome de carbone auquel ils sont liés un cyclopropyle ; et **A** est hydroxyle.

**29.** Composé de formule Ib selon la revendication 12 où **B** est un amide de formule **R$_{11a}$**N(**R$_{11b}$**)-C(O)- où **R$_{11a}$** est $C_{1-6}$ alkyle éventuellement substitué avec carboxyle ou **R$_{11a}$** est 2-tétrahydrofuranylméthyle ;

et $\mathbf{R_{11b}}$ est ($C_{0-2}$ alkyl)phényle éventuellement substitué avec carboxyle ou ($C_{1-4}$ alcoxy)carbonyle ; ou $C_{1-6}$ alkyle substitué avec carboxyle ou ($C_{1-4}$ alcoxy)carbonyle ;
ou $\mathbf{R_{11a}}$ et $\mathbf{R_{11b}}$ sont liés pour former un cycle pipéridine éventuellement substitué avec carboxyle ou ($C_{1-6}$ alcoxy) carbonyle ;
$\mathbf{R_4}$ est cyclohexyle ;
**Z** est oxo ;
$\mathbf{R_3}$ est l'hydrogène ou la chaîne latérale de Ile, Chg, Val, Glu ;
**W** est un groupe de formule III' :

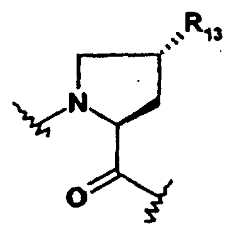

(III')

où $\mathbf{R_{13}}$ est Bn, $PhCH_2CH_2$, $PhCH_2CH_2CH_2$, O-Bn, o-tolylméthoxy, m-tolylméthoxy, p-tolylméthoxy, 1-naphtalénylméthoxy, 2-naphtalénylméthoxy, (4-*tert*-butyl)méthoxy, (3I-Ph)$CH_2O$, (4Br-Ph)O, (2Br-Ph)O, (3Br-Ph)O, (4I-Ph)O, (3Br-Ph)$CH_2O$, (3,5-$Br_2$-Ph)$CH_2O$,

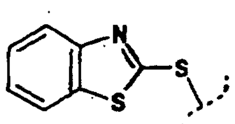 ; 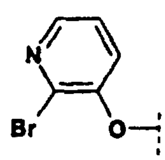 ; 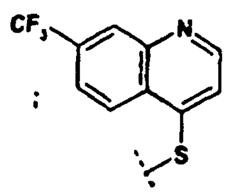 ; 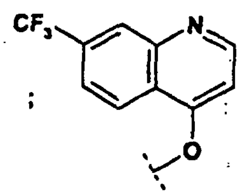 ;

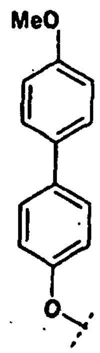 ; 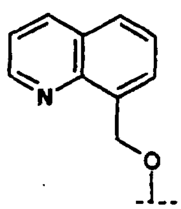 ; 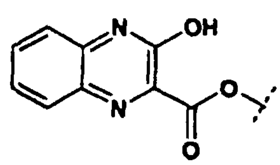 ;

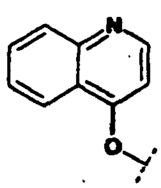 ; 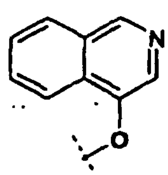 ; 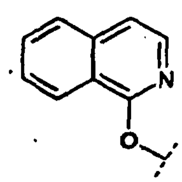 ; 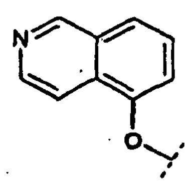 ;

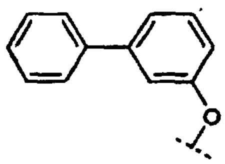 ; 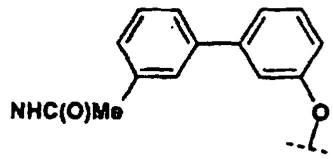 ;

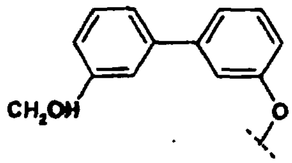 ; 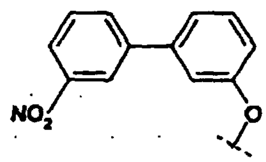 ;

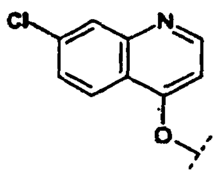 ; 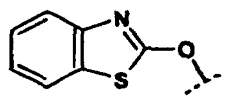 ; 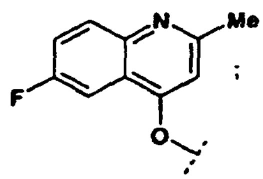 ;

**$R_{1'}$** est H et **$R_1$** est propyle ; ou
**$R_{1'}$** et **$R_1$** forment avec l'atome de carbone auquel ils sont liés un cyclopropyle ; et **A** est hydroxyle.

**30.** Composé de formule I selon la revendication 1 où **B** est un dérivé acyle de formule **$R_{11}$**-C(O)- où **$R_{11}$** est $C_{1-10}$ alkyle éventuellement substitué avec carboxyle ; $C_{3-7}$ cycloalkyle éventuellement substitué avec carboxyle ; ou un $C_{4-10}$ (alkylcycloalkyle) éventuellement substitué sur la partie cycloalkyle avec carboxyle ;
ou **$R_{11}$** est $C_6$ ou $C_{10}$ aryle ou $C_{7-16}$ aralkyle éventuellement substitué avec un $C_{1-6}$ alkyle
**a** est 0 ou 1 ;
**$R_6$,** quand il est présent, est $C_{1-6}$ alkyle éventuellement substitué avec carboxyle ;
**b** est 0 ou 1 ;
**$R_5$,** quand il est présent, est $C_{1-6}$ alkyle éventuellement substitué avec carboxyle ;
**Q** est N-**Y**, et **Y** est H ou $C_{1-6}$ alkyle ;
**$R_4$** est $C_{1-10}$ alkyle, $C_{3-7}$ cycloalkyle ou $C_{4-10}$ (alkylcycloalkyle) ;
**Z** est oxo ;
**$R_3$** est $C_{1-10}$ alkyle ; $C_{3-7}$ cycloalkyle ou $C_{4-10}$ (alkylcycloalkyle) ;
et
**W**, **$R_{1'}$** et **$R_1$** sont définis comme dans la revendication 1 ; et
**A** est hydroxyle ou un sel ou ester pharmaceutiquement acceptable de celui-ci.

**31.** Composition pharmaceutique comprenant une quantité anti-hépatite C viralement efficace d'un composé de formule I selon la revendication 1, ou d'un sel ou ester thérapeutiquement acceptable de celui-ci, en mélange avec un milieu support ou agent auxiliaire pharmaceutiquement acceptable.

**32.** Procédé *in vitro* d'inhibition de la réplication du virus de l'hépatite C par exposition du virus à une quantité inhibant la protéase NS3 du virus de l'hépatite C du composé de formule 1 selon la revendication 1, ou d'un sel ou ester thérapeutiquement acceptable de celui-ci.

**33.** Utilisation d'un composé de formule I selon la revendication 1 pour la fabrication d'un médicament pour le traitement d'une infection d'hépatite C dans un mammifère.

**34.** Composé choisi dans le groupe consistant en : un composé de formule

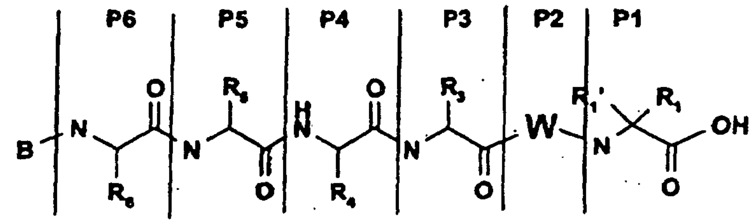

où B, P6, P5, P4, P3, W et P1 sont définis comme ci-dessous :

| Comp. n°. | B; | P6; | P5; | P4; | P3; | W; | P1; |
|---|---|---|---|---|---|---|---|
| 123; | Ac; | Asp; | Asp; | Ile; | Val; | Pro; | Nva; |
| 126; | Ac; | Asp; | Asp; | Ile; | Val; | Pro; | Acca; |
| 127; | Ac; | Asp; | Asp; | Ile; | Val; | Pip; | Nva; |
| 128; | Ac; | Asp; | D-Glu; | Ile; | Val; | Pro; | Nva; |
| 129; | Ac; | Asp; | Tbg; | Ile; | Val; | Pro; | Nva; |
| 130; | DAD; | ---; | Asp; | Ile; | Val; | Pro; | Nva; |
| 132; | Ac; | Asp; | D-Glu; | Chg; | Glu; | Glu; | Acca; |
| 133; | Ac; | Asp; | Glu; | Chg; | Val; | Glu(OBn) ; | Acca. |
| Comp. n°. | B; | P6; | P5; | P4; | P3; | W; | P1; |
| 303; | Ac; | Asp; | Asp; | Ile; | Val; | 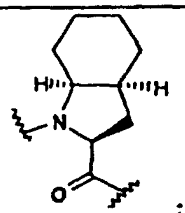 ; | Nva; |
| et 304; | Ac; | ---; | ---; | Chg; | Val; | 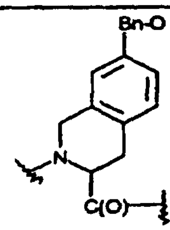 ; | Acca. |

**35.** Composé selon la revendication 1 choisi dans le groupe consistant en : un composé de formule

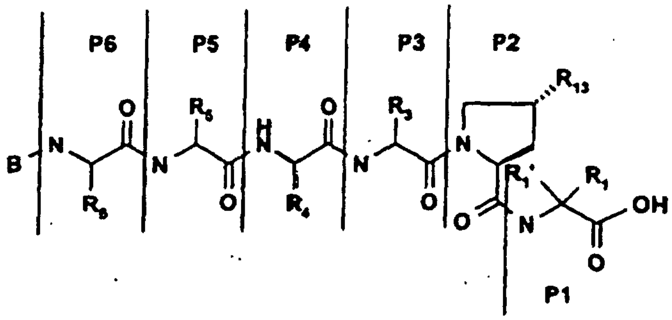

où B, P6, P5, P4, P3, $R_{13}$, et $P_1$ sont définis comme ci-dessous :

| Comp. n°. | B; | P6; | P5; | P4; | P3; | $R_{13}$; | P1; |
|---|---|---|---|---|---|---|---|
| 201 ; | Ac | Asp | Asp; | Ile; | Val; | O-Bn; | Nva; |
| 202 ; | Ac | Asp | D-Val; | Ile; | Val; | O-Bn; | Nva; |
| 203 ; | Ac | Asp | D-Glu; | Ile; | Val; | O-Bn; | Nva; |
| 204 ; | Ac | Asp | Asp; | Ile; | Val; | *o*-tolyl-methoxy; | Nva; |
| 205 ; | Ac | Asp | Asp; | Ile; | Val; | *m*-tolyl-methoxy; | Nva; |
| 206 ; | Ac | Asp | Asp; | Ile; | Val; | *p*-tolyl-methoxy; | Nva; |
| 207 ; | Ac | Asp | Asp; | Ile; | Val; | 1-$NpCH_2O$; | Nva; |
| 208 ; | Ac | Asp | Asp; | Ile; | Val; | 2-$NpCH_2O$; | Nva; |
| 209 ; | Ac | Asp | Asp; | Ile; | Val; | 4-tert-butyl-phenyl)-methoxy; | Nva; |
| 211 ; | Ac | Asp | D-Glu; | Chg; | Val; | O-Bn; | Nva; |
| 212 ; | Ac | Asp | D-Glu; | Ile; | Val; | O-Bn; | Acca; |
| 213 ; | Ac | Asp | D-Glu; | Ile; | Val; | 2-$NpCH_2O$; | Nva; |
| 214 ; | Ac | Asp | D-Glu; | Chg; | Val; | 2-$NpCH_2O$; | Nva; |
| 215 ; | Ac | Asp | D-Glu; | Chg; | Val; | 1-$NpCH_2O$; | Acca; |
| 216 | Ac | Asp | Asp; | Ile; | Val; | Bn; | Nva; |

| Comp. n°. | B; | P6; | P5; | P4; | P3; | $R_{13}$; | P1; |
|---|---|---|---|---|---|---|---|
| ; | | | | | | | |
| 217 ; | Ac | Asp | Asp; | Ile; | Val; | $Ph(CH_2)_3$; | Nva; |
| 218 ; | Ac | Asp | D-Glu; | Ile; | Val; | O-Bn; | Nva; |
| 219 ; | Ac; | ---; | Asp; | Ile; | Val; | 1-$NpCH_2O$; | Nva; |
| 220 ; | DAD; | ---; | ---; | *N*(Me)Ile; | Val; | 1-$NpCH_2O$; | Nva; |
| 221 ; | DAD; | ---; | ---; | Ile; | Val; | 1-$NpCH_2O$ ; | Nva; |
| 222 ; | DAE; | ---; | ---; | Ile; | Val; | 1-$NpCH_2O$ ; | Nva; |
| 223 ; | 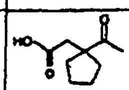 ; | ---; | ---; | Ile; | Val; | 1-$NpCH_2O$ ; | Nva; |
| 224 ; | 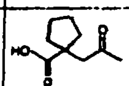 ; | ---; | ---; | Ile; | Val; | 1-$NpCH_2O$ ; | Nva; |
| 225 ; | Ac; | ---; | ---; | Ile; | Val; | 1-$NpCH_2O$ ; | Nva; |
| 226 ; | DAE; | ---; | ---; | Chg; | Val | 1-$NpCH_2O$ ; | Acca; |
| 227 ; | Ac; | ---; | ---; | Chg; | Val; | 1-$NpCH_2O$ ; | Acca; |
| 228 ; | Ac; | ---; | ---; | Chg; | Val; | O-Bn; | 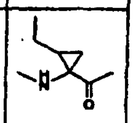 ; |
| 230 ; | Ac; | Asp; | Asp; | Ile; | Val; | $Ph(CH_2)_3$; | Nva; |
| 231 ; | Ac; | ---; | ---; | Chg; | Chg; | 1-$NpCH_2O$; | Acca; |
| 232 ; | $AcOCH_2$-C(O); | ---; | ---; | Chg; | Chg; | 1-$NpCH_2O$; | Acca; |
| 233 ; | Ac; | Asp; | Glu; | Ile; | Val; | (3I-Ph) $CH_2O$; | Acca; |
| 234 ; | Ac; | ---; | ---; | Chg; | Chg; | O-Bn; | Acca; |
| 235 ; | Boc; | ---; | ---; | Chg; | Chg; | 1-$NpCH_2O$; | Acca; |
| 236 ; | Ac; | ---; | Gly; | thioxo-Ile; | Val; | 1-$NpCH_2O$; | Nva; |
| 237 ; | DAE; | ---; | ---; | Ile; | Val; | 1-$NpCH_2O$; | Acca; |
| 238 | Ac; | ---; | ---; | Chg; | Val; | (4Br- | Acca; |

| Comp. n°. | B; | P6; | P5; | P4; | P3; | R13; | P1; |
|---|---|---|---|---|---|---|---|
| ; | | | | | | Ph)O; | |
| 239; | Ac; | ---; | ---; | Chg; | Val; | (2Br-Ph)O; | Acca; |
| 240; | Ac; | ---; | ---; | Chg; | Val; | (3Br-Ph)O; | Acca; |
| 241; | Ac; | ---; | ---; | Chg; | Val; | 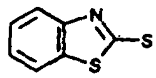 ; | Acca; |
| 242; | Ac; | ---; | ---; | Chg; | Val; | (4Br-Ph)S; | Acca; |
| 243; | Ac; | ---; | ---; | Chg; | Val; | 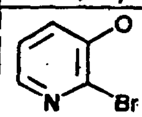 ; | Acca; |
| 244; | Ac; | ---; | ---; | Chg; | Val; | 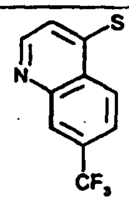 ; | Acca; |
| 245; | Ac; | ---; | ---; | Chg; | Val; | 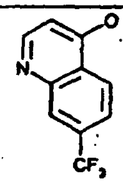 ; | Acca; |
| 246; | Ac; | ---; | ---; | Chg; | Val; | 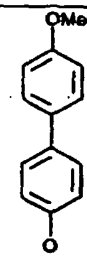 ; | Acca; |
| 247; | Ac; | Asp; | Asp; | Ile; | Val; | Ph($CH_2$)$_2$; | Nva; |
| 248; | Ac; | ---; | ---; | Chg; | Chg; | 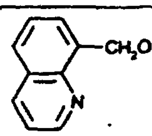 ; | Acca; |
| 249; | Ac; | ---; | ---; | Chg; | Val; | (4I-Ph)O; | Acca; |
| 250; | Ac; | ---; | ---; | Chg; | Val; | 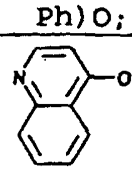 ; | Acca; |

| Comp. n°. | B; | P6; | P5; | P4; | P3; | R13; | P1; |
|---|---|---|---|---|---|---|---|
| 251 ; | Ac; | ---; | ---; | Chg; | Val; | 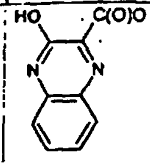 ; | Acca; |
| 252 ; | Ac; | ---; | ---; | Chg; | Val; | 1-$NpCH_2O$; | Nva; |
| 253 ; | Ac; | ---; | ---; | Chg; | Val; | 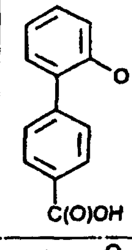 ; | Acca; |
| 254 ; | Ac; | ---; | ---; | Chg; | Val; | 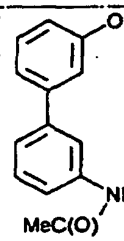 ; | Acca; |
| 255 ; | Ac; | ---; | ---; | Chg; | Val; | 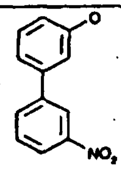 ; | Acca; |
| 256 ; | Ac; | ---; | ---; | Chg; | Val; | 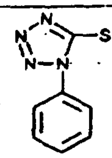 ; | Acca; |
| 257 ; | Ac; | ---; | ---; | Chg; | Val; | 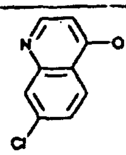 ; | Acca; |
| 258 ; | Ac; | ---; | ---; | Chg; | Val; | 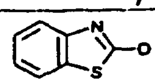 ; | Acca; |
| 259 ; | Ac; | ---; | ---; | Chg; | Val; | 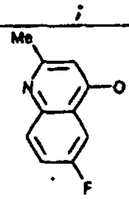 ; | Acca; |
| 262 ; | Ac; | ---; | ---; | Ile; | Val; | 1-$NpCH_2O$; | Acca; |

| Comp. n°. | B; | P6; | P5; | P4; | P3; | $R_{13}$; | P1; |
|---|---|---|---|---|---|---|---|
| 263; | 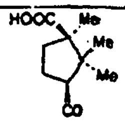 ; | ---; | ---; | Ile; | Val; | 1-NpCH$_2$O; | Acca; |
| 264; | 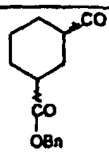 ; | ---; | ---; | Ile; | Val; | 1-NpCH$_2$O; | Acca; |
| 265; | 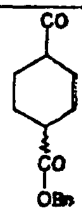 ; | ---; | ---; | Ile; | Val; | 1-NpCH$_2$O; | Acca; |
| 266; | 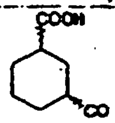 ; | ---; | ---; | Ile; | Val; | 1-NpCH$_2$O; | Acca; |
| 267; | 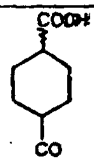 ; | ---; | ---; | Ile; | Val; | 1-NpCH$_2$O; | Acca; |
| 268; | Ac; | ---; | ---; | Chg; | Val; | (3Br-Ph)CH$_2$O; | Acca; |
| 269; | 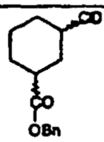 ; | ---; | ---; | Chg; | Val; | 1-NpCH$_2$O; | Acca; |
| 270; | 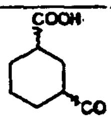 ; | ---; | ---; | Chg; | Val; | 1-NpCH$_2$O; | Acca; |
| 271; | 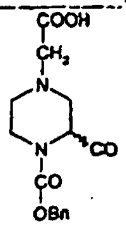 ; | ---; | ---; | Chg; | Val; | 1-NpCH$_2$O; | Acca; |
| 272; | Ac; | ---; | ---; | Chg; | Val; | (3,5-Br$_2$-Ph)CH$_2$O; | Acca; |
| 273; | Ac; | Asp; | Asp; | Ile; | Val; | H; | Nva; |

| Comp. n°. | B; | P6; | P5; | P4; | P3; | R13; | P1; |
|---|---|---|---|---|---|---|---|
| et 275 ; | Ac; | ---; | ---; | Chg; | Val; | 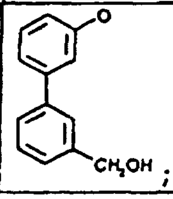 ; | Acca. |

**36.** Composé selon la revendication 1 choisi dans le groupe consistant en : un composé de formule

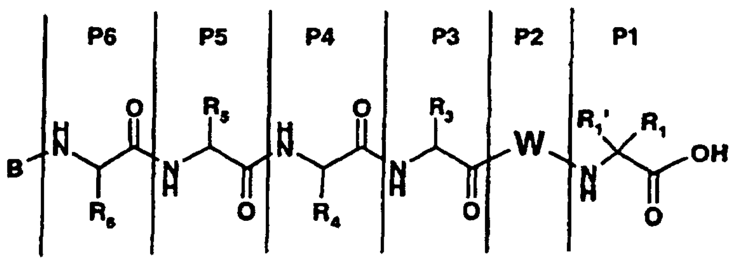

où B, P6, P5, P4, P3, W et P1 sont définis comme ci-dessous :

| Entrée n°. | B; | P6; | P5; | P4; | P3; | W; | P1; |
|---|---|---|---|---|---|---|---|
| 301; | Ac; | Asp; | Asp; | Ile; | Val; | 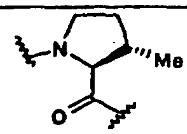 ; | Nva; |
| 302; | Ac; | Asp; | Asp; | Ile; | Val; | 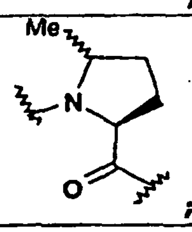 ; | Nva; |

**37.** Composé selon la revendication 1 choisi dans le groupe consistant en : un composé de formule

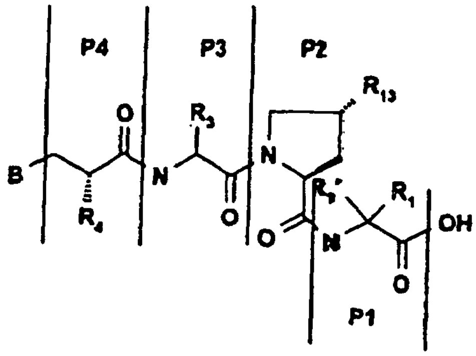

où B, $R_4$, P3, $R_{13}$ et P1 sont définis comme ci-dessous :

| Comp. | B; | $R_4$; | P3; | $R_{13}$; | P1; |
|---|---|---|---|---|---|
| 401; | 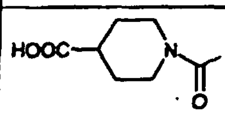 ; | cyclohexyl; | Val; | 1-$NpCH_2O$; | Acca; |
| 402; | 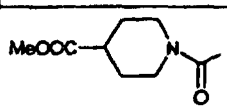 ; | cyclohexyl; | Val; | 1-$NpCH_2O$; | Acca; |
| 403; | 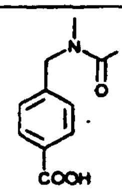 ; | cyclohexyl; | Val; | 1-$NpCH_2O$; | Acca; |
| 404; | 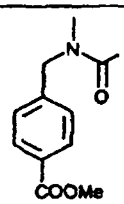 ; | cyclohexyl; | Val; | 1-$NpCH_2O$; | Acca; |
| 405; | HOOC-$CH_2CH_2$-N(Me)C(O)-; | cyclohexyl; | Val; | 1-$NpCH_2O$; | Acca; |
| 406; | MeOOC-$CH_2$-$CH_2$-N(Me)C(O)-; | cyclohexyl; | Val; | 1-$NpCH_2O$; | Acca; |
| 407; | HOOC-$CH_2CH_2$-N(CH(Me)$_2$)-C(O)-; | cyclohexyl; | Val; | 1-$NpCH_2O$; | Acca; |

| Comp. | B; | R4; | P3; | R13; | P1; |
|---|---|---|---|---|---|
| 408; | MeOOC-$(CH_2)_2$-N(CH(Me)$_2$)-C(O)-; | cyclohexyl; | Val; | 1-$NpCH_2O$; | Acca; |
| 409; | HOOC-$CH_2$-N(CH(Me)$_2$)-C(O)-; | cyclohexyl; | Val; | 1-$NpCH_2O$; | Acca; |
| 410; | EtOOC-$CH_2$-N(CH(Me)$_2$)-C(O)-; | cyclohexyl; | Val; | 1-$NpCH_2O$; | Acca; |
| 411; | HOOC-$(CH_2)_3$-N(CH(Me)$_2$)-C(O);| cyclohexyl; | Val; | 1-$NpCH_2O$; | Acca; |
| 412; | [HOOC-$CH_2$]$_2$-NC(O)-; | cyclohexyl; | Val; | 1-$NpCH_2O$; | Acca; |
| 413; | [HOOC-$(CH_2)_2$]$_2$-NC(O)-; | cyclohexyl; | Val; | 1-$NpCH_2O$; | Acca; |
| 414; | 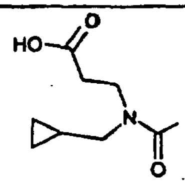 ; | cyclohexyl; | Val; | 1-$NpCH_2O$; | Acca; |
| 415; | 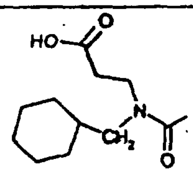 ; | cyclohexyl; | Val; | 1-$NpCH_2O$; | Acca; |
| 416; | 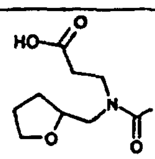 ; | cyclohexyl; | Val; | 1-$NpCH_2O$; | Acca; |
| 417; | 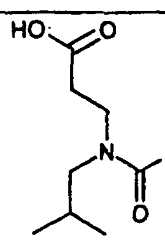 ; | cyclohexyl; | Val; | 1-$NpCH_2O$; | Acca; |

| Comp. | B; | R4; | P3; | R13; | P1; |
|---|---|---|---|---|---|
| 418; | 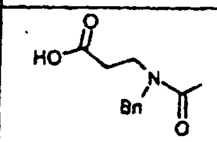 ; | cyclohexyl; | Val; | 1-NpCH2O; | Acca; |
| 419; | 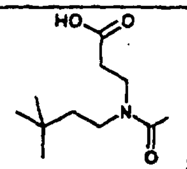 ; | cyclohexyl; | Val; | 1-NpCH2O; | Acca; |
| 420; | 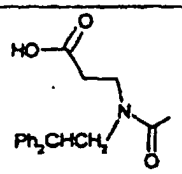 ; | cyclohexyl; | Val; | 1-NpCH2O; | Acca; |
| 421; | 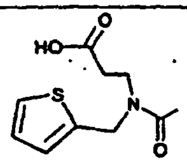 ; | cyclohexyl; | Val; | 1-NpCH2O; | Acca; |
| 422; | 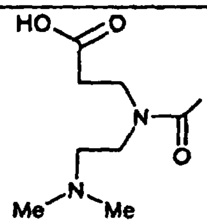 ; | cyclohexyl; | Val; | 1-NpCH2O; | Acca; |
| et 423; | 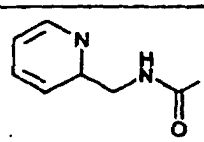 ; | cyclohexyl; | Val; | 1-NpCH2O; | Acca. |

**38.** Utilisation d'un intermédiaire de formule :

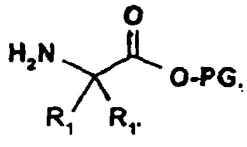

où $R_1$ et $R_{1'}$ forment un cycle à 3 chaînons éventuellement substitué avec $C_{1-6}$ alkyle, pour la synthèse d'un composé de formule I selon l'une quelconque des revendications précédentes.

**39.** Utilisation selon la revendication 38 où ledit groupe protecteur de carboxyle (PG1) est choisi dans le groupe consistant en : les alkylesters, les aralkylesters, et les esters qui sont clivables par un traitement avec une base modérée ou un moyen réducteur modéré.